# EUROPEAN PATENT SPECIFICATION

(11) **EP 1 567 489 B1**
(45) Date of publication and mention of the grant of the patent: **23.07.2008**
(21) Application number: 03780129.7
(22) Date of filing: 04.12.2003
(51) Int. Cl.: C07D 207/26, C07D 409/12, C07D 401/12, C07D 413/14, C07D 405/12, C07D 417/14, C07D 417/06, C07D 409/14, A61K 31/4025

(54) **PYRROLIDIN-2-ONE DERIVATIVES AS INHIBITORS OF THROMBIN AND FACTOR XA**
PYRROLIDIN-2-ON-DERIVATE ALS INHIBITOREN VON THROMBIN UND FAKTOR XA
DERIVES DE PYRROLYDIN-2-ONE UTILISES COMME INHIBITEURS DE LA THROMBINE ET DU FACTEUR XA

(30) Priority: 06.12.2002 GB 0228552
(43) Date of publication of application: 31.08.2005
(73) Proprietor: GLAXO GROUP LIMITED, Greenford, Middlesex UB6 ONN (GB)
(72) Inventor: BORTHWICK, Alan David, GlaxoSmithKline, Stevenage, Hertfordshire SG1 2NY (GB); CHAN, Chuen, GlaxoSmithKline, Stevenage, Hertfordshire SG1 2NY (GB); KELLY, Henry Anderson, GlaxoSmithKline, Stevenage, Hertfordshire SG1 2NY (GB); PEACE, Simon, GlaxoSmithKline, Stevenage, Hertfordshire SG1 2NY (GB); SENGER, Stefan, GlaxoSmithKline, Stevenage, Hertfordshire SG1 2NY (GB); SHAH, Gita Punjabhai, GlaxoSmithKline, Stevenage, Hertfordshire SG1 2NY (GB); SMITH, Stephen Allan, GlaxoSmithKline, Stevenage, Hertfordshire SG1 2NY (GB); SMITH, Steven, GlaxoSmithKline, Stevenage, Hertfordshire SN1 2NY (GB); WATSON, Nigel Stephen, GlaxoSmithKline, Stevenage, Hertfordshire SG1 2NY (GB); WEST, Robert Ian, GlaxoSmithKline, Stevenage, Hertfordshire SG1 2NY (GB); YOUNG, Robert John, GlaxoSmithKline, Stevenage, Hertfordshire SG1 2NY (GB)
(74) Representative: Phillips, Suzanne J.
(86) International application number: PCT/EP2003/013799
(87) International publication number: WO 2004/052851

(56) References cited:
- WO-A-93/01208
- WO-A-99/62904
- WO-A-02/100830
- WO-A-02/100886
- WO-A-03/043981

## Description

### Field of the Invention

The present invention relates to a novel class of chemical compounds, to processes for their preparation, to pharmaceutical compositions containing them and to their use in medicine, particularly use in the amelioration of a clinical condition for which a thombin inhibitor is indicated.

### Background of the Invention

Thrombin is a serine protease present in plasma. It is converted from prothrombin into thrombin by Factor Xa a member of the trypsin-like serine protease class of enzymes. Thrombin plays a central role in the mechanism of blood coagulation by converting the soluble plasma protein, fibrinogen, into insoluble fibrin. The insoluble fibrin matrix is required for the stabilisation of the primary hemostatic plug. Many significant disease states are related to abnormal hemostasis. With respect to the coronary arterial vasculature, abnormal thrombus formation due to the rupture of an established atherosclerotic plaque is the major cause of acute myocardial infarction and unstable angina. Both treatment of an occlusive coronary thrombus by thrombolytic therapy and percutaneous transluminal coronary angioplasty (PTCA) are often accompanied by an acute thrombotic reclosure of the affected vessel which requires immediate resolution. With respect to the venous vasculature, a high percentage of patients undergoing major surgery in the lower extremities or the abdominal area suffer from thrombus formation in the venous vasculature which can result in reduced blood flow to the affected extremity and a pre-disposition to pulmonary embolism. Disseminated intravascular coagulopathy commonly occurs within both vascular systems during septic shock, certain viral infections and cancer and is characterised by the rapid consumption of coagulation factors and systemic coagulation which results in the formation of life-threatening thrombi occurring throughout the vasculature leading to widespread organ failure.

Beyond its direct role in the formation of fibrin rich blood clots, thrombin has been reported to have profound bioregulatory effects on a number of cellular components within the vasculature and blood, (Shuman, M.A., Ann. NY Acad. Sci., 405: 349 (1986)).

The inhibition of thrombin has been implicated as a potential treatment for a number of disease states. A thrombin inhibitor may be useful in the treatment of acute vascular diseases such as such as acute coronary syndromes (for example primary and secondary prevention of myocardial infarction and unstable angina and treatment of prothrombotic sequalae associated with myocardial infarction or heart failure),thromboembolism, acute vessel closure associated with thrombolytic therapy and percutaneous transluminal coronary angioplasty, transient ischemic attacks, pulmonary embolism, deep vein thrombosis, peripheral arterial occlusion, prevention of vessel luminal narrowing (restenosis), and the prevention of thromboembolic events associated with atrial fibrillation, e.g. stroke. Thrombin inhibitors may also have utility as anti-coagulant agents both in-vivo and ex-vivo, and in oedema and inflammation. They may also be useful in preventing thrombosis and complications in patients genetically predisposed to arterial thrombosis or venous thrombosis and patients that have a disease-associated predisposition to thrombosis (e.g. type 2 diabetics). Thrombin has been reported to contribute to lung fibroblast proliferation, thus, thrombin inhibitors could be useful for the treatment of some pulmonary fibrotic diseases. Thrombin inhibitors could also be useful the treatment of tumour metastasis, by suppressing coagulation and thus preventing fibrin deposition and its concommittant facilitation of metastasis. A Factor Xa inhibitor may also have utility as an anti-inflammatory agent through its inhibition of FXa mediated activation of protease-activated receptors (PAR 1-4). A Factor Xa inhibitor may also have utility as an anti-atherosclerotic agent through the suppression of platelet-activation. Thrombin can induce neurite retraction and thus thrombin inhibitors may have potential in neurogenerative diseases such as Parkinson's and Alzheimer's disease. They have also been reported for use in conjunction with thrombolytic agents, thus permitting the use of a lower dose of thrombolytic agent. Thrombin inhibitors may also have utility as anticoagulant agents in connection with the preparation, storage, fractionation or use of whole blood.

Patent Applications -WO02/100886 and WO02/100830, incorporated herein by reference, disclose certain FXa inhibitors including (E)-2-(4-chlorophenyl)-N-{(3S)-1-[(1S)-1-methyl-2-morpholin-4-yl-2-oxoethyl]-2-oxopyrrolidin-3-yl}ethenesulfonamide and (E)-2-(5-chlorothien-2-yl)-N-{(3S)-1-[(1S)-1-methyl-2-morpholin-4-yl-2-oxoethyl]-2-oxopyn-olidin-3-yl}ethenesulfonamide.

### Summary of the Invention

The present invention provides compounds of formula (I): 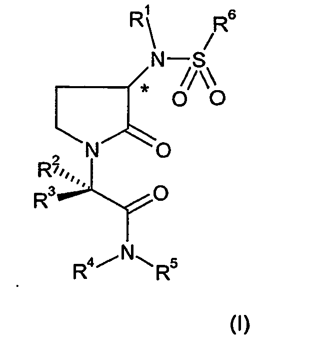 wherein:
R¹ represents hydrogen, C₁₋₄alkyl, -CH₂CO₂H, -CH₂CO₂C₁₋₂alkyl, or -CH₂CONR⁷R⁸;
R² and R³ independently represent hydrogen, -C₁₋₆alkyl, -C₁₋₃alkylCN, -C₁₋₃alkylCO₂H, -C₁₋₄alkylOC₁₋₄alkyl, -C₁₋₄alkylS(O)ₙC₁₋₄alkyl, -C₁₋₄alkylNR¹⁰R¹¹, -C₁₋₃alkylNCO₂C₁₋₄alkyl, -C₁₋₃alkylCONR⁷R⁸, -C₁₋₃alkylCO₂C₀₋₂alkylR⁹, -C₁₋₃alkylCOC₀₋₂alkylR⁹, -C₁₋₃alkylCON(R⁸)C₀₋₂alkylR⁹, -C₁₋₃alkylNCO₂C₀₋₂alkylR⁹, -C₁₋₃alkylNCOC₀₋₂alkylR⁹ or -C₀₋₂alkylR⁹, with the proviso that one of R² and R³ is hydrogen and the other is a substituent other than hydrogen; n is an integer between 0 and 2;
R⁴ and R⁵ together with the nitrogen atom to which they are attached form a morpholino ring;
R⁶ represents a group selected from:

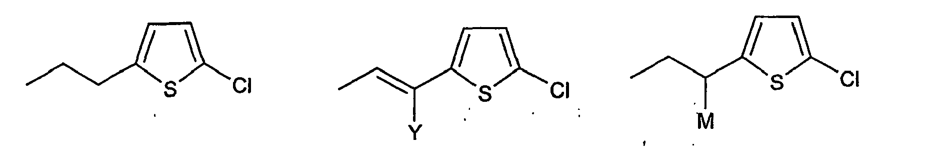

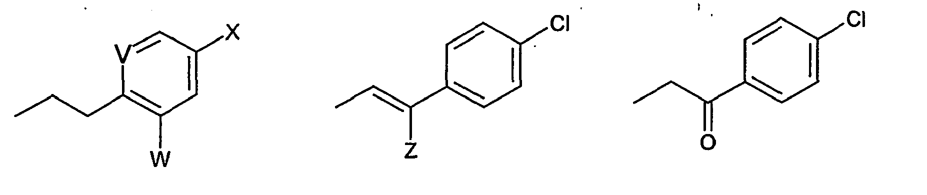

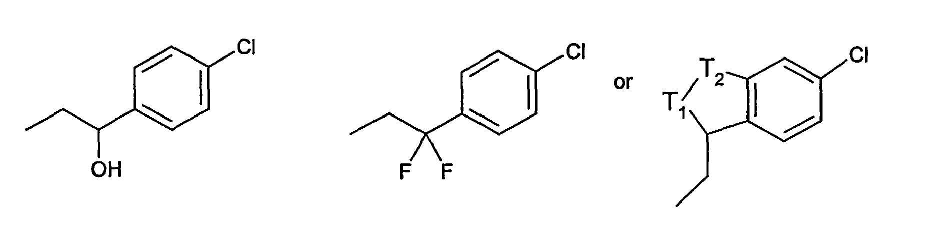

Wherein T₁ and T₂ independently represent CH₂, NH, S or O with the proviso that when one of T₁ or T₂ represents NH, S or O the other represents CH₂;
M represents CH₃, -OH or =O;
V represents CH or N;
W represents H, CH₃, Cl or F;
X represents Cl, Br, F or -CH₃;
Y represents CH₃ or CF₃;
Z represents -CH₃ or F;
R⁷ and R⁸ are independently hydrogen, C₁₋₄alkyl or together with the N atom to which they are bonded form a 5- or 6- membered non-aromatic heterocyclic ring, optionally containing an additional heteroatom selected from O, N or S;
R¹⁰ and R¹¹ independently represent C₁₋₄alkyl or together with the N atom to which they are bonded form a 5- or 6- membered non-aromatic heterocyclic ring, optionally containing an additional heteroatom selected from O, N or S;
R⁹ represents phenyl or a 5- or 6- membered aromatic or non-aromatic heterocyclic group, containing at least one heteroatom selected from O, N or S, each of which is optionally substituted by 0-2 groups selected from: C₁₋₃alkyl or halogen;
and pharmaceutically acceptable derivatives thereof.

Further aspects of the invention are:
- A pharmaceutical composition comprising a compound of the invention together with a pharmaceutical carrier and/or excipient.
- A compound of the invention for use in therapy.
- Use of a compound of the invention for the manufacture of a medicament for the treatment of a patient suffering from a condition susceptible to amelioration by a thrombin inhibitor.

The compounds of the invention show advantageous properties, since they show surprising activity against at thrombin, they may also be more efficacious, have a longer duration of action, be more bioavailable by the preferred route, or have other more desirable properties than similar known compounds.

### Detailed Description of the Invention

The compounds of formula (I) contain chiral (asymmetric) centres. The individual stereoisomers (enantiomers and diastereoisomers) and mixtures of these are within the scope of the present invention. Preferably, at the position marked "*" the stereochemistry is (S)-stereochemistry. Furthermore, some of the crystalline forms of the compounds of structure (I) may exist as polymorphs, which are included in the present invention.

In another aspect, the present invention provides compounds of formula (I): 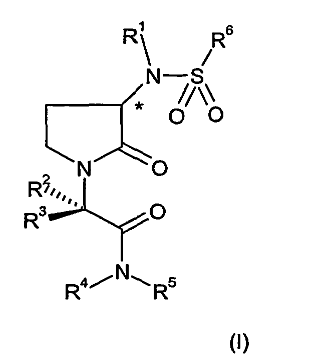 wherein:
R¹ represents hydrogen, methyl, -CH₂CO₂H, -CH₂CO₂C₁₋₂alkyl, or -CH₂CONR⁷R⁸;
R² represents -C₁₋₄alkyl, -CH₂CO₂H, -CH₂OCH₃, -CH(CH₃)OCH₃, -CH₂CON(CH₃)₂, benzyl, -CH₂CO₂-benzyl, -CH₂CO-morpholine, or -CH₂-thiophene;
R³ represents hydrogen;
R⁴ and R⁵ together with the nitrogen atom to which they are attached form a morpholino ring;
R⁶ represents a group selected from: 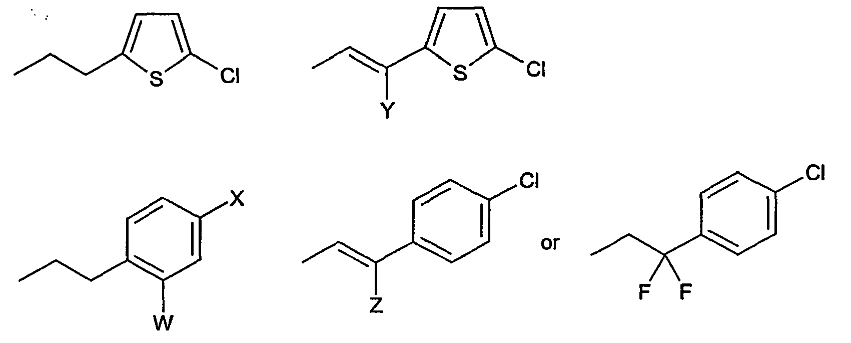
wherein W represents H, Cl or F;
X represents Cl, Br, F or -CH₃;
Y represents CH₃ or CF₃;
Z represents -CH₃ or F;
R⁷ and R⁸ are independently hydrogen or methyl;
and pharmaceutically acceptable derivatives thereof.

Preferably, R¹ represents hydrogen, methyl, -CH₂CO₂C₁₋₂alkyl, or -CH₂CONR⁷R⁸. More preferably R¹ represents hydrogen, methyl or -CH₂CONR⁷R⁸. Most preferably, R¹ represents hydrogen.

Preferably, R² and R³ independently represent -C₁₋₆alkyl, -C₁₋₃alkylCN, -C₁₋₄alkylOC₁₋₄alkyl, -C₁₋₄alkylS(O)ₙC₁₋₄alkyl, -C₁₋₄alkylNR¹⁰R¹¹, -C₁₋₃alkylCONR⁷R^{B}, -C₁₋₃alkylCO₂C₀₋₂alkylR⁹, -C₁₋₃alkylCON(R⁸)C₀₋₂alkylR⁹ or -C₀₋₂alkylR⁹, with the proviso that one of R² and R³ is hydrogen and the other is a substituent other than hydrogen. More preferably, R² and R³ independently represent -C₁₋₆alkyl, -C₁₋₄alkylOC₁₋₄alkyl, - C₁₋₃alkylCO₂C₀₋₂alkylR⁹, or -C₀₋₁alkylR⁹, with the proviso that one of R² and R³ is hydrogen and the other is a substituent other than hydrogen

Preferably, R² represents -C₁₋₆alkyl, -C₁₋₃alkylCN, , -C₁₋₃alkylCO₂H, -C₁₋₄alkylOC₁₋₄alkyl, -C₁₋₄alkylS(O)ₙC₁₋₄alkyl, -C₁₋₄alkylNR¹⁰R¹¹, -C₁₋₃alkylCONR⁷R⁸, -C₁₋₃alkylCON(R⁸)C₀₋₂alkylR⁹, -C₁₋₃alkylCO₂C₀₋₂alkylR⁹ or -C₀₋₂alkylR⁹. More preferably, R² represents -C₁₋₆alkyl, -C₁₋₃alkylCN, , -C₁₋₃alkylCO₂H, -C₁₋₄alkylOC₁₋₄alkyl, -C₁₋₄alkylS(O)ₙC₁₋₄alkyl, -C₁₋₄alkylNR¹⁰R¹¹, -C₁₋₃alkylCONR⁷R⁸, -C₁₋₃alkylCON(R⁸)C₀₋₁alkylR⁹, -C₁₋₃alkylCO₂C₀₋₂alkylR⁹ or -C₀₋₂alkylR⁹. Even more preferably, R² represents -C₂₋₆alkyl, -C₁₋₃alkylCN, , -C₁₋₃alkylCO₂H, -C₁₋₄alkylOC₁₋₄alkyl, -C₁₋₄alkylS(O)ₙC₁₋₄alkyl, -C₁₋₄alkylNR¹⁰R¹¹, -C₁₋₃alkylCONR⁷R⁸, -C₁₋₃alkylCON(R⁸)C₀₋₁alkylR⁹, -C₁₋₃alkylCO₂C₀₋₂alkylR⁹ or -C₀₋₂alkylR⁹. Most preferably, R² represents ethyl, 2-propyl, i-butyl, s-butyl, -CH₂CO₂H, -CH₂OCH₃, -CH(CH₃)OCH₃, - CH₂CON(CH₃)₂, benzyl, -CH₂CO₂-benzyl, -CH₂CO-morpholine where the morpholine group is N-linked to the rest of the molecule, or -CH₂-thiophene.

In another preferred aspect, R² represents s-butyl , CH₃OCH₂- CH₃CH₂OCH₂-, (CH₃)₂CHOCH₂-, CH₃OCH(CH₃)-, (CH₃)₂NCH₂CH₂-, 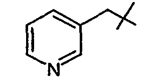 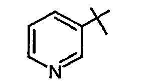 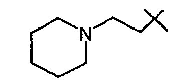 or

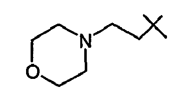

In an another preferred aspect, R² represents methyl, ethyl, 2-propyl, i-butyl, s-butyl, -CH₂CO₂H, -CH₂OCH₃, -CH(CH₃)OCH₃, -CH₂CON(CH₃)₂, benzyl, -CH₂CO₂-benzyl, - CH₂CO-morpholine, or -CH₂-thiophene.

Preferably, R³ represents hydrogen.

Preferably, T₁ and T₂ independently represent CH₂ or O, with the proviso that when one of T₁ or T₂ represents O the other represents CH₂. More preferably, T₁ represents CH₂ and T₂ represents CH₂ or O.

Preferably, M represents CH₃.

Preferably, X represents Cl, Br or -CH₃. More preferably, X represents Cl or Br. Most preferably, X represents Cl.

Preferably, W represents H or Cl. More preferably, W represents H.

Preferably, Y represents -CH₃.

Preferably, Z represents -CH₃.

In one preferred aspect of the invention R⁶ represents a group selected from:

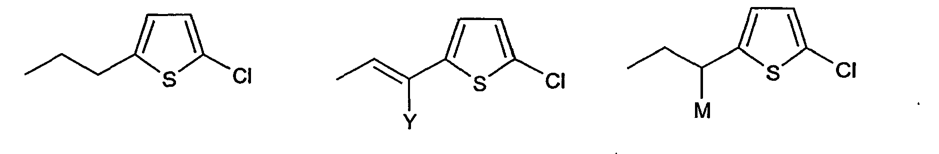

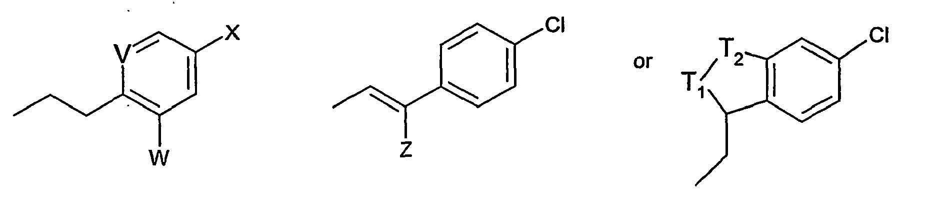

In another preferred aspect of the invention, R⁶ represents a group selected from:

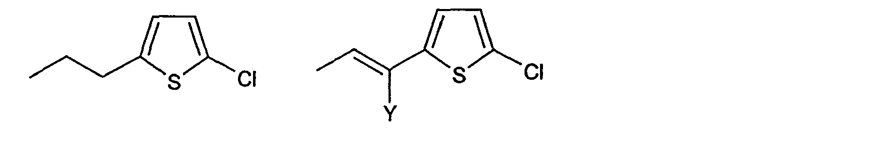

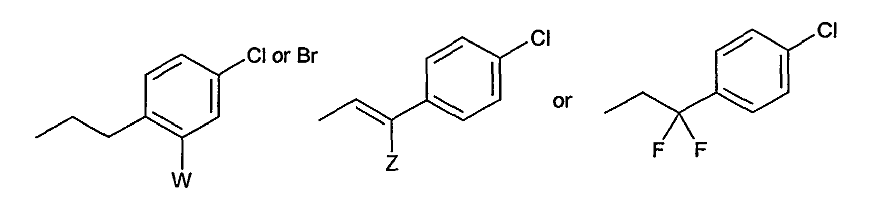

More preferably, R⁶ represents a group selected from:

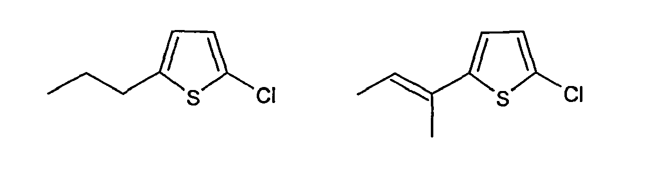

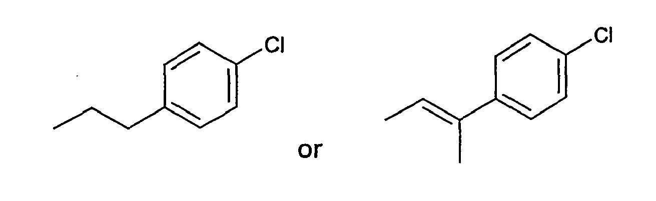

Preferably, R⁹ represents phenyl, thienyl, thiazolyl, oxadiazolyl, triazolyl, pyridyl, piperidinyl or morpholinyl. More preferably, R⁹ represents phenyl, thienyl, thiazolyl, triazolyl, pyridyl or morpholinyl. Most preferably, R⁹ represents phenyl, thienyl or morpholinyl.

Preferably, R¹⁰ and R¹¹ independently represent C₁₋₄alkyl.

It is to be understood that the present invention covers all combinations of preferred groups described hereinabove.

As used herein, the term "thrombin inhibitor" means a compound which possesses thrombin inhibitory activity. Preferably, the thrombin inhibitor has a Ki (nM) value of less than 200, more preferably less than 100, even more preferably less than 50, even more preferably less than 25, most preferably less than 10 when measured in accordance with the assay described hereinbelow. In comparison, prior art compounds (E)-2-(4-chlorophenyl)-N-{(3S)-1-[(1S)-1-methyl-2-morpholin-4-yl-2-oxoethyl]-2-oxopyrrolidin-3-yl}ethenesulfonamide and (E)-2-(5-chlorothien-2-yl)-N-{(3S)-1-[(1S)-1-methyl-2-morpholin-4-yl-2-oxoethyl]-2-oxopyrrolidin-3-yl}ethenesulfonamide have thrombin Ki (nM) values of greater than 200. In a preferred aspect of the invention, the thrombin inhibitor is a "dual thrombin-Factor Xa inhibitor". In another preferred aspect of the invention, the thrombin inhibitor is a "selective thrombin inhibitor".

As used herein, the term "dual thrombin-Factor Xa inhibitor" means a compound which has inhibitory activity at both thrombin and Factor Xa.

As used herein, the term "selective thrombin inhibitor" means a compound which is selective for thrombin over Factor Xa.

The term 'alkyl' as used herein means both straight and branched chain saturated hydrocarbon groups. Examples of alkyl groups include methyl (-CH₃), ethyl (-C₂H₅), propyl (-C₃H₇) and butyl (-C₄H₉).

As used herein, the term "halogen" means an atom selected from fluorine, chlorine, bromine and iodine.

As used herein, the term "heterocyclic group" means optionally substituted rings containing one or more heteroatoms selected from: nitrogen, sulphur and oxygen atoms. The heterocycle may be aromatic or non-aromatic, i.e., may be saturated, partially or fully unsaturated. Examples of 5-membered groups include thienyl, furanyl, pyrrolidinyl, thiazolyl, oxadiazolyl, triazolyl and imidazolyl. Preferred 5-membered groups are thienyl, thiazolyl, oxadiazolyl, triazolyl. Examples of 6-membered groups include pyridyl, piperidinyl and morpholinyl. Preferred 6-membered groups include pyridyl and morpholinyl. Certain heterocyclic groups, e.g. thienyl, thiazolyl and pyridyl are C-linked to the rest of the molecule. Other heterocyclic groups, e.g. piperidinyl and morpholinyl may be C-linked or N-linked to the rest of the molecule.

As used herein, the term "pharmaceutically acceptable" means a compound which is suitable for pharmaceutical use.

As used herein, the term "pharmaceutically acceptable derivative", means any pharmaceutically acceptable salt, solvate, or prodrug e.g. ester or carbamate, or salt or solvate of such a prodrug, of a compound of formula (I), which upon administration to the recipient is capable of providing (directly or indirectly) a compound of formula (I), or an active metabolite or residue thereof. Such derivatives are clear to those skilled in the art, without undue experimentation, and with reference to the teaching of Burger's Medicinal Chemistry And Drug Discovery, 5th Edition, Vol 1: Principles And Practice, which is incorporated herein by reference. Preferred pharmaceutically acceptable derivatives are salts and solvates.

Suitable salts according to the invention include those formed with both organic and inorganic acids and bases. Pharmaceutically acceptable acid addition salts include those formed from mineral acids such as: hydrochloric, hydrobromic, sulphuric, phosphoric, acid; and organic acids such as: citric, tartaric, lactic, pyruvic, acetic, trifluoroacetic, succinic, oxalic, formic, fumaric, maleic, oxaloacetic, methanesulphonic, ethanesulphonic, p-toluenesulphonic, benzenesulphonic and isethionic acids. Pharmaceutically acceptable base salts include ammonium salts, alkali metal salts such as those of sodium and potassium, alkaline earth metal salts such as those of calcium and magnesium and salts with organic bases, including salts of primary, secondary and tertiary amines, such as isopropylamine, diethylamine, ethanolamine, trimethylamine, dicyclohexyl amine and N-methyl-D-glucamine. Particularly preferred pharmaceutically acceptable salts include those formed from hydrochloric, trifluoroacetic and formic acids.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of formula (I) are within the scope of the invention.

Salts and solvates of compounds of formula (I) which are suitable for use in medicine are those wherein the counterion or associated solvent is pharmaceutically acceptable. However, salts and solvates having non-pharmaceutically acceptable counterions or associated solvents are within the scope of the present invention, for example, for use as intermediates in the preparation of other compounds of formula (I) and their pharmaceutically acceptable derivatives.

As used herein, the term "prodrug" means a compound which is converted within the body, e.g. by hydrolysis in the blood, into its active form that has medical effects. Pharmaceutically acceptable prodrugs are described in T. Higuchi and V. Stella, Prodrugs as Novel Delivery Systems, Vol. 14 of the A.C.S. Symposium Series, Edward B. Roche, ed., Bioreversible Carriers in Drug Design, American Pharmaceutical Association and Pergamon Press, 1987, and in D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

Prodrugs are any covalently bonded carriers that release a compound of structure (I) in vivo when such prodrug is administered to a patient. Prodrugs are generally prepared by modifying functional groups in a way such that the modification is cleaved, either by routine manipulation or in vivo, yielding the parent compound. Prodrugs include, for example, compounds of this invention wherein hydroxyl, amine or carboxylic acid groups are bonded to any group that, when administered to a patient, cleaves to form the hydroxyl, amine or carboxylic acid groups.

Preferred compounds of the invention include:
2-(5-Chlorothien-2-yl)-N-{(3S)-1-[(1S)-1-methyl-2-morpholin-4-yl-2-oxoethyl]-2-oxopyrrolidin-3-yl}ethanesulfonamide;
(1 E)-2-(5-Chlorothien-2-yl)-N-{(3S)-1-[(1S)-1-methyl-2-morpholin-4-yl-2-oxoethyl]-2-oxopyrrolidin-3-yl}prop-1-ene-1-sulfonamide;
(1 E)-2-(4-Chlorophenyl)-N-{(3S)-1-[(1S)-1-methyl-2-morpholin-4-yl-2-oxoethyl]-2-oxopyrrolidin-3-yl}prop-1-ene-1-sulfonamide;
2-(4-Chlorophenyl)-N-{(3S)-1-[(1S)-1-methyl-2-morpholin-4-yl-2-oxoethyl]-2-oxopyrrolidin-3-yl}ethanesulfonamide;
2-(5-Chlorothien-2-yl)-N-{(3S)-1-[(1 S)-1-(morpholin-4-ylcarbonyl)propyl]-2-oxopyrrolidin-3-yl}ethanesulfonamide;
2-(4-Chlorophenyl)-N-{(3S)-1-[(1 S)-1-(morpholin-4-ylcarbonyl)propyl]-2-oxopyrrolidin-3-yl}ethanesulfonamide;
(1 E)-2-(5-Chlorothien-2-yl)-N-{(3S)-1-[(1 S)-1-(morpholin-4-ylcarbonyl)propyl]-2-oxopyrrolidin-3-yl}prop-1-ene-1-sulfonamide;
2-(5-Chlorothien-2-yl)-N-((3S)-1-[(1S)-2-methyl-1-(morpholin-4-ylcarbonyl)propyl]-2-oxopyrrolidin-3-yl)ethanesulfonamide;
(1 E)-2-(5-Chlorothien-2-yl)-N-{(3S)-1-[(1 S)-2-methyl-1-(morpholin-4-ylcarbonyl)propyl]-2-oxopyrrolidin-3-yl}prop-1-ene-1-sulfonamide;
2-(5-Chlorothien-2-yl)-N-{(3S)-1-[(1S,2S)-2-methyl-1-(morpholin-4-ylcarbonyl)butyl]-2-oxopyrrolidin-3-yl}ethanesulfonamide;
(1E)-2-(5-Chlorothien-2-yl)-N-{(3S)-1-[(1S,2S)-2-methyl-1-(morpholin-4-ylcarbonyl)butyl]-2-oxopyrrolidin-3-yl}prop-1-ene-1-sulfonamide;
2-(4-Bromophenyl)-N-{(3S)-1-[(1S,2S)-2-methyl-1-(morpholin-4-ylcarbonyl)butyl]-2-oxopyrrolidin-3-yl}ethanesulfonamide;
N-{(3S)-1-[(1S)-1-Benzyl-2-morpholin-4-yl-2-oxoethyl]-2-oxopyrrolidin-3-yl}-2-(5-chlorothien-2-yl)ethanesulfonamide;
(1 E)-N-{(3S)-1-[(1S)-1-Benzyl-2-morpholin-4-yl-2-oxoethyl]-2-oxopyrrolidin-3-yl}-2-(5-chlorothien-2-yl)prop-1-ene-1-sulfonamide;
2-(5-Chlorothien-2-yl)-N-{(3S)-1-[(1S)-1-(methoxymethyl)-2-morpholin-4-yl-2-oxoethyl]-2-oxopyrrolidin-3-yl}ethanesulfonamide;
(1E)-2-(5-Chlorothien-2-yl)-N-{(3S)-1-[(1S)-1-(methoxymethyl)-2-morpholin-4-yl-2-oxoethyl]-2-oxopyrrolidin-3-yl}prop-1-ene-1-sulfonamide;
2-(5-Chlorothien-2-yl)-N-{(3S)-1-[(1S,2R)-2-methoxy-1-(morpholin-4-ylcarbonyl)propyl]-2-oxopyrrolidin-3-yl}ethanesulfonamide;
(1E)-2-(5-Chlorothien-2-yl)-N-{(3S)-1-[(1S,2R)-2-methoxy-1-(morpholin-4-ylcarbonyl)propyl]-2-oxopyrrolidin-3-yl}prop-1-ene-1-sulfonamide;
2-(5-Chlorothien-2-yl)-*N*-methyl-*N*-{(3*S*)-1-[(1*S*)-1-methyl-2-morpholin-4-yl-2-oxoethyl]-2-oxopyrrolidin-3-yl}ethanesulfonamide;
*N*²-{[2-(5-Chlorothien-2-yl)ethyl]sulfonyl}-*N*²-{(3S)-1-[(1*S*)-1-methyl-2-morpholin-4-yl-2-oxoethyl]-2-oxopyrrolidin-3-yl}glycinamide;
Benzyl (3S)-3-[(3S)-3-({[2-(5-chlorothien-2-yl)ethyl]sulfonyl}amino)-2-oxopyrrolidin-1-yl]-4-morpholin-4-yl-4-oxobutanoate;
Benzyl (3*S*)-3-[(3*S*)-3-({[(1*E*)-2-(5-Chlorothien-2-yl)prop-1-enyl]sulfonyl}amino)-2-oxopyrrolidin-1-yl]-4-morpholin-4-yl-4-oxobutanoate;
2-(5-Chlorothien-2-yl)-*N*-{(3*S*)-1-[(1*S*)-2-morpholin-4-yl-2-oxo-1-(thien-2-ylmethyl)ethyl]-2-oxopyrrolidin-3-yl}ethanesulfonamide;
2-(4-Chlorophenyl)-N-{(3*S*)-1-[(1*S*)-2-morpholin-4-yl-2-oxo-1-(thien-2-ylmethyl)ethyl]-2-oxopyrrolidin-3-yl}ethanesulfonamide;
2-(4-Chloro-2-fluorophenyl)-N-{(3*S*)-1-[(1S)-1-methyl-2-morpholin-4-yl-2-oxoethyl]-2-oxopyrrolidin-3-yl}ethanesulfonamide;
2-(4-Bromophenyl)-N-{(3S)-1-[(1S)-1-(morpholin-4-ylcarbonyl)propyl]-2-oxopyrrolidin-3-yl}ethanesulfonamide;
2-(4-Chlorophenyl)-2,2-difluoro-*N*-{(3*S*)-1-[(1*S*)-1-methyl-2-morpholin-4-yl-2-oxoethyl]-2-oxopyrrolidin-3-yl}ethanesulfonamide;
(*Z*)-2-(4-Chlorophenyl)-2-fluoro-*N*-{(3*S*)-1-[(1*S*)-1-methyl-2-morpholin-4-yl-2-oxoethyl]-2-oxopyrrolidin-3-yl}ethenesulfonamide;
2-(4-Chlorophenyl)-2,2-difluoro-*N*-{(3*S*)-1-[(1*S*,2*S*)-2-methyl-1-(morpholin-4-ylcarbonyl)butyl]-2-oxopyrrolidin-3-yl}ethanesulfonamide;
(*Z*)-2-(4-Chlorophenyl)-2-fluoro-*N*-{(3*S*)-1-[(1*S*,2*S*)-2-methyl-1-(morpholin-4-ylcarbonyl)butyl]-2-oxopyrrolidin-3-yl}ethenesulfonamide;
(1*E*)-2-(5-Chlorothien-2-yl)-*N*-{(3*S*)-1-[(1*S*)-3-morpholin-4-yl-1-(morpholin-4-ylcarbonyl)-3-oxopropyl]-2-oxopyrrolidin-3-yl}prop-1-ene-1-sulfonamide;
(3*S*)-3-[(3*S*)-3-({[(1*E*)-2-(5-Chlorothien-2-yl)prop-1-enyl]sulfonyl}amino)-2-oxopyrrolidin-1-yl]-*N*,*N*-dimethyl-4-morpholin-4-yl-4-oxobutanamide;
2-(4-Bromophenyl)-N-{(3S)-1-[(1S)-1-methyl-2-morpholin-4-yl-2-oxoethyl]-2-oxopyrrolidin-3-yl}ethanesulfonamide;
Ethyl *N*-{[2-(5-chlorothien-2-yl)ethyl]sulfonyl}-*N*-{(3*S*)-1-[(1*S*)-1-methyl-2-morpholin-4-yl-2-oxoethyl]-2-oxopyrrolidin-3-yl}glycinate;
Methyl *N*-{[2-(5-chlorothien-2-yl)ethyl]sulfonyl}-N-{(3*S*)-1-[(1*S*)-1-methyl-2-morpholin-4-yl-2-oxoethyl]-2-oxopyrrolidin-3-yl}glycinate;
*N*-{[2-(5-Chlorothien-2-yl)ethyl]sulfonyl}-*N*-{(3*S*)-1-[(1*S*)-1-methyl-2-morpholin-4-yl-2-oxoethyl]-2-oxopyrrolidin-3-yl}glycine;
2-(5-Chlorothien-2-yl)-*N*-{(3*S*)-1-[(1*S*)-3-methyl-1-(morpholin-4-ylcarbonyl)butyl]-2-oxopyrrolidin-3-yl}ethanesulfonamide;
2-(5-Chlorothien-2-yl)-N-methyl-*N* -{(3S)-1-[(1S,2S)-2-methyl-1-(morpholin-4-ylcarbonyl)butyl]-2-oxopyrrolidin-3-yl}ethanesulfonamide;
*N*²-{[2-(5-Chlorothien-2-yl)ethyl]sulfonyl}-*N*¹-methyl-*N*²-{(3*S*)-1-[(1*S*,2*S*)-2-methyl-1-(morpholin-4-ylcarbonyl)butyl]-2-oxopyrrolidin-3-yl}glycinamide;
*N*²-{[2-(5-Chlorothien-2-yl)ethyl]sulfonyl}-*N*¹,*N*¹-dimethyl-*N*²-{(3*S*)-1-[(1*S*,2*S*)-2-methyl-1-(morpholin-4-ylcarbonyl)butyl]-2-oxopyrrolidin-3-yl}glycinamide;
(1*E*)-2-(5-Chlorothien-2-yl)-3,3,3-trifluoro-*N*-{(3*S*)-1-[(1*S*)-1-methyl-2-morpholin-4-yl-2-oxoethyl]-2-oxopyrrolidin-3-yl}prop-1-ene-1-sulfonamide;
2-(2,4-Dichlorophenyl)-N-{(3S)-1-[(1 S)-1-methyl-2-morpholin-4-yl-2-oxoethyl]-2-oxopyrrolidin-3-yl}ethanesulfonamide;
2-(4-Fluorophenyl)-N-{(3S)-1-[(S)-1-methyl-2-morpholin-4-yl-2-oxoethyl]-2-oxopyrrolidin-3-yl}ethanesulfonamide;
2-(4-Methylphenyl)-N-{(3S)-1-[(1 S)-1-methyl-2-morpholin-4-yl-2-oxoethyl]-2-oxopyrrolidin-3-yl}ethanesulfonamide;
2-(4-Chlorophenyl)-N-{(3S)-1-[(1 S)-2-methyl-1-(morpholin-4-ylcarbonyl)propyl]-2-oxopyrrolidin-3-yl}ethanesulfonamide;
(3*S*)-3-[(3*S*)-3-({[2-(5-Chlorothien-2-ethane]sulfonyl}amino)-2-oxopyrrolidin-1-yl]-4-morpholin-4-yl-4-oxobutanoic acid;
2-(5-Chloro-2-pyridinyl)-*N*-{(3S)-1-[(1*S*)-1-methyl-2-(4-morpholinyl)-2-oxoethyl]-2-oxo-3-pyrrolidinyl}ethanesulfonamide;
2-(5-Chloro-2-pyridinyl)-*N*-{(3*S*)-1-[(1*S*,2*S*)-2-methyl-1-(4-morpholinylcarbonyl)butyl]-2-oxo-3-pyrrolidinyl}ethanesulfonamide;
2-(5-Chloro-2-pyridinyl)-*N*-{(3*S*)-1-[(1*S*)-1-[(ethyloxy)methyl]-2-(4-morpholinyl)-2-oxoethyl]-2-oxo-3-pyrrolidinyl}ethanesulfonamide;
2-(5-Chloro-2-pyridinyl)-*N*-{(3*S*)-1-[(1*S*)-1-[(methyloxy)methyl]-2-(4-morpholinyl)-2-oxoethyl]-2-oxo-3-pyrrolidinyl}ethanesulfonamide;
2-(4-Chlorophenyl)-*N*-{(3*S*)-1-[(1*S*,2*S*)-2-methyl-1-(4-morpholinylcarbonyl)butyl]-2-oxo-3-pyrrolidinyl}-2-oxoethanesulfonamide;
2-(4-Chlorophenyl)-*N*-{(3*S*)-1-[(1*S*)-1-(methyloxy)-2-(4-morpholinyl)-2-oxoethyl]-2-oxo-3-pyrrolidinyl}-2-oxoethanesulfonamide;
2-(4-Chlorophenyl)-2-hydroxy-*N*-{(3*S*)-1-[(1*S*,2*S*)-2-methyl-1-(4-morpholinylcarbonyl)butyl]-2-oxo-3-pyrrolidinyl}ethanesulfonamide;
2-(5-Chloro-2-thienyl)-*N*-{(3*S*)-1-[(1*S*)-1-methyl-2-(4-morpholinyl)-2-oxoethyl]-2-oxo-3-pyrrolidinyl}-1-propanesulfonamide;
(2*R*)-2-(5-Chloro-2-thienyl)-*N*-{(3*S*)-1-[(1*S*)-1-methyl-2-(4-morpholinyl)-2-oxoethyl]-2-oxo-3-pyrrolidinyl}-1-propanesulfonamide;
(2*S*)-2-(5-Chloro-2-thienyl)-*N*-{(3*S*)-1-[(1*S*)-1-methyl-2-(4-morpholinyl)-2-oxoethyl]-2-oxo-3-pyrrolidinyl}-1-propanesulfonamide;
2-(5-Chloro-2-thienyl)-*N*-{(3*S*)-1-[(1*S*,2*S*)-2-methyl-1-(4-morpholinyicarbonyl)butyl]-2-oxo-3-pyrrolidinyl}-1-propanesulfonamide;
2-(5-Chloro-2-thienyl)-*N*-{(3*S*)-1-[(1*S*)-1-[(methyloxy)methyl]-2-(4-morpholinyl)-2-oxoethyl]-2-oxo-3-pyrrolidinyl}-1-propanesulfonamide;
2-(5-Chloro-2-thienyl)-*N*-{(3*S*)-1-[(1*S*)-1-[(ethyloxy)methyl]-2-(4-morpholinyl)-2-oxoethyl]-2-oxo-3-pyrrolidinyl}-1-propanesulfonamide;
2-(5-Chloro-2-thienyl)-*N*-{(3*S*)-1-[(1*S*)-1-[(3-methyl-1,2,4-oxadiazol-5-yl)methyl]-2-(4-morpholinyl)-2-oxoethyl]-2-oxo-3-pyrrolidinyl}-1-propanesulfonamide;
1-(5-Chloro-2,3-dihydro-1*H*-inden-1-yl)-*N*-{(3*S*)-1-[(1*S*)-1-methyl-2-(4-morpholinyl)-2-oxoethyl]-2-oxo-3-pyrrolidinyl}methanesulfonamide;
1-(5-Chloro-2,3-dihydro-1*H*-inden-1-yl)-*N*-{(3*S*)-1-[(1*S*)-1-[(methyloxy)methyl]-2-(4-morpholinyl)-2-oxoethyl]-2-oxo-3-pyrrolidinyl}methanesulfonamide;
1-(5-Chloro-2,3-dihydro-1*H*-inden-1-yl)-*N*-{(3*S*)-1-[(1*S*,2*S*)-2-methyl-1-(4-morpholinylcarbonyl)butyl]-2-oxo-3-pyrrolidinyl}methanesulfonamide;
1-(6-Chloro-2,3-dihydro-1-benzofuran-3-yl)-*N*-{(3*S*)-1-[(1*S*)-1-methyl-2-(4-morpholinyl)-2-oxoethyl]-2-oxo-3-pyrrolidinyl}methanesulfonamide;
1-(6-Chloro-2,3-dihydro-1-benzofuran-3-yl)-*N*-{(3*S*)-1-[(1*S*)-1-[(methyloxy)methyl]-2-(4-morpholinyl)-2-oxoethyl]-2-oxo-3-pyrrolidinyl}methanesulfonamide;
1-(5-Chloro-1,3-dihydro-2-benzofuran-1-yl)-*N*-{(3*S*)-1-[(1*S*)-1-methyl-2-(4-morpholinyl)-2-oxoethyl]-2-oxo-3-pyrrolidinyl}methanesulfonamide;
2-(5-Chloro-2-thienyl)-*N*-{(3*S*)-1-[2-(4-morpholinyl)-2-oxo-1-(tetrahydro-2*H*-pyran-4-yl)ethyl]-2-oxo-3-pyrrolidinyl}ethanesulfonamide;
1-[(1*R*)-5-Chloro-2,3-dihydro-1*H*-inden-1-yl]-*N*-{(3*S*)-1-[(1*S*)-1-[(methyloxy)methyl]-2-(4-morpholinyl)-2-oxoethyl]-2-oxo-3-pyrrolidinyl}methanesulfonamide;
2-(5-Chloro-2-thienyl)-*N*-{(3*S*)-1-[(1*S*)-2-(4-morpholinyl)-2-oxo-1-phenylethyl]-2-oxo-3-pyrrolidinyl}ethanesulfonamide;
2-(5-Chloro-2-thienyl)-*N*-{(3*S*)-1-[(1*S*)-1-[(4-fluorophenyl)methyl]-2-(4-morpholinyl)-2-oxoethyl]-2-oxo-3-pyrrolidinyl}ethanesulfonamide;
2-(5-Chloro-2-thienyl)-*N*-{(3*S*)-1-[(1*S*)-2-(4-morpholinyl)-2-oxo-1-(1,3-thiazol-4-ylmethyl)ethyl]-2-oxo-3-pyrrolidinyl}ethanesulfonamide;
2-(4-Chlorophenyl)-N-{(3*S*)-1-[(1*S*)-2-(4-morpholinyl)-2-oxo-1-(1,3-thiazol-4-ylmethyl)ethyl]-2-oxo-3-pyrrolidinyl}ethanesulfonamide;
2-(5-Chloro-2-thienyl)-*N*-{(3*S*)-1-[(1*R*)-2-(4-morpholinyl)-2-oxo-1-(2-thienyl)ethyl]-2-oxo-3-pyrrolidinyl}ethanesulfonamide;
2-(4-Chlorophenyl)-*N*-{(3*S*)-1-[(1*R*)-2-(4-morpholinyl)-2-oxo-1-(2-thienyl)ethyl]-2-oxo-3-pyrrolidinyl}ethanesulfonamide;
2-(5-Chloro-2-thienyl)-*N*-{(3*S*)-1-[(1*R*)-1-[(ethylthio)methyl]-2-(4-morpholinyl)-2-oxoethyl]-2-oxo-3-pyrrolidinyl}ethanesulfonamide;
2-(5-Chloro-2-thienyl)-*N*-{(3*S*)-1*-*[(1*S*)-1-(cyanomethyl)-2-(4-morpholinyl)-2-oxoethyl]-2-oxo-3-pyrrolidinyl}ethanesulfonamide;
2-(5-Chloro-2-thienyl)-*N*-{(3*S*)-1-[(1*S*)-2-(4-morpholinyl)-1-(1,2,4-oxadiazol-5-ylmethyl)-2-oxoethyl]-2-oxo-3-pyrrolidinyl}ethanesulfonamide;
2-(5-Chloro-2-thienyl)-*N*-{(3*S*)-1-[(1*S*)-1-[(3-methyl-1,2,4-oxadiazol-5-yl)methyl]-2-(4-morpholinyl)-2-oxoethyl]-2-oxo-3-pyrrolidinyl}ethanesulfonamide;
2-(5-Chloro-2-thienyl)-*N*-methyl-*N*-{(3*S*)-1-[(1*S*)-1-[(3-methyl-1,2,4-oxadiazol-5-yl)methyl]-2-(4-morpholinyl)-2-oxoethyl]-2-oxo-3-pyrrolidinyl}ethanesulfonamide;
2-(5-Chloro-2-thienyl)-*N*-{(3*S*)-1-[(1*S*)-1-[(1-methyl-1*H*-1,2,4-triazol-3-yl)methyl]-2-(4-morpholinyl)-2-oxoethyl]-2-oxo-3-pyrrolidinyl}ethanesulfonamide;
2-(5-Chloro-2-thienyl)-*N*-{(3*S*)-1-[(1*S*)-1-[(1-methyl-1*H*-1,2,4-triazol-5-yl)methyl]-2-(4-morpholinyl)-2-oxoethyl]-2-oxo-3-pyn-olidinyl}ethanesulfonamide;
2-(4-Chlorophenyl)-*N*-{(3*S*)-1-[(1*S*)-1-[(3-methyl-1,2,4-oxadiazol-5-yl)methyl]-2-(4-morpholinyl)-2-oxoethyl]-2-oxo-3-pyrrolidinyl}ethanesulfonamide;
(3*S*)-3-[(3*S*)-3-({[2-(5-Chloro-2-thienyl)ethyl]sulfonyl}amino)-2-oxo-1-pyrrolidinyl]-*N-*methyl-4-(4-morpholinyl)-4-oxo-*N*-(phenylmethyl)butanamide;
2-(5-Chloro-2-thienyl)-*N*-{(3*S*)-1-[(1*S*)-1-{[(1-methylethyl)oxy]methyl}-2-(4-morpholinyl)-2-oxoethyl]-2-oxo-3-pyrrolidinyl}ethanesulfonamide;
2-(5-Chloro-2-thienyl)-*N*-{(3*S*)-1-[(1*S*)-1-[(ethyloxy)methyl]-2-(4-morpholinyl)-2-oxoethyl]-2-oxo-3-pyrrolidinyl}ethanesulfonamide;
2-(4-Chlorophenyl)-*N*-{(3*S*)-1-[(1*S*)-1-[(ethyloxy)methyl]-2-(4-morpholinyl)-2-oxoethyl]-2-oxo-3-pyrrolidinyl}ethanesulfonamide;
(1*E*)-2-(5-Chloro-2-thienyl)-*N*-{(3*S*)-1-[(1*S*)-1-[(ethyloxy)methyl]-2-(4-morpholinyl)-2-oxoethyl]-2-oxo-3-pyrrolidinyl}-1-propene-1-sulfonamide;
2-(5-Chloro-2-thienyl)-*N*-{(3*S*)-1-[(1*S*)-3-(methyloxy)-1-(4-morpholinylcarbonyl)propyl]-2-oxo-3-pyrrolidinyl}ethanesulfonamide;
2-(4-Chlorophenyl)-*N*-{(3*S*)-1-[(1S)-1-[(methyloxy)methyl]-2-(4-morpholinyl)-2-oxoethyl]-2-oxo-3-pyrrolidinyl}ethanesulfonamide;
2-(5-Chloro-2-thienyl)-*N*-{(3*S*)-1-[(1*S*)-2-(4-morpholinyl)-2-oxo-1-(4-pyridinylmethyl)ethyl]-2-oxo-3-pyrrolidinyl}ethanesulfonamide;
2-(5-Chloro-2-thienyl)-*N*-{(3*S*)-1-[(1*S*)-2-(4-morpholinyl)-2-oxo-1-(3-pyridinylmethyl)ethyl]-2-oxo-3-pyrrolidinyl}ethanesulfonamide;
2-(5-Chloro-2-thienyl)-*N*-{(3*S*)-1-[2-(4-morpholinyl)-2-oxo-1-(3-pyridinyl)ethyl]-2-oxo-3-pyrrolidinyl}ethanesulfonamide;
2-(5-Chloro-2-thienyl)-*N*-{(3*S*)-1-[(1*S*)-3-(dimethylamino)-1-(4-morpholinylcarbonyl)propyl]-2-oxo-3-pyrrolidinyl}ethanesulfonamide;
2-(5-Chloro-2-thienyl)-*N*-{(3*S*)-1-[(1*S*)-1-(4-morpholinylcarbonyl)-3-(1-piperidinyl)propyl]-2-oxo-3-pyrrolidinyl}ethanesulfonamide;
2-(5-Chloro-2-thienyl)-*N*-{(3*S*)-1-[(1*S*)-3-(4-morpholinyl)-1-(4-morpholinylearbonyl)propyl]-2-oxo-3-pyrrolidinyl}ethanesulfonamide;
(1*E*)-2-(5-Chloro-2-thienyl)-*N*-{(3*S*)-1-[(1*S*)-3-(4-morpholinyl)-1-(4-morpholinylcarbonyl)propyl]-2-oxo-3-pyrrolidinyl}-1-propene-1-sulfonamide;
*N*²-{[2-(5-Chloro-2-thienyl)ethyl]sulfonyl}-*N*¹,*N*¹-dimethyl-*N*²-{(3*S*)-1-[(1*S*)-1-[(methyloxy)methyl]-2-(4-morpholinyl)-2-oxoethyl]-2-oxo-3-pyrrolidinyl}glycinamide;
*N*²-{[2-(5-Chloro-2-thienyl)ethyl]sulfonyl}-*N*¹-methyl-*N*²-{(3*S*)-1-[(1*S*)-1-[(methyloxy)methyl]-2-(4-morpholinyl)-2-oxoethyl]-2-oxo-3-pyrrolidinyl}glycinamide;
2-(4-Chlorophenyl)-*N*-{(3*S*)-1-[(1*S*)-3-(methylsulfonyl)-1-(4-morpholinylcarbonyl)propyl]-2-oxo-3-pyrrolidinyl}ethanesulfonamide;
and pharmaceutically acceptable derivatives thereof.

In the following preferred aspects of the invention, the Example numbers correspond to the Example numbers in the Experimental section.

In a preferred aspect, the compounds of the invention are Examples 1, 2, 3, 4, 5, 6, 7, 8, 9, 10, 11, 12, 13, 14, 15, 16, 17, 18, 19, 20,21, 22, 23, 24, 25, 26, 27, 28, 29, 30, 31, 32, 33, 34, 35, 36, 37, 38, 39, 40, 41, 42, 45, 46, 47, 48, 49, 50, 51, 52, 53, 54, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99. In a more preferred aspect, the compounds of the invention are Examples 1, 2, 3, 4, 5, 6, 7, 8, 9, 10, 11, 12, 13, 14, 15, 16, 17, 18, 19, 20,21, 22, 23, 24, 26, 27, 28, 29, 32, 33, 34, 35, 36, 37, 38, 39, 40, 41, 45, 47, 48, 49, 51, 52, 53, 54, 57, 58, 59, 60, 61, 63, 64, 65, 66, 68, 69, 70, 71, 73, 74, 75, 76, 77, 78, 79, 80, 81, 82, 83, 84, 85, 86, 87, 88, 89, 90, 91, 92, 93, 94, 95, 96, 97, 98. In an even more preferred aspect, the compounds of the invention are Examples 1, 2, 3, 4, 5, 7, 8, 9, 10, 11, 12, 13, 14, 15, 16, 17, 18, 19, 20,21, 22, 23, 24, 27, 28, 29, 32, 33, 34, 35, 38, 39, 40, 45, 48, 49, 51, 52, 57, 59, 60, 63, 66, 68, 70, 71, 73, 75, 77, 78, 79, 80, 83, 84, 85, 86, 87, 88, 89, 90, 91, 92, 96, 97, 98. In an even more preferred aspect, the compounds of the invention are Examples 2, 3, 5, 7, 8, 9, 10, 11, 12, 13, 14, 16, 17, 18, 20,21, 22, 23, 24, 27, 28, 29, 33, 34, 35, 38, 39, 40, 48, 49, 57, 59,77, 78, 84, 85, 86, 87, 88, 91, 98. In a most preferred aspect, the compounds of the invention are Examples 2, 7, 8, 9, 10, 11, 14, 16, 17, 18, 21, 22, 23, 38, 40, 77, 84, 85, 87, 91, 98.

In another preferred aspect of the invention, the compounds of the invention are Examples 2, 3, 19, 20, 25, 28, 34, 35, 36, 41, 42, 47, 54, 61, 94, 96. In another preferred aspect of the invention, the compounds of the invention are Examples 2, 20, 34, 35, 36, 41, 42, 94, 96.

Thrombin inhibitory activity is measured by the ability to inhibit human α-thrombin in a chromogenic assay using N-p-tosyl-gly-pro-lys p-nitroanilide as the chromogenic substrate, or in a fluorogenic assay using Rhodamine 110, bis-(CBZ-L-valyl-L-prolyl-L-arginine amide) as the fluorogenic substrate.

Factor Xa inhibitory activity is measured by the ability to inhibit human Factor Xa in a chromogenic assay using N-α-benzyloxycarbonyl-D-Arg-Gly-Arg-p-nitroanilide as the chromogenic substrate, or in a fluorogenic assay using Rhodamine 110, bis-(CBZ-glycylglycyl-L-arginine amide as the fluorogenic substrate.

Furthermore, compounds of formula (I) exhibit effective anti-coagulant activity in vitro as indicated by the APTT assays described in the Examples below.

Thus, the compounds of formula (I) are useful in the treatment of clinical conditions susceptible to amelioration by administration of a thrombin inhibitor. Such conditions include acute vascular diseases such as coronary thrombosis (for example myocardial infarction and unstable angina), thromboembolism, acute vessel closure associated with thrombolytic therapy and percutaneous transluminal coronary angioplasty (PTCA), transient ischemic attacks, pulmonary embolism, deep vein thrombosis, peripheral arterial occlusion, prevention of vessel luminal narrowing (restenosis), and the prevention of thromboembolic events associated with atrial fibrillation, e.g. stroke; treatment of ischemic stroke; in oedema and PAF mediated inflammatory diseases such as adult respiratory shock syndrome, septic shock and reperfusion damage; the treatment of pulmonary fibrosis; the treatment of tumour metastasis; neurogenerative disease such as Parkinson's and Alzheimer's diseases; viral infection; Kasabach Merritt Syndrome; Haemolytic uremic syndrome; arthritis; osteoporosis; as anti-coagulants for extracorporeal blood in for example, dialysis, blood filtration, bypass, and blood product storage; and in the coating of invasive devices such as prostheses, artificial valves and catheters in reducing the risk of thrombus formation.

Accordingly, one aspect of the present invention provides a compound of formula (I) or a physiologically acceptable derivative thereof for use in medical therapy, particularly for use in the amelioration of a clinical condition in a mammal, including a human, for which a thombin inhibitor is indicated.

In another aspect, the invention provides a method for the treatment and/or prophylaxis of a mammal, including a human, suffering from a condition susceptible to amelioration by a thrombin inhibitor which method comprises administering to the subject an effective amount of a compound of formula (I) or a pharmaceutically acceptable derivative thereof.

In another aspect, the present invention provides the use of a compound of formula (I) or a pharmaceutically acceptable derivative thereof, for the manufacture of a medicament for the treatment and/or prophylaxis of a condition susceptible to amelioration by a thrombin inhibitor.

Preferably, the condition susceptible to amelioration by a thrombin inhibitor is selected from treatment of acute vascular diseases such as acute coronary syndromes (for example primary and secondary prevention of myocardial infarction and unstable angina and treatment of prothrombotic sequalae associated with myocardial infarction or heart failure), thromboembolism, acute vessel closure associated with thrombolytic therapy and percutaneous transluminal coronary angioplasty, transient ischemic attacks, pulmonary embolism, deep vein thrombosis, peripheral arterial occlusion, prevention of vessel luminal narrowing (restenosis), and the prevention of thromboembolic events associated with atrial fibrillation, e.g. stroke.

More preferably, the condition susceptible to amelioration by thrombin inhibitor is selected from acute coronary syndromes (for example primary and secondary prevention of myocardial infarction and unstable angina and treatment of prothrombotic sequalae associated with myocardial infarction or heart failure), pulmonary embolism, deep vein thrombosis and the prevention of thromboembolic events associated with atrial fibrillation, e.g. stroke.

References in this specification to treatment include prophylactic treatment as well as the alleviation of symptoms.

In a further aspect, the present invention provides a compound of formula (I) or a pharmaceutically acceptable derivative thereof for use as a therapeutic agent for use in medicine, particularly human medicine.

While it is possible that, for use in therapy, a compound of the present invention may be administered as the raw chemical, it is preferable to present the active ingredient as a pharmaceutical formulation.

In a further aspect, the invention provides a pharmaceutical composition comprising at least one compound of formula (I) or a pharmaceutically acceptable derivative thereof in association with a pharmaceutically acceptable carrier and/or excipient.

The carrier and/or excipient must be "acceptable" in the sense of being compatible with the other ingredients of the formulation and not deleterious to the recipient thereof.

Accordingly, the present invention further provides a pharmaceutical formulation comprising at least one compound of formula (I) or a pharmaceutically acceptable derivative thereof, thereof in association with a pharmaceutically acceptable carrier and/or excipient. The carrier and/or excipient must be "acceptable" in the sense of being compatible with the other ingredients of the formulation and not deleterious to the recipient thereof.

In another aspect, the invention provides a pharmaceutical composition comprising, as active ingredient, at least one compound of formula (I) or a pharmaceutically acceptable derivative thereof in association with a pharmaceutically acceptable carrier and/or excipient for use in therapy, and in particular in the treatment of human or animal subjects suffering from a condition susceptible to amelioration by a thrombin inhibitor.

There is further provided by the present invention a process of preparing a pharmaceutical composition, which process comprises mixing at least one compound of formula (I) or a pharmaceutically acceptable derivative thereof, together with a pharmaceutically acceptable carrier and/or excipient.

The compounds for use according to the present invention may be formulated for oral, buccal, parenteral, topical, rectal, or transdermal administration or in a form suitable for administration by inhalation or insufflation (either through the mouth or the nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds according to the present invention may be formulated for parenteral administration by injection e.g. by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds according to the present invention may be formulated for topical administration by insufflation and inhalation. Examples of types of preparation for topical administration include sprays and aerosols for use in an inhaler or insufflator, or a formulated powder for use in an inhaler.

Powders for external application may be formed with the aid of any suitable powder base, for example, lactose, talc, or starch. Spray compositions may be formulated as aqueous solutions or suspensions or as aerosols delivered from pressurised packs, such as metered dose inhalers, with the use of a suitable propellant.

The compounds according to the present invention may also be formulated in rectal compositions such as suppositories or retention enemas, e.g. containing conventional suppository bases such as cocoa butter or other glycerides.

In addition to the formulations described previously, the compounds may also be formulated as a depot preparation. Such long acting formulations may be administered by implantation (for example subcutaneously, transcutaneously or intramuscularly) or by intramuscular injection. Thus, for example, the compounds according to the present invention may be formulated with suitable polymeric or hydrophobic materials (for example as an emulsion in an acceptable oil) or ion exchange resins, or as sparingly soluble derivatives, for example, as a sparingly soluble salt.

The compounds of the present invention may be in the form of and/or may be administered as a pharmaceutically acceptable salt. For a review on suitable salts see Berge et al, J. Pharm. Sci., 1977, 66, 1-19.

Typically, a pharmaceutically acceptable salt may be readily prepared by using a desired acid or base as appropriate. The salt may precipitate from solution and be collected by filtration or may be recovered by evaporation of the solvent.

A proposed dose of the compounds according to the present invention for administration to a human (of approximately 70kg body weight) is 0.1mg to 1g, preferably to 1 mg to 500mg of the active ingredient per unit dose, expressed as the weight of free base. The unit dose may be administered, for example, 1 to 4 times per day. The dose will depend on the route of administration. It will be appreciated that it may be necessary to make routine variations to the dosage depending on the age and weight of the patient as well as the severity of the condition to be treated. The precise dose and route of administration will ultimately be at the discretion of the attendant physician or veterinarian.

No toxicological effects are expected when a compound of the present invention is administered in the above-mentioned dosage range.

The compounds of formula (I) may also be used in combination with other therapeutic agents. The invention thus provides, in a further aspect, a combination comprising a compound of formula (I) or a pharmaceutically acceptable derivative thereof together with a further therapeutic agent.

When a compound of formula (I) or a pharmaceutically acceptable derivative thereof is used in combination with a second therapeutic agent active against the same disease state the dose of each compound may differ from that when the compound is used alone. The compounds of the present invention may be used in combination with other antithrombotic drugs such as FXa inhibitors, thromboxane receptor antagonists, prostacyclin mimetics, phosphodiesterase inhibitors, fibrinogen antagonists, thrombolytic drugs such as tissue plaminogen activator and streptokinase, non-steroidal anti-inflammatory drugs such as aspirin, and the like), anti-hypertensive agents (such as angiotensin-converting enzyme inhibitors, angiotensin-II receptor antagonists, ACE / NEP inhibitors, β-blockers, calcium channel blockers, PDE inhibitors, aldosterone blockers), anti-atherosclerotic / dyslipidaemic agents (such as HMG-CoA reductase inhibitors) and anti-arrhythmic agents.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations by any convenient route.

When administration is sequential, either the thrombin inhibitor or the second therapeutic agent may be administered first. When administration is simultaneous, the combination may be administered either in the same or different pharmaceutical composition.

When combined in the same formulation it will be appreciated that the two compounds must be stable and compatible with each other and the other components of the formulation. When formulated separately they may be provided in any convenient formulation, conveniently in such manner as are known for such compounds in the art.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art. It will be appreciated that the amount of a compound of the invention required for use in treatment will vary with the nature of the condition being treated and the age and the condition of the patient and will be ultimately at the discretion of the attendant physician or veterinarian.

The compounds of formula (I) and physiologically acceptable derivatives thereof may be prepared by the processes described hereinafter, said processes constituting a further aspect of the invention. In the following description, the groups are as defined above for compounds of formula (I) unless otherwise stated.

According to a further aspect of the present invention, there is provided a process (A) for preparing a compound of formula (I), which process comprises reacting a compound of formula (II) with a compound of formula (III): 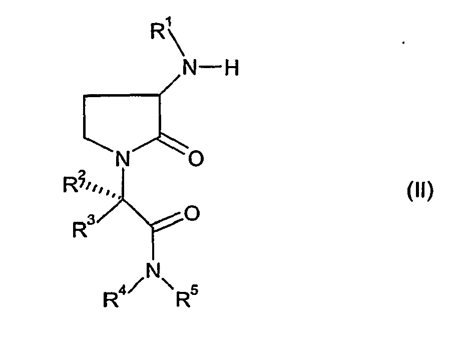 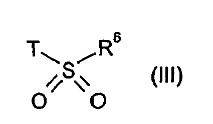 wherein T is a suitable leaving group, such as a halide, preferably chloride. The reaction is conveniently carried out in the presence of a base, e.g. pyridine, and in a suitable solvent, e.g. acetonitrile, suitably at room temperature.

A compound of formula (II) where R¹ is hydrogen may be prepared from a compound of formula (IV) 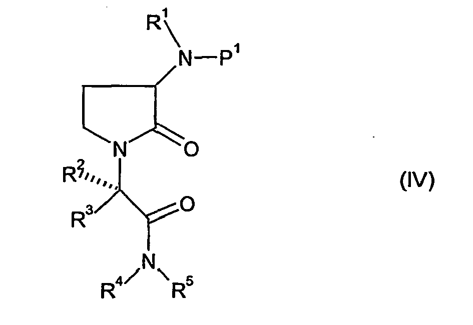 wherein P¹ is a suitable amino protecting group, e.g. Boc (t-butyloxycarbonyl) or Cbz (benzyloxycarbonyl), by removal of the protecting group under standard conditions. For example, when P¹ represents Boc, removal of the protecting group may be effected under acidic conditions, using for example HCI in a solvent such as dioxan. For example, where P¹ represents Cbz, the protecting group may be removed by reaction with hydrogen in the presence of a metal catalyst, e.g. palladium/charcoal at atmospheric pressure. Suitably, the reaction is carried out in an alcoholic solvent, e.g. ethanol, suitably at room temperature.

A compound of formula (IV) may be prepared by reacting a compound of formula (V) with a compound of formula (Vi): 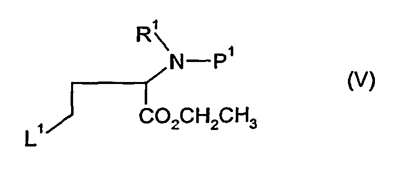 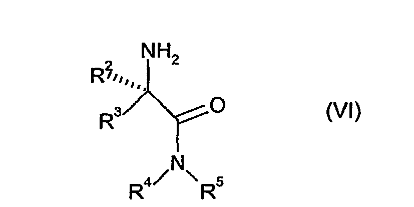 where L¹ is a suitable leaving group e.g. iodide, in the presence of a base e.g. triethylamine and/or 4-(dimethylamino)pyridine, in a suitable solvent e.g. acetonitrile, at elevated temperature, preferably under reflux.

A compound of formula (VI) where R² is CH(CH₃)OCH₃ and R³ is hydrogen, may be prepared from a compound of formula (VII) 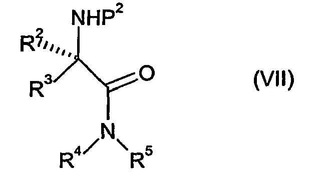 wherein P² is a suitable amino protecting group, e.g. Boc, by removal of the protecting group under standard conditions. For example, when P² represents Boc, removal of the protecting group may be effected under acidic conditions, using for example HCl in a solvent such as dioxan.

A compound of formula (VII) may be prepared from a compound of formula (VIII) 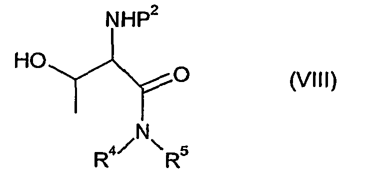 by reaction with trimethyloxonium tetrafluoroborate in the presence of a suitable solvent e.g. dichloromethane, suitably at room temperature.

A compound of formula (VIII) may be prepared from a compound of formula (IX) 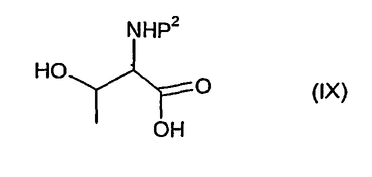 by reaction with TBTU (Benzotriazol-1-yl-N,N,N',N'-tetramethyluronium tetrafluoroborate), N,N-diisopropyl ethylamine and morpholine in the presence of a suitable solvent e.g. dichloromethane or dimethylformamide, suitably at room temperature.

It will be appreciated by persons skilled in the art that compounds of formula (I), may be prepared by interconversion, utilising other compounds of formula (I) which are optionally protected by standard protecting groups, as precursors. For instance, compounds of formula (I) where R² is -CH₂CO₂-benzyl, may be converted into compounds of formula (I) possessing alternative substituents at R², e.g. -CH₂CO₂H, -CH₂CONR⁷R⁸ and -CH₂CON(R⁸)C₀₋₂alkyl(R⁹), by reaction with e.g. HBr in acetic acid, suitably at room temperature, optionally followed by reaction with TBTU, N,N-diisopropyl ethylamine, and a suitable amine, in the presence of a suitable solvent e.g. dichloromethane or dimethylformamide, suitably at room temperature.

According to a process (B), a compound of formula (IV) may also be prepared from a compound of formula (X) 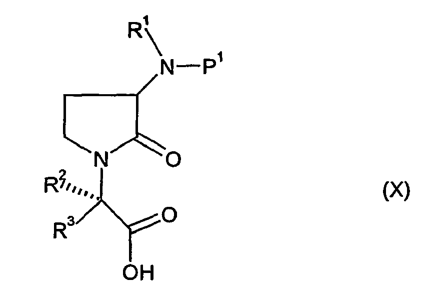 by reaction with TBTU, N,N-diisopropyl ethylamine and morpholine in the presence of a suitable solvent e.g. dichloromethane or dimethylformamide, suitably at room temperature.

A compound of formula (X) may be prepared from a compound of formula (XI) 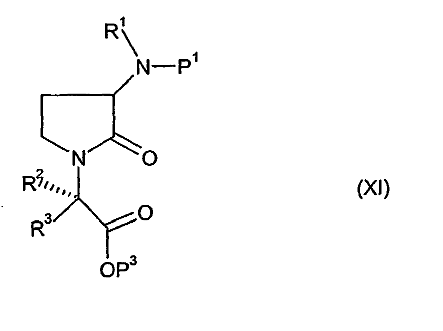 wherein P³ is a suitable carboxyl protecting group, e.g. t-Butyl or benzyl, by removal of the protecting group under standard conditions. For example, when P³ represents t-Butyl, removal of the protecting group may be effected under acidic conditions, using for example trifluoroacetic acid in a solvent such as dichloromethane. For example, when P³ represents benzyl, the protecting group may be removed by reaction with hydrogen in the presence of a metal catalyst, e.g. palladium/charcoal at atmospheric pressure in a suitable solvent, e.g. ethanol, suitably at room temperature.

A compound of formula (XI) may be prepared from a compound of formula (XII) 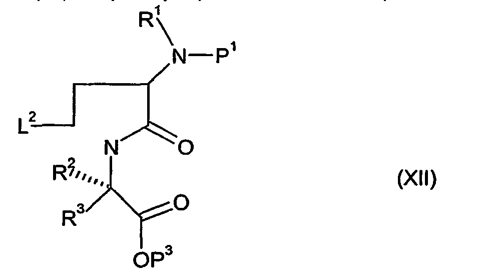 where L² represents a potential leaving group, e.g. SMe, by treatment with methyl iodide, followed by ring closure with Dowex 2 x 8 400 mesh OH⁻ resin in a suitable solvent, e.g. MeCN (acetonitrile). Alternatively, the ring closure may be performed by treatment with potassium carbonate in a suitable solvent, e.g. MeCN.

A compound of formula (XII) may be prepared by reacting a compound of formula (XIII) with a compound of formula (XIV) 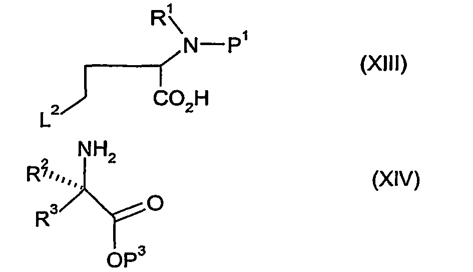 by treatment with a coupling agent, for example 1-[3-(dimethylamino)propyl]-3-ethyl carbodiimide hydrochloride, HOBt (1-hydroxybenzotriazole), a base, e.g. Et₃N (triethylamine), and an organic solvent, e.g. dimethylformamide or dichloromethane, suitably at room temperature.

The various general methods described above may be useful for the introduction of the desired groups at any stage in the stepwise formation of the required compound, and it will be appreciated that these general methods can be combined in different ways in such multi-stage processes. The sequence of the reactions in multi-stage processes should of course be chosen so that the reaction conditions used do not affect groups in the molecule which are desired in the final product. For example, those skilled in the art will appreciate that, with the use of appropriate protecting groups, the coupling to any of groups -R¹, -SO₂R⁶ or -NR⁴R⁵ can be the final step in the preparation of a compound of formula (I). Hence, in another aspect of the invention, the final step in the preparation of a compound of formula (I) may comprise the coupling to group -R¹ by reacting a compound of formula (XV) with a compound of formula (XVI):

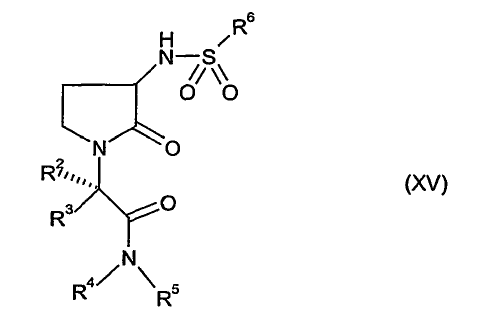

R¹-x (XVI)

Suitably, where X is a leaving group such as a halogen atom, e.g. bromine, the reaction is carried out in the presence of a base, e.g. LiHMDS (lithium hexamethyldisilylamide), potassium carbonate or sodium carbonate. Preferably, the reaction is effected in a suitable organic solvent, e.g. tetrahydrofuran, dimethylformamide, at a temperature from -78°C to +50°C, preferably -78°C to +20°C.

A compound of formula (XV) may be prepared under the conditions described above wherein R¹ is hydrogen.

In a further aspect of the present invention, the final step in the preparation of a compound of formula (I) may comprise the coupling to group -NR⁴R⁵ by reacting a compound of formula (XVII) with a compound of formula (XVIII):

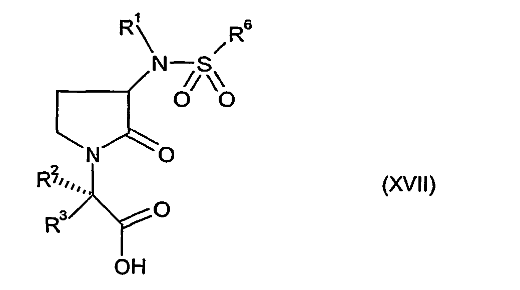

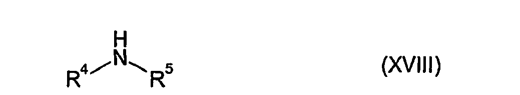

Suitably, the reaction may be carried out in the presence of a coupling agent, for example 1-[3-(dimethylamino)propyl]-3-ethyl carbodiimide hydrochloride, HOBt (1-hydroxybenzotriazole), a base, e.g. Et₃N (triethylamine), and an organic solvent, e.g. dichloromethane, suitably at room temperature.

A compound of formula (XVII) may be prepared by reacting a compound of formula (X) wherein P¹ is hydrogen with a compound of formula (III) under the conditions described above.

According to a process (C), a compound of formula (I) may also be prepared by reacting a compound of formula (XIX) with a compound of formula (XX): 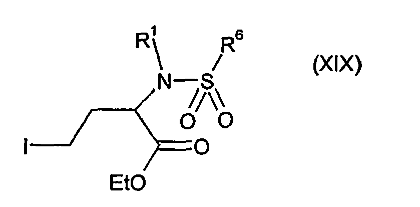 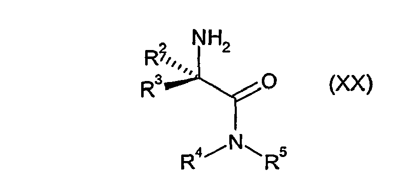 in a suitable solvent e.g. acetonitrile in the presence of a suitable base e.g. N-methyl morpholine suitably at elevated temperature, preferably at reflux, followed by treatment with a strong base e.g. 4-dimethylaminopyridine suitably at elevated temperature, preferably at reflux.

A compound of formula (XX) may be prepared from a compound of formula (VII) under conditions described above.

A compound of formula (I) where R² represents -C₁₋₂alkylR⁹ and R⁹ represents a 5-membered heteroaromatic group containing at least one N atom may be prepared from a compound of formula (I) where R² represents -C₁₋₂alkylCO₂H. Thus, for example, a compound of formula (I) having the structure (XXI) may be prepared from a compound of formula (XXII): 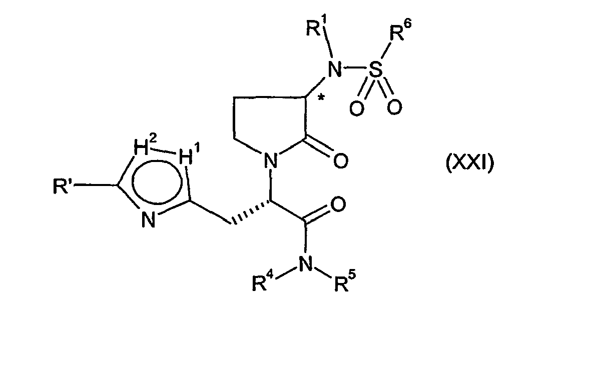 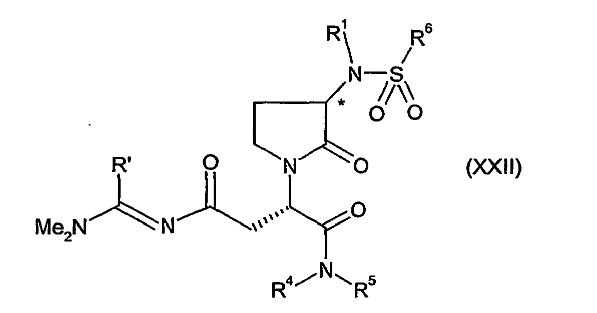 where R' represents hydrogen or methyl, H¹ represents O, N, NH or N-methyl and H² represents N or N-methyl, by reacting with an appropriate bis nucleophile e.g. hydrazine or hydroxylamine in a solvent e.g. glacial acetic acid at elevated temperature, preferably between 70°C and 80°C.

A compound of formula (XXII) may be prepared from a compound of formula (XXIII): 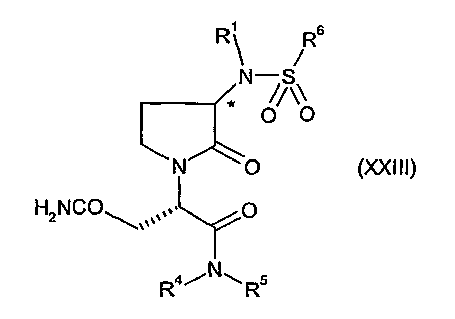 by reaction with dimethylformamide dimethyl acetal in a suitable solvent e.g. dimethylformamide, suitably at elevated temperature, preferably 50-70°C.

A compound of formula (XXIII) may be prepared from a compound of formula (I) where R² represents -C₁₋₂alkylCO₂H by reaction with ammonia in the presence of a suitable solvent e.g. dimethylformamide and a suitable base e.g. N,N-diisopropylethylamine.

Alternatively, a compound of formula (I) where R² represents -C₁₋₂alkylR⁹ and R⁹ represents a 5-membered heteroaromatic group containing at least one N atom may be prepared from a compound of formula (IV) where R² represents -C₁₋₂alkylCO₂H using the methodology described above followed by removal of the protecting group under standard conditions followed by reaction with a compound of formula (III) as described above.

Compounds of formula (III) are either known in the art or may be prepared from a compound of formula (XXIV) 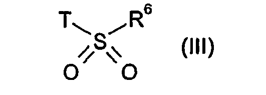 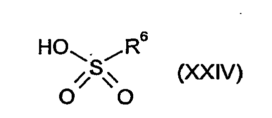 by treatment with a chlorinating agent e.g. POCl₃ preferably at elevated temperature, preferably 100-150°C. Compounds of formula (XXIV) are either known in the art or may be prepared from a compound of formula (XXV)

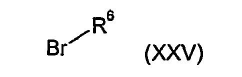

By treating with sodium sulfite in a suitable solvent e.g. water suitably at elevated temperature, preferably at reflux. Compounds of formula (XXV) are either known in the art or may be prepared from compounds of formula (XXVI) 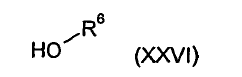 by treatment with suitable bromination agents e.g. triphenylphosphine and carbon tetrabromide in a suitable solvent e.g. tetrahydrofuran or dichloromethane between - 20°C and room temperature, preferably 0°C to room temperature.

Compounds of formula (III) may also be prepared from compounds of formula (XXVI) by treatment with a derivative of thioacetic acid e.g. potassium thioacetate in a suitable solvent e.g. dimethylformamide at elevated temperature, suitably 80-120°C, followed by treatment with a suitable oxidant e.g. chlorine in a suitable solvent mixture e.g. water and chloroform at 0-20°C.

A compound of formula (IV) where R² represents -C₁₋₃alkylCN may be prepared from a compound of formula (IV) where R² represents -C₁₋₃alkylCONR⁷R⁸. Thus, for example, a compound of formula (IV) having the structure (XXVII) may be prepared from a compound of formula (XXVIII): 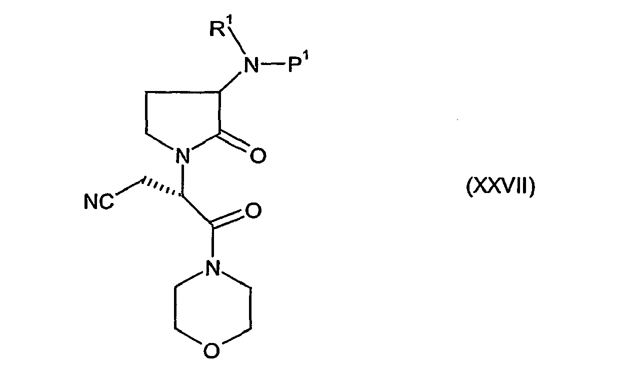 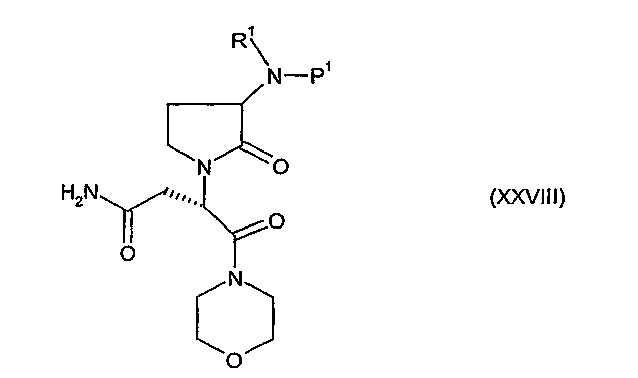 by treatment in a suitable solvent e.g. tetrahydrofuran with a suitable electrophile e.g. trifluoroacetic anhydride and a suitable base e.g. triethylamine at 0-20°C.

A compound of formula (IV), for example as represented by a compound of formula (XXIX) may be prepared, for example from a compound of formula (XXX): 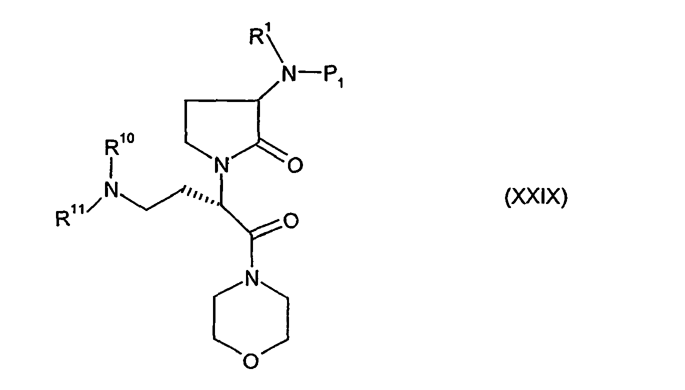 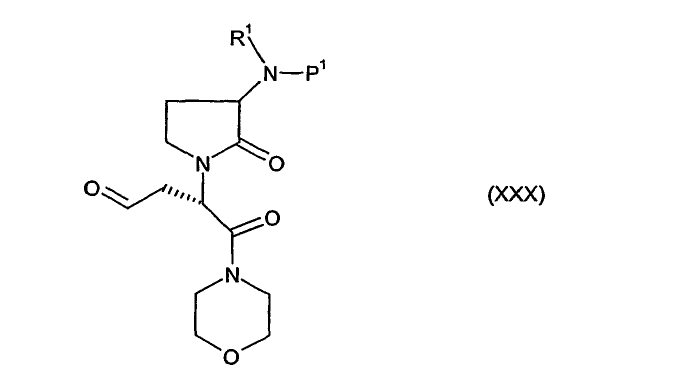 by reaction with a suitable secondary amine NHR¹⁰R¹¹, e.g. dimethylamine, piperidine, or morpholine in the presence of a suitable reducing agent e.g. sodium triacetoxyborohydride in a suitable solvent e.g. dichloromethane, suitably at room temperature.

As an example, a compound of formula (XXX) may be prepared from a compound of formula (XXXI) 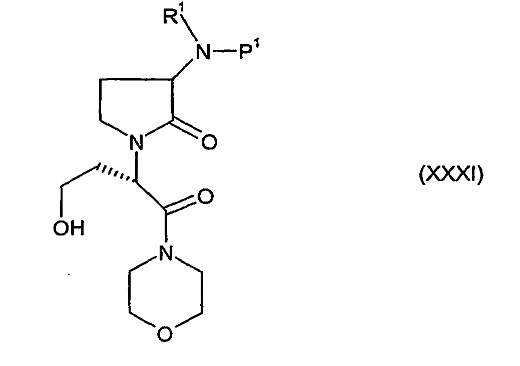 by reaction with a suitable oxidant mixture e.g. tetrapropylammonium perruthenate and N-methylmorpholine oxide in a suitable solvent e.g. dichloromethane at a suitable temperature e.g. 0-20°C.

A compound of formula (XXXI) may be prepared from a compound of formula (XXXII): 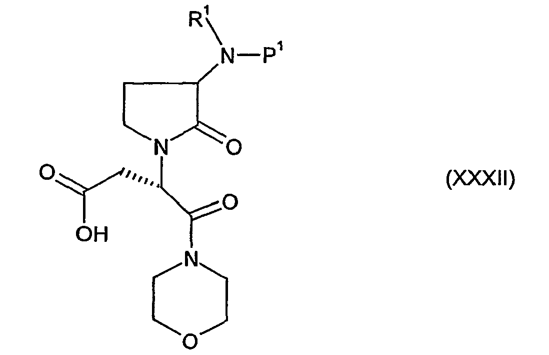 by reaction with a suitable activating agent e.g. isopropyl chloroformate in the presence of a suitable base e.g. N-methyl morpholine in a suitable solvent e.g. tetrahydrofuran at a temperature between -50°C - 0°C, preferably -15°C - 0°C followed by reaction with a suitable reducing agent e.g. sodium borohydride preferably at -15°C - 0°C.

A compound of formula (XXXII) is prepared from a compound of formula (IV), where R² is CH₂CO₂benzyl by removal of the benzyl protecting group by reaction with hydrogen in the presence of a metal catalyst, e.g. palladium/charcoal at atmospheric pressure in a suitable solvent, e.g. acetic acid, suitably at room temperature.

Those skilled in the art will appreciate that in the preparation of the compound of formula (I) or a solvate thereof it may be necessary and/or desirable to protect one or more sensitive groups in the molecule to prevent undesirable side reactions. Suitable protecting groups for use according to the present invention are well known to those skilled in the art and may be used in a conventional manner. See, for example, "Protective groups in organic synthesis" by T.W. Greene and P.G.M. Wuts (John Wiley & sons 1991) or "Protecting Groups" by P.J. Kocienski (Georg Thieme Verlag 1994). Examples of suitable amino protecting groups include acyl type protecting groups (e.g. formyl, tifluoroacetyl, acetyl), aromatic urethane type protecting groups (e.g. benzyloxycarbonyl (Cbz) and substituted Cbz), aliphatic urethane protecting groups (e.g. 9-fluorenylmethoxycarbonyl (Fmoc), t-butyloxycarbonyl (Boc), isopropyloxycarbonyl, cyclohexyloxycarbonyl) and alkyl type protecting groups (e.g. benzyl, trityl, chlorotrityl). Examples of suitable oxygen protecting groups may include for example alky silyl groups, such as trimethylsilyl or tert-butyldimethylsilyl; alkyl ethers such as tetrahydropyranyl or tert-butyl; or esters such as acetate.

Compounds of formulae (III), (V), (IX), (XIII), (XIV), (XVI), (XVIII), (XIX), (XXIV), (XXV), (XXVI) are known compounds and/or can be prepared by processes well known in the art.

Various intermediate compounds used in the above-mentioned process, including but not limited to certain compounds of formulae (II), (III), (IV), (VI), (VII), (VIII), (X), (XI), (XII), (XV), (XVII), (XX), (XXI), (XXII), (XXIII), (XXIV), (XXV), (XXVII), (XXVIII), (XXIX), (XXX), (XXXI), (XXXII) formulae are novel and accordingly constitute a further aspect of the present invention.

The present invention will now be further illustrated by the accompanying examples.

### Examples

### Abbreviations

- 9-BBN: 9-Borabicyclo[3.3.1]nonane
- BAST: Bis (2-methoxyethyl) sulfur trifluoride
- DCM: Dichloromethane
- DIPEA: N,N-Di-isopropylethylamine
- DMAP: 4-(Dimethylamino)pyridine
- DMF: Dimethylformamide
- HOBT: 1-Hydroxybenzotriazole
- MCPBA: m-Chloroperbenzoic acid
- SPE: Solid phase extraction column
- TBAF: Tetrabutylammonium fluoride
- TBTU: Benzotriazol-1-yl-N,N,N',N'-tetramethyluronium tetrafluoroborate
- THF: Tetrahydrofuran
- TFA: Trifluoroacetic acid
- TPAP: Tetrapropylammonium perruthenate

### General purification and analytical methods

### LC/MS Method

Analytical HPLC was conducted on a Supelcosil LCABZ+PLUS column (3µm, 3.3cm x 4.6mm ID) eluting with 0.1% HCO₂H and 0.01 M ammonium acetate in water (solvent A), and 95% MeCN and 0.05% HCO₂H in water (solvent B), using the following elution gradient 0-0.7 minutes 0%B, 0.7-4.2 minutes 0→100%B, 4.2-5.3 minutes 100%B, 5.3-5.5 minutes 100→0%B at a flow rate of 3 ml/minute (System 1). The mass spectra (MS) were recorded on a Fisons VG Platform mass spectrometer using electrospray positive ionisation [(ES+ve to give MH⁺ and M(NH₄)⁺ molecular ions] or electrospray negative ionisation [(ES-ve to give (M-H)⁻ molecular ion] modes.

¹H nmr spectra were recorded using a Bruker DPX 400MHz spectrometer using tetramethylsilane as the internal standard. The following abbreviations are used.
- t: triplet
- m: multiplet
- d: doublet
- s: singlet
- br: broad

Biotage™ chromatography refers to purification carried out using equipment sold by Dyax Corporation (either the Flash 40i or Flash 150i) and cartridges pre-packed with KPSil.

Mass directed directed high performance liquid chromatography was conducted on a HPLCABZ+ 5µm column (5cm x 10mm i.d.) with 0.1% HCO₂H in water and 95% MeCN, 5% water (0.5% HCO₂H) utilising the following gradient elution conditions: 0-1.0 minutes 5%B, 1.0-8.0 minutes 5→30%B, 8.0-8.9 minutes 30%B, 8.9-9.0 minutes 30→95%B, 9.0-9.9 minutes 95%B, 9.9-10 minutes 95→0%B at a flow rate of 8ml minutes⁻¹ (System 2). The Gilson 202-fraction collector was triggered by a VG Platform Mass Spectrometer on detecting the mass of interest.

Automated preparative HPLC employed the following conditions.
• Column: 10cm x 21.2mm ID, 5um ABZ+PLUS
• Flow Rate: 4mUmin
• Temp: RT
Solvents:
A: HPLC water + 0.1 % Formic Acid
B: Acetonitrile + 0.05% Formic Acid

| Gradient: | Time | A% | B% |
|---|---|---|---|
| | 0.00 | 95 | 5 |
| | 1.45 | 95 | 5 |
| | 20 | 10 | 90 |
| | 30 | 10 | 90 |
| | 32 | 95 | 5 |

SPE (solid phase extraction) refers to the use of cartridges sold by International Sorbent Technology Ltd.

SCX SPE (solid phase extraction) refers to the use of acidic ion exchange cartridges sold by International Sorbent Technology Ltd.

### Intermediates

### Route 1

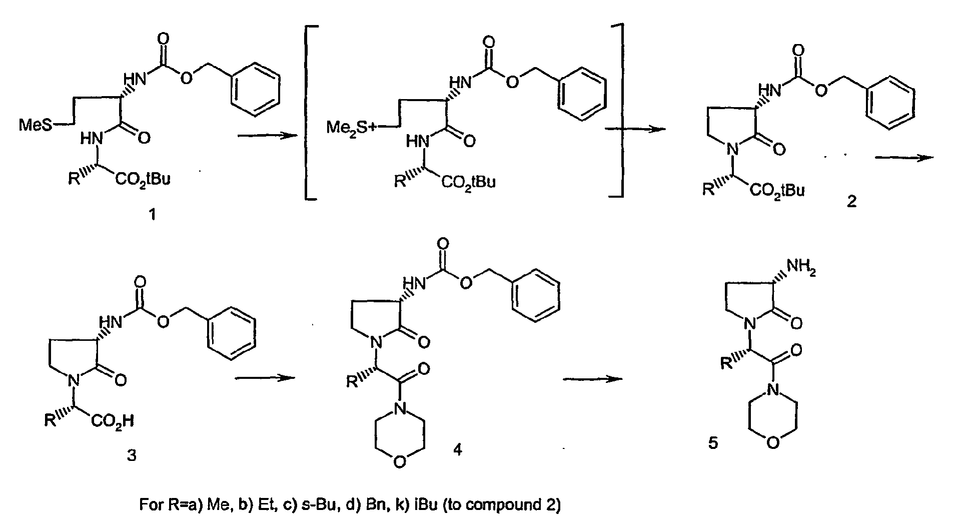

### Intermediate 1a) tert-Butyl N-[(benzyloxy)carbonyl]-L-methionyl-L-alaninate

N-Benzyloxycarbonyl methionine (10g) was dissolved in DMF (200ml) and 1-[3-(dimethylamino)propyl]-3-ethylcarbodiimide hydrochloride (8.13g) was added followed by HOBT (5.72g) and triethylamine (19.7ml). The mixture was stirred for 1 h then L-alanine *tert*-butyl ester (7.7g) was added and stirring continued for 18h. The mixture was concentrated under reduced pressure and partitioned between diethyl ether and water. The separated organic phase was washed with hydrochloric acid (1N), saturated sodium bicarbonate solution and brine, dried (over magnesium sulphate) and concentrated under reduced pressure to give the title compound as an orange oil which crystallised on standing. RT 3.11min, MH⁺ 410

### Intermediate 1b) tert-Butyl (2S)-2({N-[(benzyloxy)carbonyl]-L-methionyl}amino) butanoate

A mixture of (S)-2-aminobutyric acid *tert*-butyl ester (1.96g) in DCM (50ml) was treated with N-benzyloxycarbonyl methionine (2.98g) and cooled to 10°C. N,N-Diisopropylethylamine (3.85ml) was added dropwise giving a clear solution. TBTU (3.37g) was added in portions over 2 minutes. The reaction mixture was stirred in the ice bath for 5 minutes and then allowed to warm to room temperature and stirred for 1.5 hours. The reaction mixture was stirred vigorously with saturated aqueous sodium hydrogen carbonate (70ml) for 5 minutes. The layers were separated and the organic layer was dried and evaporated to dryness. The residue was purified by Biotage™ chromatography eluted with ethyl acetate:cyclohexane to give the title compound.
RT 3.53min, MH⁺ 425

### Intermediate 1c) tert-Butyl N-[(benzyloxy)carbonyl]-L-methionyl-L-isoleucinate

N-Benzyloxycarbonyl methionine (2.83g), isoleucine *tert*-butyl ester hydrochloride (2.24g), HOBT (1.35g) and 1-(3-dimethylaminopropyl)-3-ethylcarbodiimide hydrochloride (1.92g) were stirred together in DMF (50ml) at 0°C. To the mixture was added triethylamine (2.78ml) and stirring was continued for 3.5 hours, during which the mixture was allowed to warm to room temperature. The mixture was partitioned between water (100ml) and ethyl acetate (100ml). The organic phase was diluted with a further 50ml of ethyl acetate and washed with 5% aqueous sodium hydrogen carbonate, 10% aqueous citric acid and brine (50ml each). Drying (sodium sulphate) and evaporation gave a colourless gum which was used without further purification. RT 3.72min MH⁺ 453

### Intermediate 1d) tert-Butyl N-[(benzyloxy)carbonyl]-L-methionyl-L-phenylalaninate

N-Benzyloxycarbonyl methionine (14.16g), L-phenylalanine *tert*-butyl ester hydrochloride (12.88g), HOBT (6.75g), 1-(3-dimethylaminopropyl)-3-ethylcarbodiimide hydrochloride (9.6g) and triethylamine (13.9ml,) were mixed in DMF (600ml) at 0°C. The mixture was stirred for 3.5 hours, during which the mixture was allowed to warm to room temperature. The mixture was partitioned between water (500ml) and ethyl acetate (500ml). The organic phase was diluted with a further 50ml of ethyl acetate and washed with 5% aqueous sodium hydrogen carbonate, 10% aqueous citric acid and brine (300ml each) to give the title compound as a white solid .
RT 3.71 min, MH⁺ 487

### Intermediate 1k) tert-Butyl N-[(benzyloxy)carbonyl]-L-methionyl-L-leucinate

Prepared in the manner of intermediate 1d) from N-Benzyloxycarbonyl methionine and L-leucine *tert*-butyl ester.
RT 3.55min, MH⁺ 453

### Intermediate 2a) tert-Butyl (2S)-2-((3S)-3-{[(benzyloxy)carbonyl]amino}-2-oxopyrrolidin-1-yl)propanoate

A solution of intermediate 1a) (11.9g) in acetone (75ml) was treated with methyl iodide (18ml) and stirred at room temperature for 72 hours. The reaction mixture was then concentrated under reduced pressure to give an orange solid that was dissolved in acetonitrile (200ml). Dowex (OH⁻ form) resin (19.42g) was added and the mixture stirred for 18 hours at room temperature. The mixture was filtered and the resin washed with ethyl acetate. The filtrate was concentrated under reduced pressure to afford a yellow oil which was purified by Biotage™ chromatography (silica, eluting with cyclohexane:ethyl acetate 3:2) to give the title compound as a colourless oil.
Mass spectrum: Found: MH⁺ 363

### Intermediate 2b) tert-Butyl (2S)-2-((3S)-3-{[(benzyloxy)carbony]amino}-2-oxopyrrolidin-1-yl)butanoate

A solution of intermediate 1b) (4.5g) in acetone (30ml) was treated with iodomethane (6.6ml) dropwise over 5 minutes. There was no temperature rise. The reaction was stirred at room temperature, under nitrogen for 19 hours and then evaporated to give the sulfonium iodide as a sticky yellow foam (5.39g, RT 2.48min M⁺ 439). A solution of the sulfonium iodide (5.35g) in dry acetonitrile (80ml) was then treated with Dowex (OH form) resin (7.2g) and stirred at room temperature for 19 hours. The reaction mixture was filtered through celite and resin was washed with acetonitrile (50ml) and ethyl acetate (50ml). The filtrate was evaporated to dryness and purified on 2x50g SPE eluted with [3:2] to [2:1] cyclohexane:ethyl acetate to give the title compound as a pale yellow oil which solidified on standing.
RT 3.34min M⁺ 377.

### Intermediate 2c) tert-Butyl (2S,3S)-2-((3S)-3-{[(benzyloxy)carbonyl]amino}-2-oxopyrrolidin-1-yl)-3-methylpentanoate

Intermediate 1c) (4.2g) in acetone (25ml) was stirred with iodomethane (5.8ml) at room temperature for 18 hours. Evaporation of the solvent gave the sulfonium salt as a foam which was used without further purification.This was stirred in acetonitrile (80ml) for 18hours with Dowex (OH form) resin (7.5g). The suspension was filtered, the resin washed with acetonitrile and the combined filtrates evaporated. The crude product was purified by silica gel chromatography (Biotage™, ethyl acetate:cyclohexane, 1:3) to afford the title compound as an oil which became a white solid on standing. RT 3.60min MH⁺ 405, M+NH₄⁺ 422,

### Intermediate 2d) tert-Butyl (2S)-2-((3S)-3-{[(benzyloxy)carbonyl]amino}-2-oxopyrroridin-1-yl)-3-phenyl-propanoate

To a solution of intermediate 1d) (12.15g) in acetone (70ml) was added iodomethane (15.6ml). After overnight stirring the solvents were evaporated giving the crude sulfonium salt (15.7g) as a foam which was used directly. This was stirred in acetonitrile (200ml) with Dowex (OH form) resin (19.4g) for 18hours. The resin was filtered off and washed with 3 portions of acetonitrile. Evaporation followed by silica gel chromatography (Biotage™, ethyl acetate:cyclohexane, 1:8 to 1:4) provided the title compound as a colourless gum.
RT 3.61 min MH⁺ 439

### Intermediate 2k) tert-Butyl (2S)-2-((3S)-3-{[(benzyloxy)carbonyl]amino}-2 oxopyrrolidin-1-yl)-4-methylpentanoate

Prepared in the manner as for intermediate 2d) from intermediate 1k). RT 3.42min

### Intermediate 3a) (2S)-2-((3S)-3-{[(Benzyloxy)carbonyl]amino}-2-oxopyrrolidin-1-yl) propanoic acid

Intermediate 2a) (0.5g) was dissolved in DCM (7ml), and TFA (4.7ml) was added. The mixture was stirred at room temperature for 4 hours and then concentrated under reduced pressure to give the title compound as a colourless oil, which crystallised after azeotroping with toluene.
Mass spectrum: Found: MH⁺ 307

### Intermediate 3b) (2S)-2-((3S)-3-{[(Benzyloxy)carbonyl]amino}-2-oxopyrrolidin-1-yl) butanoic acid

A solution of intermediate 2b) (1.56g) in dry DCM (5ml) was cooled in an ice bath and treated with TFA (2.5ml) and the reaction mixture was stirred at room temperature, under nitrogen, for 2 hours. A further 2.5ml TFA was added and the reaction mixture was stirred for 1 hour. The reaction mixture was evaporated to dryness and partitioned between ethyl acetate (25ml) and brine (20ml). The layers were separated and the organic layer was washed with brine (10ml). A precipitate formed which was dissolved by adding DCM (25ml). The organic layer was separated and dried (sodium sulphate) and evaporated to dryness. The oil was mixed with diethyl ether (30ml) and water (50ml) and a thick precipitate formed which was collected by filtration and dried to give the title compound as colourless solid. RT 2.73 min, MH⁺ 321.

### Intermediate 3c) (2S,3S)-2-((3S)-3-{[(Benzyloxy)carbonyl]amino}-2-oxopyrrolidin-1-yl)-3-methylpentanoic acid

Intermediate 2c) (2.05g) was stirred in dry DCM (5ml) at room temperature as TFA (5ml) was added. The mixture was stirred for 2hours and the solvents evaporated. Further DCM was added and evaporated. In order to remove residual TFA, the gum was dissolved in ethyl acetate (30ml) and was washed twice with water and with brine. Drying over sodium sulphate and evaporation gave the acid as a white solid.
RT 3.02min, MH⁺ 349

### Intermediate 3d) (2S)-2-((3S)-3-{[(Benzyloxy)carbonyl]amino}-2-oxopyrrolidin-1-yl)-3-phenyl propanoic acid

Intermediate 2d) (8.84g) in DCM (30ml) was stirred with TFA (20ml) for 3.5 hours. The solvent was evaporated and the residue taken up in ethyl acetate and washed several times with water. Drying and evaporation gave the title compound as a white foam.
RT 3.07min MH⁺ 383

### Intermediate 4a) Benzyl (3S)-1-[(1S)-1-methyl-2-morpholin-4-yl-2-oxoethyl]-2-oxopyrrolidin-3-ylcarbamate

Intermediate 3a) (84.5g) was dissolved in DMF (21) and TBTU (161g) was added, followed by N,N-diisopropylethylamine (92ml) and morpholine (46ml). The mixture was stirred under nitrogen for 2.5h, and saturated aqueous ammonium chloride was added. The mixture was stirred for 15min then partitioned between water and ethyl acetate. The separated organic phase was washed with lithium chloride (10% by weight), followed by saturated sodium bicarbonate and brine. The organic layer was dried (over sodium sulphate) and concentrated under reduced pressure to give the title compound as a yellow solid.
Mass spectrum: Found: MH⁺ 376

### Intermediate 4b) Benzyl (3S)-1-[(1S)-1-morpholin-4-ylcarbonylpropyl]-2-oxopyrrolidin-3-ylcarbamate

A solution of intermediate 3b) (1.14g) in dry DMF (25ml) was cooled in an ice bath, under nitrogen. Morpholine (0.62ml) was added followed by N,N-diisopropylethylamine (1.24ml). TBTU (2.28g) was added in portions over 5 minutes. The pale yellow solution was stirred in the ice bath for 30 minutes and then at room temperature 16 hours. Sat. ammonium chloride (30ml) was added and then the mixture was partitioned between water (50ml) and ethyl acetate (100ml). The layers were separated and the aqueous layer was washed with ethyl acetate (50ml). The organic extracts were combined and washed with 0.5N sodium carbonate solution, 10% aq. Lithium chloride solution (2x50ml) and brine, dried (sodium sulphate) and evaporated to a gum which was purified on a 10g SPE column eluted with ethyl acetate to give the title compound as a colourless foam.
RT 2.67min M⁺ 390.

### Intermediate 4c) Benzyl (3S)-1-[(1S,2S)-2-methyl-1-(morpholin-4-ylcarbonyl)butyl]-2 oxopyrrolidin-3-ylcarbamate

To intermediate 3c) (1.81g) stirred in DMF (40ml) at room temperature was added TBTU (3.34g), N,N-diisopropylethylamine (1.81ml) and morpholine (0.95ml). After 5 hours the reaction was quenched with saturated aqueous ammonium chloride (50ml) then partitioned between ethyl acetate (150ml) and water (50ml). The aqueous phase was extracted with more EtOAc (50ml) and the combined organics washed with 2N sodium carbonate (50ml), lithium chloride (10% aq., 2 x 50ml), brine (50ml) and dried over sodium sulphate. Removal of solvent gave a gum which crystallised upon trituration with diethyl ether. The solid was filtered, washed with ether and dried.
RT 2.95min MH⁺ 418

### Intermediate 4d) Benzyl (3S)-1-[(1S)-1-benzyl-2-morpholin-4-yl-2-oxoethyl]-2-oxopyrrolidin-3-ylcarbamate

Following a similar protocol to that used for intermediate 4c), intermediate 3d) (7g), TBTU (11.75g), N,N-diisopropylethylamine (6.37ml) and morpholine (3.34ml) were stirred together in DMF (140ml) for 18 hours. Workup as for intermediate 4c) gave a gum which was purified by silica gel chromatography (Biotage™, DCM then DCM:MeOH, 20:1) to afford the title compound.
RT 2.96min MH⁺ 452,

### Intermediate 5a) (3S)-3-Amino-1-[(1S)-1-methyl-2-moroholin-4-yl-2-oxoethyl]pyrrolidin-2-one

A mixture of intermediate 4a) (20g), 10 % palladium on carbon (2g) and ethanol (1.3l) was stirred under an atmosphere of hydrogen for 16 hours. The reaction mixture was filtered through celite and the filtrate was concentrated under reduced pressure to give the title compound as a pale white oil.
¹H NMR (D₄ MeOH): δ5.05 (1H, dd), 3.59 (9H, m), 3.37 (2H, m), 2.42 (1H, m), 1.75 (1 H, m), 1.30 (3H, d) ppm.

### Intermediate 5b) (3S)-3-Amino-1-[(S)-1-(morpholin-4-ylcarbonyl)propyl]pyrrolidin-2-one

A solution of intermediate 4b) (1.14g) in ethanol (25ml) was hydrogenated at room temperature and atmospheric pressure in the presence of 10% palladium on carbon (200mg of 50% wet catalyst) for 24 hours. The mixture was filtered through celite, washed through with ethanol (40ml) and the filtrate was evaporated to dryness. The residue was azeotroped with toluene and evaporated to a colourless oil.
RT 1.14min, M⁺ 256.

### Intermediate 5c) (3S)-3-amino-1-[(1S,2S)-2-methyl-1-(morpholin-4-ylcarbonyl)butyl]pyrrolidin-2-one

Intermediate 4c) (1.3g) in ethanol (25ml) was hydrogenated over 10% palladium on carbon (200mg of 50% wet catalyst) at atmospheric pressure for 18 hours. The catalyst was filtered using celite and washed with ethanol. The filtrates were evaporated giving the title compound.
RT 1.88min, MH⁺ 284

### Intermediate 5d) (3S)-3-amino-1-[(1S)-1-benzyl-2-morpholin-4-yl-2-oxoethyl]pyrrolidin-2-one

Intermediate 4d) (1.35g) in ethanol (35ml) was hydrogenated over 10% palladium on carbon (200mg of 50% wet catalyst) for 18 hours. After 18 hours the mixture was treated with fresh catalyst (200mg of 50% wet catalyst) and hydrogenated for a further 18 hours. The catalyst was filtered off, the filtrate evaporated, redissolved in toluene and evaporated (x2) to give the title compound.
RT 1.89min, MH⁺ 318

### Route 2

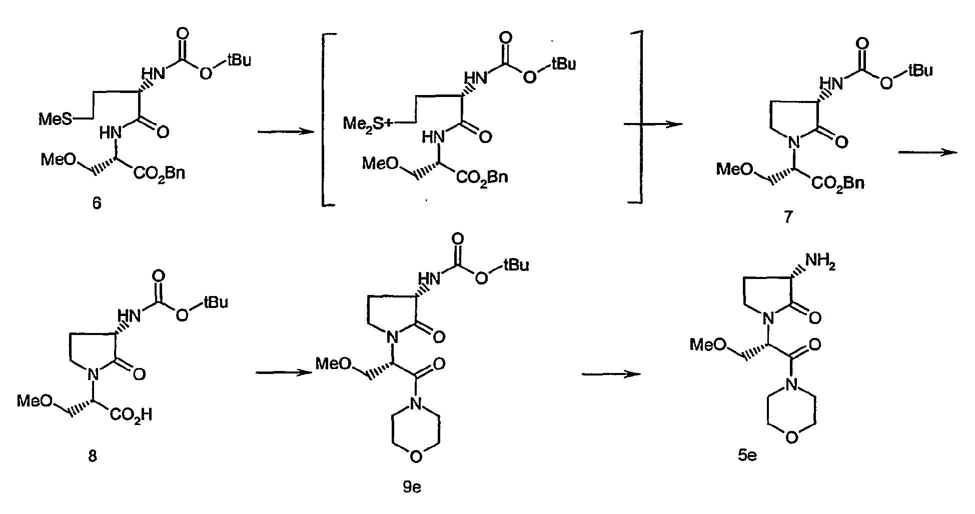

### Intermediate 6 Benzyl N-(tert-butoxycarbonyl)-L-methionyl-O-methyl-L-serinate

A solution of (S)-2-amino-3-methoxypropionic acid benzyl ester hydrochloride (1.40g) in dry DCM (20ml) was cooled in an ice bath under nitrogen. Boc-L-methionine (1.57g) was added followed by N,N-diisopropylethylamine (2.19ml). After stirring for 5 minutes TBTU (2.02g) was added in portions over 5 minutes. The reaction mixture was stirred for 15 minutes in the cooling bath and then stirred at room temperature for 3 hours. The reaction mixture was partitioned between DCM (25ml) and saturated sodium bicarbonate solution (40ml). The layers were separated and the aqueous layer was washed with DCM (25ml). The organic extracts were combined, washed with brine, dried (sodium sulphate) and evaporated to dryness. The oil was purified via silica gel chromatography eluted with cyclohexane:ethyl acetate (1:1) and trituration with diethyl ether/cyclohexane to give the title compound as a colourless solid.
RT 3.21 min M⁺ 441.

### Intermediate 7 Benzyl (2S)-2-{(3S)-3-[(tert-butoxycarbonyl)amino]-2-oxopyrrolidin-1-yl}-3-methoxypropanoate

A suspension of intermediate 6 (1.60g) in acetone (17.5ml) was treated with iodomethane (2.3ml) dropwise over 5 minutes. The yellow solution was stirred at room temperature for 18 hours and then further iodomethane (1.5ml) was added and the reaction was stirred for 3 hours and evaporated to a yellow foam (2.22g, RT 2.42min M⁺ 455). A solution of the resulting sulfonium iodide (1.10g, 1.89 mmoles) in dry acetonitrile (15ml) was treated with Dowex (OH form) resin (2.6g) and stirred at room temperature for 18 hours. 1.5g more resin was added and reaction mixture was stirred for 2 hours. The reaction mixture was filtered through celite and resin was washed with acetonitrile (50ml) and ethyl acetate (50ml). The filtrate was evaporated to dryness and purified via silica gel chromatography eluted with hexane:ethyl acetate (2:1) to give the title compound as a pale yellow oil as an approximately [2:1] mixture of isomers.
RT 3.01 min M⁺ 393.

### Intermediate 8 (2S)-2-{(3S)-3-[(tert-Butoxycarbonyl)amino]-2-oxopyrrolidin-1-yl}-3-methoxypropanoic acid

A solution of intermediate 7 (0.449g) in ethanol (10ml) was hydrogenated at room temperature and atmospheric pressure in the presence of 10% palladium on carbon (300mg of 50% wet catalyst) for 18 hours. The mixture was filtered through celite, washed through with ethanol (40ml) and the filtrate was evaporated to dryness. The residue was azeotroped with toluene and DCM and evaporated to give the title compound as a colourless oil as an approximately [2:1] mixture of isomers.
RT 2.21 min, M⁺ 303.

### Intermediate 9e) tert-Butyl (3S)-1-[(1S)-1-(methoxymethyl)-2-morpholin-4-yl-2-oxoethyl]-2-oxopyrrolidin-3-ylcarbamate

A solution of intermediate 8 (0.377g) in dry DMF (10ml) was cooled in an ice bath under nitrogen. Morpholine (217ul) was added followed by DIPEA (0.435ml). TBTU (0.80g) was added in portions over 5 minutes. The pale yellow solution was stirred in the ice bath for 30 minutes and then at room temperature for 18 hours. Sat. ammonium chloride (10ml) was added and then the mixture was partitioned between water (10ml) and ethyl acetate (40ml). The layers were separated and the aqueous layer was washed with ethyl acetate (2x20ml). The organic extracts were combined and washed with saturated sodium bicarbonate solution, water (10ml) and brine (10ml), dried (sodium sulphate) and evaporated to a gum which was mixed with diethyl ether and the title compound filtered off as a colourless solid.
RT 2.20min M⁺ 372.

### Intermediate 5e) (3S)-3-amino-1-[(1S)-1-(methoxymethyl)-2-morpholin-4-yl-2-oxoethyl]pyrrolidin-2-one hydrochloride.

A solution of intermediate 9e) (0.215g) in dry DCM (3.5ml) was treated with 4M HCl in dioxan (0.88ml) and the solution was stirred at room temperature for 4 hours and evaporated to dryness. The residue was mixed with dichloromethane and evaporated to a colourless foam.
RT 0.38min M⁺ 272.

### Route 3

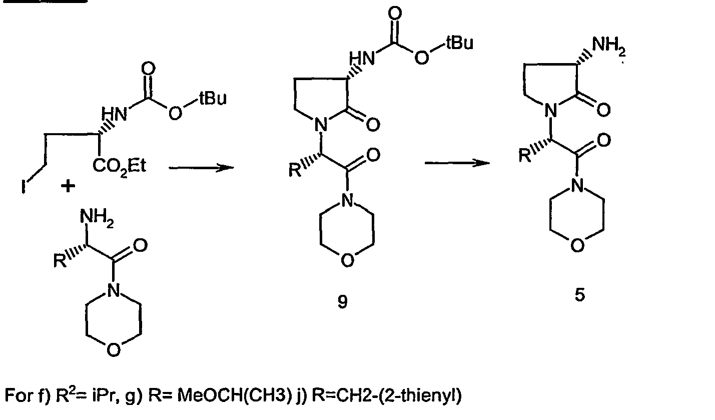

### Intermediate 9f) tert-Butyl (3S)-1-[(1S)-2-methyl-1-(morpholin-4-ylcarbonyl)propyl]-2-oxopyrrolidin-3-ylcarbamate

A solution of 2(S) ethyl 2-(tert butyloxycarbonylamino)-4-iodo butanoate* (392mg), 2-(S)-2-amino-3-methyl morpholinylbutanamide** (225mg) and triethylamine (1.1eq) in acetonitrile (5.5mL) was stirred at 80°C for 4 hours and DMAP (1.0eq) added. The mixture was then stirred at 80°C for 24 hours, solvent removed *in vacuo* and the title compound isolated via silica gel chromatography and amino-propyl SPE.
RT 2.44min, MH⁺ 370
* J.Med.Chem. 1994, 2950-2957
** J.Chem.Soc.Perkin Trans.1, 1975; 830-841

### Intermediate 5f) (3S)-3-Amino-1-[(1S)-2-methyl-1-(morpholin-4-ylcarbonyl)propyl]pyrrolidin-2-one hydrochloride

To a solution of intermediate 9f) (0.23g) in DCM (3mL) was added 4M HCl/dioxan solution (6.0eq) and the mixture stirred for 4 hours. The solution was reduced in vacuo to give the title compound. The amine was liberated from the HCI salt immediately prior to the next step via retention on SCX SPE column (2x10g), washing with methanol and recovery of the free amine via elution with methanolic ammonia (2M) and solvent removal under reduced pressure.
RT 1.53min, MH⁺ 270.

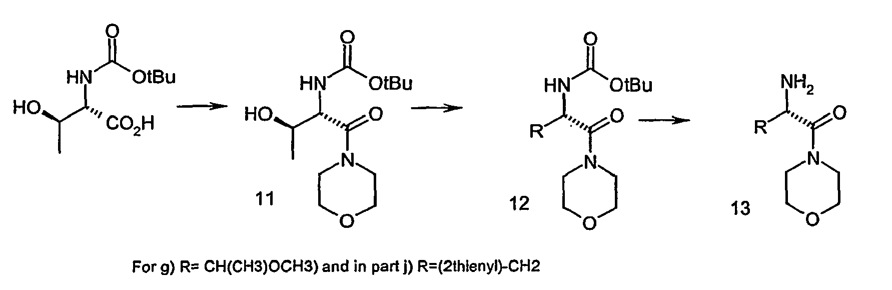

### Intermediate 11 tert-Butyl [(1S,2R)-2-hydroxy-1-(1-morpholin-4-yl-methanoyl)-propyl]carbamate

To a mixture of N-*tert*-butoxycarbonyl threonine (2.4g, 11.2mmol), DIPEA (1.2eq) and morpholine (1.0eq) in DCM (40mL) was added TBTU (1.1eq) and the mixture stirred at room temperature for 2 hours. Saturated sodium bicarbonate solution and 40mL DCM was added, the mixture stirred vigorously for 10 minutes and the layers separated. Reduction of the organic phase *in vacuo* gave the crude product which was then purified by silica gel chromatography (ethyl acetate:cyclohexane 3:1) to give the title compound.
RT 2.04min MH⁺ 289.

### Intermediate 12g) tert-Butyl [(1S,2R)-2-methoxyoxy-1-(1-morpholin-40-yl-methanoyl)-propyl]carbamate

A solution of intermediate 11 (1.15g, 4.0mmol) in DCM (20mL) was stirred vigorously at 0°C in the dark. To this was added Proton Sponge (1.3eq), trimethyloxonium tetrafluoroborate (1.3eq) and the mixture allowed to warm to room temperature upon which it was stirred for 8 hours. The solution was filtered, washed with 2N HCl (20mL) and reduced *in vacuo*. The resulting residue was purified via silica gel chromatography to give the title compound.
RT 2.31min MH⁺ 303.

### Intermediate 13g) (3R)-3-methoxy-1-morpholin-4-yl-1-oxobutan-2-amine HCl salt

Prepared in a similar manner to intermediate 5f) from intermediate 12h).
RT 0.46min, MH⁺ 203

### Intermediate 9g) tert-butyl (3S)-1-[(1S,2R)-2-methoxy-1-(morpholin-4-ylcarbonyl)propyl]-2-oxopyrrolidin-3-ylcarbamate

Prepared in a similar fashion to intermediate 9f) from intermediate 13g) + 2(S) ethyl 2-(tert butyloxycarbonylamino)-4-iodo butanoate*.
RT 2.31 min, MH⁺ 386
* J.Med.Chem. 1994, 2950-2957

### Intermediate 5g) (3S)-3-amino-1-[(1S,2R)-2-methoxy-1-(morpholin-4-ylcarbonyl)propyl]pyrrolidin-2-one

Prepared in a similar fashion to Intermediate 5f), from Intermediate 9g).
RT 1.3min MH⁺ 286

### Intermediate 12j) tert Butyl ((S)-2-morpholin-4-yl-2-oxo-1-thien-2-ylmethyl-ethyl)carbamate

To Boc-L-2-thienylalanine (360mg) in DCM (10ml) was DIPEA (0.28ml), TBTU (0.47g) and morpholine (0.13ml). The mixture was stirred for two hours, then sat. sodium bicarbonate solution (10ml) was added and the mixture stirred for 10 minutes. The organic layer was separated and evaporated in vacuo to give the title compound.
RT 2.76min, MH⁺ 341

### Intermediate 13j) (2S)-1-morpholin-4-yl-1-oxo-3-thien-2-ylpropan-2-amine

Intermediate 12j) (470mg) was taken up in DCM (7ml). 4M HCl in dioxan (2ml) was added and the mixture was stirred for 2 hours. Solvent was then evaporated in vacuo, and the residue purified on an NH2-ion exchange column (eluting with methanol followed by methanol/ammonia) to give the title compound as the free base
RT 1.47min, MH⁺ 241

### Intermediate 9j) tert-butyl (3S)-1-[(1S)-2-morpholin-4-yl-2-oxo-1-(thien-2-ylmethyl)ethyl]-2-oxopyrrolidin-3-ylcarbamate

Intermediate 13j) (316mg) and 2(S) ethyl 2-(*tert*-butyloxycarbonylamino)-4-iodo butanoate* (420mg) were stirred in acetonitrile (5ml) with triethylamine (0.18ml) and heated to 85°C. After 2 hours, DMAP (160mg) was added, and the mixture was stirred overnight at 90°C. Solvent was then evaporated in vacuo, and purified by silica gel chromatography (ethyl acetate) to give the title compound. RT 2.79min, MH⁺ 424
* J.Med.Chem. 1994, 2950-2957

### Intermediate 5j) (3S)-3-amino-1-[(1S)-2-morpholin-4-yl-2-oxo-1-(thien-2-ylmethyl)ethyl]pyrrolidin-2-one hydrochloride

Intermediate 9j) (190mg) was taken up in DCM (2ml). 4M HCI in Dioxan (0.68ml) was added, and the mixture stirred for 2 hours. Solvent was then evaporated under reduced pressure to give the title compound as the hydrochloride salt.
RT 1.74min, MH⁺ 324

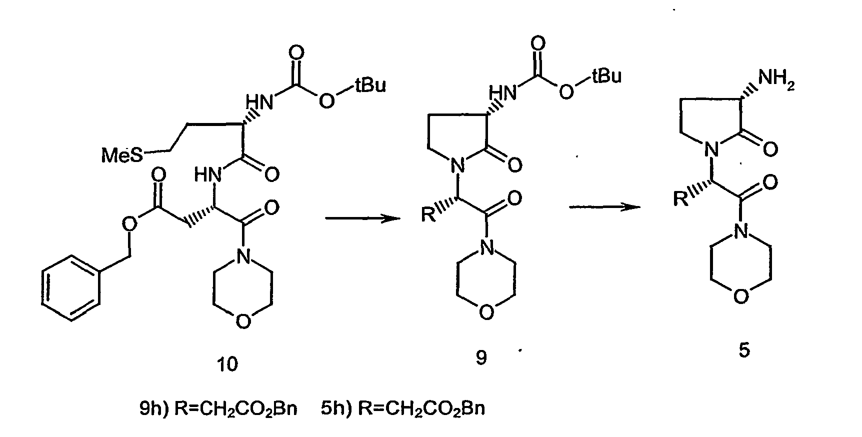

### Intermediate 10 Benzyl (S)-3-((S)-2-tert-butoxycarbonyamino-4-methylsulfonyl-butanoylamino)-4-morpholin-4-yl-4-oxo)butanoate

L-Boc-methionine (3.79g) was stirred in DMF at room temperature. TBTU (9.77g) was added followed by di-isopropylethylamine (7.9ml) and (3S)- benzyl (3*S*)-3-amino-4-morpholin-4-yl-4-oxobutanoate* (5g as the hydrochloride salt). After stirring for 3 hours, the mixture was quenched with sat. aq. Ammonium chloride (100ml) then partitioned between water (100ml) and ethyl acetate (100ml). The organic layer was washed with 2N sodium carbonate solution, 10% aq. Lithium chloride solution, then dried over sodium sulphate and solvent evaporated in vacuo. Purification via silica gel chromatography (ethyl acetate:cyclohexane 1:1 to 2:1) gave the title compound.
RT 3.02min, MH⁺ 524
* Hilpert et al, J. Med. Chem., 1994, 37, 3889-3901

### Intermediate 9h) Benzyl (3S)-3-{(3S)-3-[(tert-butoxycarbonyl)amino]-2-oxopyrrolidin-1-yl}-4-morpholin-4-yl-4-oxobutanoate

Intermediate 10 (6.0g) was stirred in acetone (35ml) and iodomethane (7.2ml) was added dropwise at room temperature. After stirring overnight, solvent was evaporated in vacuo to give the sulfonium iodide. The resulting sulfonium salt (7.6g) was not isolated but was stirred in acetonitrile (100ml). DOWEX (OH form) resin (8.3g) was added and the mixture stirred overnight. After filtration, solvent was evaporated under reduced pressure to give a colourless foam. Precipitation from ethyl acetate/cyclohexane gave the title compound.
RT 2.87min, MH⁺ 476

### Intermediate 5h) Benzyl (3S)-3-[(3S)-3-amino-2-oxopyrrolidin-1-yl]-4-moroholin-4-yl-4-oxobutanoate hydrochloride

Intermediate 9h) (4g) was stirred in a mixture of 1,4-dioxan and 4M HCl in 1-4-dioxan for 5 hours. After evaporation of solvent under reduced pressure, ether (30ml) was added, and the mixture triturated then solvent re-evaporated. The ether treatment was repeated three more times to give the title compound as the hydrochloride salt.
RT 1.93min, MH⁺ 376

### Intermediate 14 2-(2-bromoethyl)-5-chlorothiophene

To a solution of 2-(5-chloro-2-thienyl)-ethanol* (12.2 g) and triphenylphosphine (21.4 g) in anhydrous THF (150 ml) at 0°C was added carbon tetrabromide (27.5 g). The reaction was stirred at 5 °C for 15 minutes then at room temperature for 2.5 hours. Ether was added and the reaction was then filtered and the filtrate concentrated. The resultant residue was purified by silica gel chromatography eluting with 8:1 cyclohexane: DCM to give the title compound.
RT 3.50min* Schick et al., J.Amer. Chem. Soc., 70, 1948, 1646.

### Intermediate 15 2-(5-chlorothien-2-yl)ethanesulfonyl chloride

To a stirred solution of intermediate 14 (14 g) in acetone (125 ml) was added an aqueous solution of sodium sulfite (10.5 g in 125 ml of H₂O). The reaction was heated at reflux for 18 hours then concentrated to yield a pearly pink solid, which was dried under vacuum at 50 °C for 18 hours. A suspension of the salt in POCl₃ (90ml) was heated at 150 °C for 2.5 hours. The reaction was concentrated and DCM and water added to the resultant residue. The organic portion was collected, concentrated and the resultant oil purified by silica gel chromatography (7:3 petroleum ether: toluene) to yield a brown oil.
RT 3.33min

### Intermediate 16 1-(2-bromoethyl)-4-chlorobenzene

To a solution of 2-(4-chlorophenyl)ethanol (15 g, 95.8 mmol) in diethyl ether (225 ml) were added triphenylphosphine (31.2 g, 119 mmol) and carbon tetrabromide (38.4 g, 116 mmol). The mixture was stirred at room temperature for 16 hours, diluted with petroleum ether (bp 40-60 °C, 360 ml), and filtered. The filter cake was washed with a mixture of diethyl ether/petroleum ether (1:1, 250 ml). The filtrate was concentrated, and the residue distilled *in vacuo* to the title compound as a colourless oil:
bp 104-105°C (0.25 mbar); ¹H NMR (CDCl₃) δ 3.12 (t, 2H), 3.53 (t, 2H), 7.13 (d, 2H), 7.28 (d, 2H); ¹³C NMR (CDCl₃) δ 33.0, 39.0, 129.1 (2C), 130.4 (2C), 133.2, 137.7.

### Intermediate 17 Sodium 2-(4-chlorophenyl)ethanesulfonate

Intermediate 16 (17.3 g) was dissolved in 1,4-dioxane (50 ml) and added to a solution of sodium sulfite (13.5 g) in water (170 ml). The mixture was heated under reflux for 3 hours, then evaporated to dryness. The residual solid was washed with diethyl ether, and then recrystallised from water to give the title compound.

### Intermediate 18 2-(4-chlorophenyl)ethanesulfonyl chloride

Intermediate 17 (13.74 g) was suspended in a mixture of toluene (180 ml) and DMF (1.2 ml). Thionyl chloride (4.4 ml, 60.3 mmol) was added and the mixture was heated at 85°C for 3 hours. The mixture was cooled, then filtered through a celite pad. The filtrate was concentrated to one half the original volume, then chromatographed on silica gel using toluene as the eluent. Recrystallisation from petroleum ether gave the title compound as colourless needles.
mp 87.0-87.5°C; ¹H NMR (CDCl₃) δ 3.30-3.34 (2H, m), 3.87-3.91 (2H, m), 7.19 (2H, d), 7.34 (2H, d); ¹³C NMR (CDCl₃) δ 30.2, 66.3, 129.7, 130.3, 134.0, 134.4.

### Intermediate 19 Ethyl 2-(5-chlorothien-2-yl)-2-hydroxypropane-1-sulfonate

A solution of ethyl methanesulfonate (4.97g) in THF (20ml) was added dropwise to a solution of lithium hexamethyldisilylamine (42.0 ml of 1 M solution in THF plus 20ml of THF) at -78°C under nitrogen and the solution was stirred for 30 minutes. A solution of 2-acetyl-5-chlorothiophene (6.75g) in THF (70ml) was added to this over fifteen minutes and the temperature maintained at -78°C for 90 minutes. The reaction was quenched with 100ml of saturated aqueous ammonium chloride and the mixture extracted with 2 x 200ml of ethyl acetate. The combined organic fractions were washed with brine; dried (MgSO₄) and evaporated under reduced pressure to afford a crude oil that was purified by Biotage™ chromatography (4 x 90g) eluted with 1:3 ether:cyclohexane. The title compound was obtained as a colourless oil.
¹H NMR (CDCl₃): δ 6.79(1H, d), 6.73(1H, d), 4.26(2H, m), 4.14(1H, s), 3.32(1 H, d), 3.52(1 H, d), 1.8(3H, s), 1.36(3H, t) ppm.
RT 2.92 min, MH⁺-H₂O 267 M+NH₄⁺ 302.

### Intermediate 20 Ethyl 2-(4-chlorophenyl)-2-hydroxypropane-1-sulfonate

Prepared in similar fashion to intermediate 19 from ethyl methanesulfonate and 2-acetyl-4-chlorobenzene.
RT 2.88min M+NH₄⁺ 296

### Intermediate 21 Ethyl (1E)-2-(5-chlorothien-2-yl)prop-1-ene-1-sulfonate

A solution of intermediate 19 (10.9g) in DCM (300 ml) was cooled to 0°C under nitrogen, to which was added methanesulphonic acid (15.0ml) in a dropwise fashion. After stirring for 90 min, 200ml of saturated aqueous sodium bicarbonate was added, plus 50ml of water and 50 ml of brine. The layers were separated and the aqueous layer back extracted with 100 ml of DCM; the organics were combined, washed with brine and dried over magnesium sulphate and evaporated. The crude mixture was loaded onto an 800g Biotage™ column in 30 ml of chloroform and eluted with 15% *tert*butylmethyl ether in cyclohexane. The title compound was obtained as a white crystalline solid (Rf 0.5 1:1 ether cyclohexane) along with the unstable ethyl 2-(5-chlorothien-2-yl)prop-2-ene-1-sulfonate isomer (Rf 0.45 1:1 ether cyclohexane).
¹H NMR (CDCl₃): δ 7.16(1H, d), 6.92(1 H, d), 6.47(1H, d) 4.26(2H, q), 2.50(3H, d), 1.42 (3H, t) ppm.
RT 3.34min MH⁺ 267 M+NH₄⁺ 284

### Intermediate 22 Ethyl (1E)-2-(4-chlorophenyl)-proa-1-ene-1-sulfonate

Prepared in a similar fashion to intermediate 21 from intermediate 20 and methane sulphonic acid.
RT 3.31 min M+NH₄⁺ 278

### Intermediate 23 (1E)-2-(5-Chlorothien-2-yl)prop-1-ene-1-sulfonyl chloride

Tetrabutylammonium iodide (4.03g) was added to a solution of Intermediate 21 (2.9g) in acetone (180ml) under nitrogen and the solution heated under reflux for 17 hours. The solution was cooled and evaporated under reduced pressure to produce a yellow-brown solid. This was stirred in phosphorus oxychloride (30ml) at room temperature for 3.5 hours, after which the volatiles were evaporated and the residue coevaporated twice with toluene. The residue was applied, in 30ml chloroform, to 2 x 50g silica columns conditioned with chloroform. These were washed with 4x 40 ml of cyclohexane and eluted with 2 x 40 ml of 1:1 ether cyclohexane. The elution fractions were evaporated to yield the title compound as a yellow crystalline solid.
¹H NMR (CDCl₃): δ 7.31 (1 H, d), 6.99(1 H, d), 6.96(1H, q), 2.64(3H, d) ppm.
LCMS of a sample treated with 0.1 ml of 2M dimethylamine in THF afforded the clean dimethyl sulphonamide.
RT 3.22 minutes, MH⁺ 266.

### Intermediate 24 (1E)-2-(4-chlorophenyl)prop-1-ene-1-sulfonyl chloride

Prepared in similar fashion from intermediate 22. Treatment with dimethylamine gave the dimethylsulphonamide.
MX 260

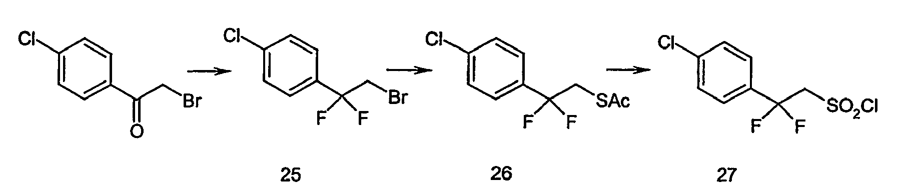

### Intermediate 25 1-(2-bromo-1,1-difluoroethyl)-4-chlorobenzene

1-(2-bromoacetyl)-4-chlorobenzene (4.0g) was stirred at 0°C in DCM (35ml). BAST (5.0ml) was added slowly and the mixture was stirred at 0°C for 1 hour then allowed to warm to room temperature and stirrd for a further 2 hours, then heated to 40°C for 4 hours. Purification by silica gel chromatography (cyclohexane:DCM 4:1) gave the title compound.
RT 3.44 min.

### Intermediate 26 S-[2-(4-chlorophenyl)-2,2-difluoroethyl] ethanethioate

A solution of intermediate 15 (1.5g) in DMF (27ml) was added to a solution of potassium thioacetate (1.3g) in DMF (90ml). The mixture was then stirred at 50°C for 18 hours. DCM (200ml) was added to the mixture together with water (200ml) and the layers separated. Evaporation of the organic layer under reduced pressure gave the title compound.
RT 3.41 min

### Intermediate 27 2-(4-chlorophenyl)-2,2-difluoroethanesulfonyl chloride

Chlorine gas was bubbled through water (250ml) in an icebath to give a yellow green solution. Intermediate 26 (536mg) was added as a chloroform solution (3ml). The reaction was stirred vigorously for 10 minutes then was allowed to warm to room temperature and was stirred for a further 5 minutes. After purging with nitrogen, the reaction mixture was extracted with chloroform. The organic portion was evaporated in vacuo to give the title compound as a white solid.
To confirm the nature of the product, a sample was treated with an excess of methylamine in THF, to give the expected sulphonamide.
RT 2.88min, M⁺ 270

### Intermediate 28 Sodium 2-(2,4-dichlorophenyl)ethanesulphonate

1-(2-bromoethyl)-2,4-chlorobenzene * (6.8g) was mixed with sodium sulphite (3.36g) in a 4:1 mixture of water:dioxan (50ml) and heated to 140°C overnight. A further 25ml of dioxan was added and reflux continued for a further 24 hours.. The mixture was then cooled to room temperature and concentrated in vacuo. The residue was triturated with diethyl ether and dried to give the title compound.
RT 3.68min [M-Na]+ 253.
* Sharafian et al., J. Het. Chem. 1994, 31, 6, 1421

### Intermediate 29 2-(2,4-dichlorophenyl)ethanesulfonyl chloride

A suspension of Intermediate 28 in toluene (90ml). DMF (0.58ml) was added, followed by thionyl chloride (2ml) over 5 minutes. After stirring for 4 hours, the mixture was cooled and filtered through celite, then concentrated under reduced pressure. The residue was purified by silica gel chromatography (toluene) to give the title compound.

### Intermediate 30 4-chloro-2-fluorostyrene

A solution of potassium t-butoxide (3.55g) was stirred in THF (40ml) at 0°C. Methyl triphenylphosphonium bromide (11.4g) was added and the mixture was stirred for 10 min at 0°C then at room temperature for 1 hour. After cooling to 10 °C, 4-chloro-2-fluorobenzaldehyde (4.2g) was added in THF (30ml) over 10 minutes and the mixture was stirred at room temperature for 3 hours. Toluene (20ml) was added and solvent volume reduced by half in vacuo. After addition of petroleum ether, the mixture was filtered and solvent removed in vacuo. The residue was purified by silica gel chromatography (toluene:petroleum ether 3:7) to give the title compound.
RT 3.52min

### Intermediate 31 2-(4-chloro-2-fluorophenyl)ethanol

To a 0.5M solution of 9-BBN in THF (50ml) was added intermediate 30 (3.2g) in THF (30ml). The mixture was stirred at room temperature for 18 hours, then cooled to 0°C. 10N NaOH (2.5ml) was added followed by hydrogen peroxide (30%, 7.7ml) keeping temperature<15°C. The mixture was cautiously heated to 50 °C for 2 hours, then cooled to 10 °C and sat. aq. sodium sulfite (21 ml) added. The organic layer was separated, and washed with sat. aq. sodium bicarbonate solution, then brine, then dried over magnesium sulphate. Solvent was evaporated under reduced pressure and the residue was purified via silica gel chromatography (DCM, then DCM:ethyl acetate 9:1) to give the title compound.

### Intermediate 32 1-(2-bromoethyl)-4-chloro-2-fluorobenzene

To a solution of intermediate 31 (2.8g) and 2,6-lutidine (0.44g) in ether (40ml) was added triphenylphosphine (5.2g) and carbon tetrabromide (6.4g) (with cooling to approx. 15°C). The mixture was stirred overnight at room temperature then diluted with petroleun ether (100ml) and filtered. The residue was concentrated in vacuo and then distilled under reduced pressure to give the title compound.
BPt 85-95°C @0.19mbar

### Intermediate 33 Sodium 2-(4-chloro-2-fluorophenyl)ethanesulphonate

Prepared from intermediate 32 according to the procedure for intermediate 28.
RT 3.48min, [M-Na]⁺ 237

### Intermediate 34 2-(4-chloro-2-fluorophenyl)ethanesulfonyl chloride

Prepared from intermediate 33 according to the procedure for intermediate 29.

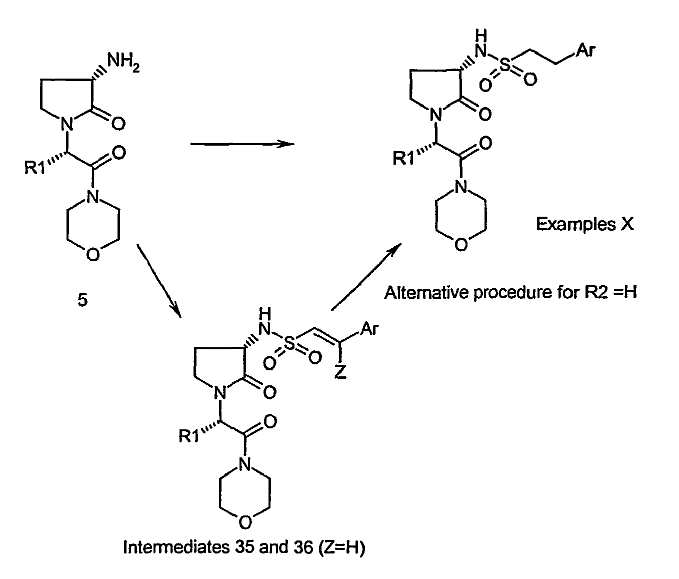

### Intermediate 35) (E)-2-(5-Chlorothien-2-yl)-N-{(3S)-1-[(1S)-1-methyl-2-morpholin-4-yl-2-oxoethyl]-2-oxopyrrolidin-3-yl}ethenesulfonamide

To a solution of intermediate 5a) (14.9g) in anhydrous acetonitrile (750ml) were added (E)-2-(5-chlorothien-2-yl)ethenesulfonyl chloride (16.5g) in acetonitrile (250ml) and pyridine (11 ml), and the mixture was stirred at room temperature for 72 hours. Saturated ammonium chloride solution was added and the resultant mixture stirred at room temperature for 30min. The mixture was concentrated under reduced pressure and the residue partitioned between chloroform and 1 N HCl. The organic layer was washed with a 1:1 mixture of saturated sodium bicarbonate solution and water, and brine. The organic layer was isolated, dried (over magnesium sulphate) and concentrated under reduced pressure to give the title compound as a white solid.
RT 2.71 min, MH⁺ 448

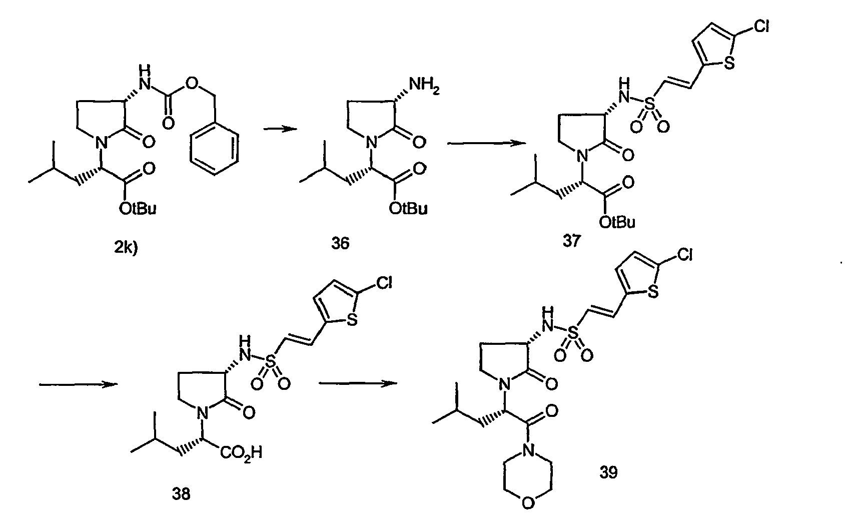

### Intermediate 36 tert-Butyl (2S)-2-[(3S)-3-amino-2-oxopyrrolidin-1-yl]-4-methylpentanoate

Prepared according to the process for intermediate 5a), from intermediate 2k).

### Intermediate 37 tert-Butyl (2S)-2-[(3S)-3-({[(E)-2-(5-chlorothien-2-yl)ethenyl]sulfonyl}amino)-2-oxopyrrolidin-1-yl]-4-methylpentanoate

Prepared according to the process for intermediate 35, from intermediate 36 and (E)-2-(5-chlorothien-2-yl)ethenesulfonyl chloride.
MH⁺ 477

### Intermediate 38 (2S)-2-[(3S)-3-({[(E)-2-(5-chlorothien-2-yl)ethenyl]sulfonyl}amino)-2-oxopyrrolidin-1-yl]-4-methylpentanoic acid

Prepared according to the process for intermediate 3a), from intermediate 37.
RT 3.24min, MH⁺ 421

### Intermediate 39 (E)-2-(5-chlorothien-2-yl)-N-{(3S)-1-[(1S)-3-methyl-1-(morpholin-4-ylcarbonyl)butyl]-2-oxopyrrolidin-3-yl}ethenesulfonamide

Prepared according to the process for intermediate 4a), from intermediate 38 and morpholine.
RT 3.07, MH⁺ 490

### Intermediate 40 Ethyl (1E)-2-(5-chlorothien-2-yl)-3,3,3-trifluoroprop-1-ene-1-sulfonate

A solution of ethyl (diethoxyphosphoryl)methanesulfonate (606mg) was stirred in THF at -78°C. n-Butyllithium (2.8mmol) was added as a 1.6M solution in hexanes and the mixture was stirred for 20 minutes. 2-Chloro-5-trifluoroacetylthiophene was then added as a 10ml THF solution. The mixture was stirred for a further 1 hour then was allowed to warm to room temperature. After partitioning between ethyl acetate and water, the organic portion was washed with brine, dried (sodium sulphate)and solvent evaporated in vacuo. Purification via silica gel chromatography (cyclohexane;ethyl acetate 50:1 to 19:1) gave the title compound which eluted separately from the geometrical isomer.
RT 3.50min, MH⁺ 338

### Intermediate 41 Tetra n-butylammonium (1E)-2-(5-chlorothien-2-yl)-3,3,3-trifluoroprop-1-ene-1-sulfonate

A solution of intermediate 40 (101 mg) was stirred in acetone and was treated with tetra n-butylammonium iodide (117mg). The mixture was heated to reflux overnight, then solvent evaporated in vacuo to give the title compound. RT 3.50min

### Intermediate 42 (1E)-2-(5-chlorothien-2-yl)-3,3,3-trifluoroprop-1-ene-1-sulfonyl chloride

Intermediate 41 (500mg) was treated with phosphorus oxychloride (3.6ml) and stirred at room temperature for 5 hours. The reaction was then evaporated in vacuo and azeotroped (x3) with toluene in vacuo. The crude product was purified via silica gel chromatography (cyclohexane) to give the title compound. RT 3.83min, MH⁺ 311

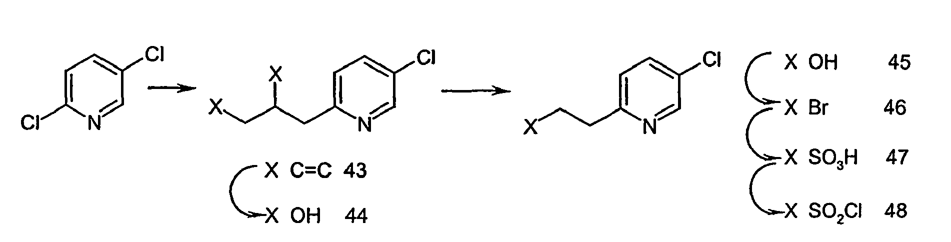

### Intermediate 43 5-Chloro-2-(2-propen-1-yl)pyridine

2,5-Dichloropyridine (2.4g), allyltributyltin (6.03ml) and bis triphenylphosphine palladium dichloride (0.46g) were mixed in DMF (30ml) and heated to 70°C for 1.5 hours, then to 90°C for 2.5 hours. The mixture was then cooled, concentrated to remove solvent, and partitioned between ethyl acetate and water (+ 5ml saturated aq. NaHCO₃ solution) and filtered through celite. The aqueous phase was reextracted with ethyl acetate, then the organics were combined, and washed with water and brine, then dried over K₂CO₃. Solvent was evaporated under reduced pressure, and the crude product purified via silica chromatography (DCM) to give the title compound.
RT 2.72min, MH⁺ 154

### Intermediate 44 3-(5-Chloro-2-pyridinyl)-1,2-propanediol

Intermediate 43 (0.7g) was taken up in acetone (30ml), water (15ml) and t-BuOH (7.5ml). N-methyl morpholine oxide (1.12g) was added followed by osmium tetroxide (0.5ml of a 2.5% solution in t-BuOH). The mixture was stirred at room temperature overnight. Aqueous sodium metabisulphite (10% solution, 50ml) was added (mixture cooled with an external cold-water bath) and the mixture was stirred for 30 minutes, followed by extraction with DCM (1x100ml, 1x50ml). The organic portions were washed with brine, dried over K₂CO₃ and solvent evaporated in vacuo to give the title compound.
RT 1.74min

### Intermediate 45 2-(5-Chloro-2-pyridinyl)ethanol

Intermediate 44 (0.33g) was taken up in THF (20ml) and sodium periodate (0.75g) was added as a 7ml aqueous solution. The mixture was stirred at room temperature for 2 hours, followed by partitioning of the mixture between water and diethyl ether. The organic portion was washed with brine, and then concentrated to approx. 10ml. THF (10ml) and water (4ml) were added, followed by sodium borohydride (0.132g) over 5 minutes. The mixture was stirred at room temperature for 90 minutes, then was diluted with water and diethyl ether and the mixture partitioned. The organic portion was washed with brine, then dried over K₂CO₃, followed by solvent evaporation under reduced pressure. Purification via silica chromatography (ethyl acetate) gave the title compound.
RT 1.83min, MH⁺ 158

### Intermediate 46 2-(2-Bromoethyl)-5-chloropyridine

Intermediate 45 (0.25g) was taken up in 20ml concentrated aqueous HBr, and the mixture was heated to reflux for 4 hours. The mixture was then cooled, and concentrated under reduced pressure, then the residue was partitioned between ethyl acetate and sat. aq. NaHCO₃. The organic layer was washed with brine, and dried over K₂CO₃. Solvent removal under reduced pressure gave the title compound. ¹H NMR (CDCl₃) δ 3.32 (t, 2H), 3.76 (t, 2H), 7.17 (d, 1 H), 7.62 (dd, 1H), 8.53 (d, 1H)

### Intermediate 47 2-(5-Chloro-2-pyridinyl)ethanesulfonic acid

Intermediate 46 (2.5g) was taken up in dioxane (15ml) and sodium sulphite (1.95g in 50ml water) was added. The mixture was heated to reflux for 1.5 hours. The mixture was then concentrated to dryness under reduced pressure, and the residue partitioned between water and diethyl ether. To the aqueous layer was added ammonium formate buffer to 150ml. The mixture was then chromatographed on a 10g Sep-Pak column (water:acetonitrile 50:1 to 4:1). Removal of solvent under reduced pressure gave the title compound.
RT 2.20min, MH⁺ 222

### Intermediate 48 2-(5-Chloro-2-pyridinyl)ethanesulfonyl chloride

Intermediate 47 (0.09g) was suspended in POCl₃ (0.4ml). Phosphorus pentachloride (0.12g) was added, and the mixture was stirred at 60°C for 2 hours. After cooling, solvent was evaporated under reduced pressure to give the title compound which was used without further purification.

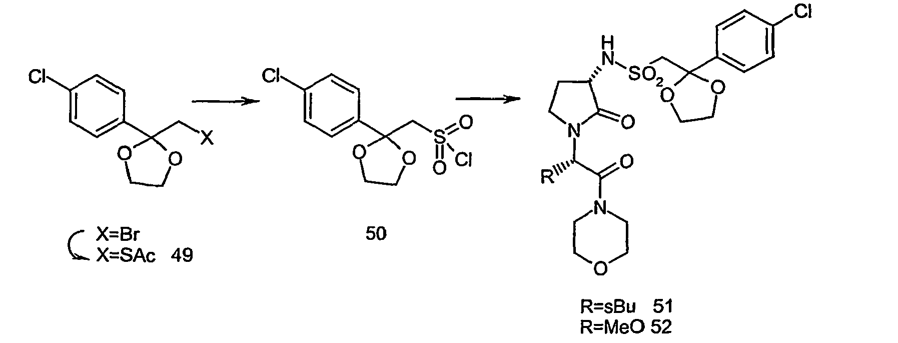

### Intermediate 49 S-{[2-(4-Chlorophenyl)-1,3-dioxolan-2-yl]methyl}ethanethioate

2-(Bromomethyl)-2-(4-chlorophenyl)-1,3-dioxoiane (1.0g) was taken up in DMF (15ml) and added to a solution of potassium thioacetate (774mg) in DMF (45ml). The reaction was stirred at 100°C overnight, then concentrated to half volume. DCM and aqueous LiCl solution were added, and the organic layer separated and concentrated. Purification via silica chromatography (cyclohexane:DCM 80:20 to 100% DCM) to give the title compound.
RT 3.30min, MH⁺ 273

### Intermediate 50 [2-(4-Chlorophenyl)-1,3-dioxolan-2-yl]methanesulfonyl chloride

Chlorine gas was passed through water (260ml) at 0°C to give a green solution. Intermediate 49 (660mg) was suspended in chloroform (6ml) and added to the chlorine solution. After stirring vigorously for 10 minutes, 100ml chloroform was added, and the mixture partitioned. Evaporation of solvent under vacuum gave the title compound.
RT 3.29min

### Intermediate 51 1-[2-(4-Chlorophenyl)-1,3-dioxolan-2-yl]-N-{(3S)-1-(1S,2S)-2-methyl-1-(4-morpholinylcarbonyl)butyl]-2-oxo-3-pyrrolidinyl}methanesulfonamide

Intermediate 5c) (257mg), DMAP (30mg) and DIPEA (235ul) were stirred in acetonitrile (3ml) at 0°C. Intermediate 50 (300mg) was added in acetonitrile (1ml) and the mixture was stirred for 1 hour. Solvent was then evaporated under vacuum, the residue was taken up in DCM and purified via silica chromatography (ethyl acetate) to give the title compound.
RT 3.05min, MH⁺ 544

Prepared in a similar fashion was:

### Intermediate 52 1-[2-(4-Chlorophenyl)-1,3-dioxolan-2-yl]-N-{(3S)-1-[(1S)-1-(methyloxy)-2-(4-morpholinyl)-2-oxoethyl]-2-oxo-3-pyrrolidinyl}methanesulfonamide

From Intermediate 5e) and Intermediate 50.
RT 2.69min, MH⁺ 532

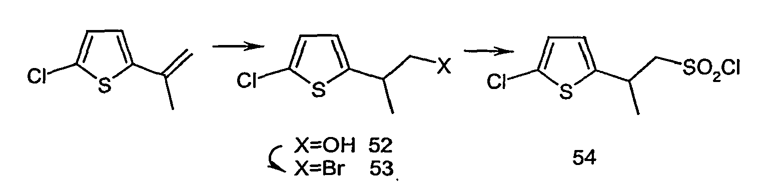

### Intermediate 52 2-(5-Chloro-2-thienyl)-1-propanol

2-Chloro-5-(1-methylethenyl)thiophene (2.29g) was added as a THF solution (12ml) to a solution of 9-BBN (0.5M) in THF (38ml) at room temperature. The mixture was then stirred for 18 hours at room temperature. The reaction mixture was then cooled to 0°C and aq. NaOH (10N, 1.9ml) was added, followed by careful addition of H₂O₂ (4.6ml of 30% by weight in water) (HAZARD) in 1ml portions (keeping temperature below 20 °C). The reaction mixture was then cautiously heated to 50 °C for 2 hours, followed by cooling to 0°C. The reaction mixture was quenched carefully by addition of aq. Na₂SO₃ (1.85g in 15ml). At this stage a negative peroxide test was obtained. Ethyl acetate (30ml) was added to the mixture, which was then partitioned, and the organic layer washed with brine, dried over Na₂SO₄, and solvent evaporated under reduced pressure. Purification via Biotage™ chromatography (cyclohexane:ethyl acetate 1:1) gave the title compound.
RT 2.88min

### Intermediate 53 2-(2-Bromo-1-methylethyl)-5-chlorothiophene

Intermediate 52 (1.0g) and PPh₃ (1.65g) were taken up in dry THF (12ml) and the mixture was cooled to 0°C under an inert atmosphere. Carbon tetrabromide (2.1 g) was added portionwise, the mixture was stirred for a further 15 minutes, then was allowed to warm to room temperature and was stirred for 18 hours. Cyclohexane (30ml) was added to the reaction mixture, which was stirred for 20 minutes then filtered. Solvent was evaporated under reduced pressure, and purification via SPE chromatography (cyclohexane) gave the title compound.
RT 3.63min

### Intermediate 54 2-(5-Chloro-2-thienyl)-1-propanesulfonyl chloride

Intermediate 53 (3.17g) was taken up in dioxan (25ml). Na₂SO₃ (22.25g) in water (40ml) was added, and the mixture was heated to reflux for 18 hours. The reaction was then cooled and solvent evaporated under reduced pressure to yield the crude sodium sulfonate. This material was dried under reduced pressure, then taken up in POCl₃ (15ml) and heated to 150 °C for 2 hours. Solvent was removed under reduced pressure, and the residue partitioned between DCM and water. The organic layer was reduced under vacuum, then purified via silica chromatography (cyclohexane, followed by DCM) to give the title compound.
RT 3.43min

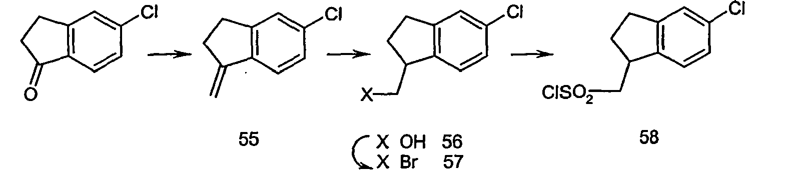

### Intermediate 55 5-Chloro-1-methylidene-2,3-dihydro-1H-indene

Methyltriphenylphosphonium bromide (3.0g) was suspended in ether (15ml) at 20°C. Potassium t-butoxide (954mg) was added, followed by 5-chloro-1-indanone (1.29g). After stirring for 24 hours, ether (20ml) and cyclohexane (50ml) were added, and the mixture was filtered. The filtrate was reduced under vacuum, and purified (Biotage™ chromatography, DCM:cyclohexane 20:80 to 50:50) to give the title compound.
¹H NMR (CDCl₃) δ 2.78 (m, 2H), 2.97 (m, 2H), 5.05 (t, 1 H), 5.43 (t, 1 H), 7.16 (m, 1H), 7.24 (br.s, 1 H), 7.40 (d, 1 H)

### Intermediate 56 (5-Chloro-2,3-dihydro-1H-inden-1-yl)methanol

To Intermediate 55 (510mg) in THF (4ml) was added 9-BBN (8ml of a 0.5M solution in hexane) dropwise. The mixture was stirred for 18 hours, then cooled to 0°C and 10N NaOH (0.4ml) added. Hydrogen peroxide (1.3ml of a 30% aqueous solution) was then added (keeping temperature<30 °C). The mixture was allowed to warm to room temperature, then was heated to 50°C for 2 hours. After cooling to room temperature, the mixture was quenched with sodium sulfite (1.5g in 15ml water) and stirred for 20 minutes. Partitioning between ethyl acetate and water followed by removal of organic solvent under vacuum gave the crude product. Purification via SPE chromatography (cyclohexane:ethyl acetate 4:1) gave the title compound.
RT 2.98

### Intermediate 57 1-(Bromomethyl)-5-chloro-2,3-dihydro-1H-indene

Intermediate 56 (518mg) was stirred in DCM (14ml), and triphenylphosphine (808mg) was added. The mixture was cooled to 0°C and carbon tetrabromide (1.02g) was added portionwise. After stirring for 20 minutes at 0°C, the mixture was allowed to warm to room temperature, and stirred for a further hour. Solvent was then removed under vacuum, and purification via Biotage™ chromatography (cyclohexane:DCM 4:1) gave the title compound.
¹H NMR (CDCl₃) δ 2.00 (m, 1 H), 2.40 (m, 1 H), 2.88 (m, 1H), 2.95 (m, 1H), 3.47 (m, 1 H), 3.55 (m, 1 H), 3.70 (dd, 1H), 7.20 (m, 3H)

### Intermediate 58 (5-Chloro-2,3-dihydro-1H-inden-1-yl)methanesulfonyl chloride

Intermediate 57 (522mg) was stirred in dioxan (4.5ml), and sodium sulfite (350mg in 6ml water) was added. The mixture was then heated at reflux for 20 hours. Solvent was then removed under vacuum to give the crude sodium sulfonate, used without further purification. This was taken up in POCl₃ (2.8ml) and heated to 140 °C. After 3 hours, the mixture was cooled and the residue partitioned between water and DCM. The organic layer was removed and solvent evaporated under vacuum. Purification via Biotage™ chromatography (cyclohexane:ethyl acetate 4:1) gave the title compound, which was used directly.

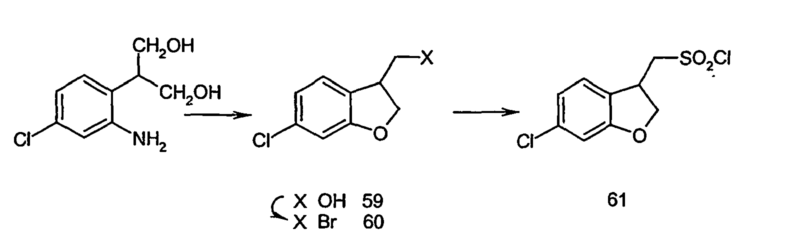

### Intermediate 59 (6-Chloro-2,3-dihydro-1-benzofuran-3-yl)methanol

2-(2-amino-4-chlorophenyl)-1,3-propanediol (700mg) was dissolved in a 3:1 mixture of water and concentrated sulphuric acid (15ml). The mixture was stirred vigorously and cooled to 0-5°C. Sodium nitrite (265mg) was added portionwise over a 10 minute period. Stirring was continued for 10 minutes, then for 90 minutes warming to room temperature. The temperature was then raised to 50 °C over 10 minutes, and the mixture stirred for a further 15 minutes. After cooling to room temperature, the mixture was extracted with DCM (2x 30ml), the organic portions were combined and dried (Na₂SO₄) and solvent evaporated under reduced pressure. Purification via SPE chromatography (cyclohexane: ethyl acetate 2:1) gave the title compound.
RT 2.68min

### Intermediate 60 3-(Bromomethyl)-6-chloro-2,3-dihydro-1-benzofuran

Prepared according to the process for Intermediate 57 from Intermediate 59. Purification via SPE chromatography (cyclohexane:DCM 4:1) gave the title compound.
RT 3.40min

### Intermediate 61 (6-Chloro-2,3-dihydro-1-benzofuran-3-yl)methanesulfonyl chloride

Intermediate 60 (160mg) was stirred in dioxan (3ml). A solution of sodium sulphite (107mg) in water (4ml) was added and the mixture was heated to reflux overnight. After cooling to room temperature, solvent was evaporated under reduced pressure, and the residue azeotroped twice with dioxan to give the crude sodium sulphonate. This material was stirred in POCl₃ (1ml) at 140°C for 3 hours, then allowed to cool and solvent evaporated under reduced pressure. Ice-water (10ml) was added, and the mixture was extracted with DCM (2x 15ml). The organic portions were dried (Na₂SO₄), and solvent evaporated under reduced pressure to give the title compound.
RT 3.35min

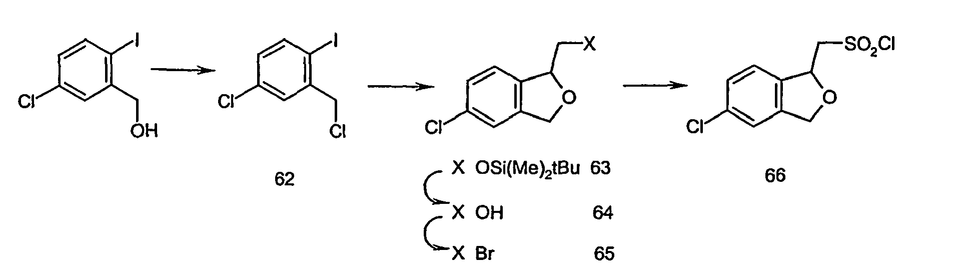

### Intermediate 62 4-Chloro-2-(chloromethyl)-1-iodobenzene

(5-Chloro-2-iodophenyl)methanol (2.9g) was taken up in dry DCM (30ml) and thionyl chloride (3ml). Pyridine (1 drop) was added and the mixture was heated to reflux overnight. After cooling to room temperature, the mixture was cautiously quenched with water, then partitioned between water (40ml) and DCM (30ml). The organic portion was separated and solvent removed under reduced pressure. Purification via Biotage™ chromatography (cyclohexane:DCM 100:0 to 90:10) gave the title compound.
¹H NMR (CDCl₃) δ 4.62 (s, 2H), 7.02 (dd, 1H), 7.49 (d, 1 H), 7.78 (d, 1 H)

### Intermediate 63 {[(5-Chloro-1,3-dihydro-2-benzofuran-1-yl)methyl]oxy}(1,1-dimethylethyl)dimethylsilane

4-Chloro-2-(chloromethyl)-1-iodobenzene (100mg) was stirred in dry THF (2ml) at room temperature under an inert atmosphere. Isopropylmagnesium bromide (1.15 equivalents) was added as a THF solution, and stirring was continued for 2 hours. {[(1,1-dimethylethyl)(dimethyl)silyl]oxy}acetaldehyde (1.2 equivalents) was added and the mixture was then heated to reflux overnight. The mixture was then partitioned between ethyl acetate and sat. aq. NaCl, and the organic phase dried over Na₂SO₄. After evaporation of solvent under reduced pressure, purification via SPE chromatography (cyclohexane, then cyclohexane: ethyl acetate 25:1) gave the title compound.
RT 4.04 MH⁺ 299

### Intermediate 64 (5-Chloro-1,3-dihydro-2-benzofuran-1-yl)methanol

Intermediate 63 (160mg) was taken up in dry THF (5ml). TBAF (1ml of 1 M solution in THF containing 5% water) was added and the mixture was stirred for 1 hour. The reaction mixture was then partitioned between ethyl acetate and water, and the organic phase dried (Na₂SO₄) and solvent removed in vacuo. Purification via SPE chromatography (cyclohexane: ethyl acetate 1:2) gave the title compound.
RT 2.37min

### Intermediate 65 1-(Bromomethyl)-5-chloro-1,3-dihydro-2-benzofuran

Prepared according to the process for Intermediate 57 from Intermediate 64. Purification via SPE chromatography (cyclohexane:DCM 3:1) gave the title compound.
RT 3.18min

### Intermediate 66 (5-Chloro-1,3-dihydro-2-benzofuran-1-yl)methanesulfonyl chloride

Prepared according to the procedure for Intermediate 61 from Intermediate 65. RT 3.28min

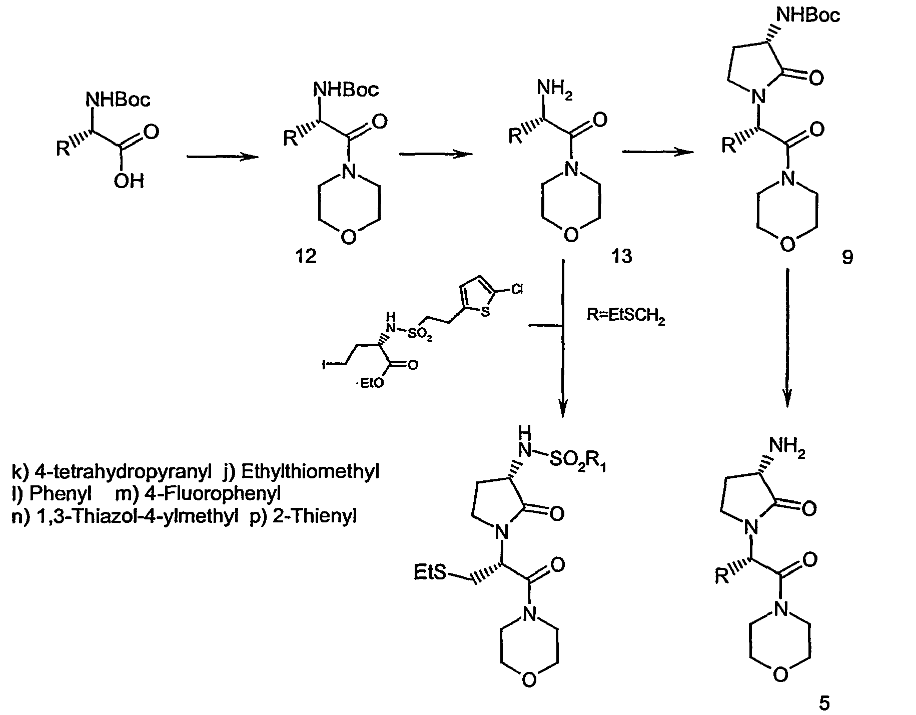

### Intermediate 12k) 1,1-Dimethylethyl [2-(4-morpholinyl)-2-oxo-1-(tetrahydro-2H-pyran-4-yl)ethyl]carbamate

Prepared from ({[(1,1-dimethylethyl)oxy]carbonyl}amino)(tetrahydro-2*H*-pyran-4-yl)acetic acid according to the method for Intermediate 11. RT 2.23min, MH⁺ 329

Also prepared in the same manner were:

### Intermediate 12j) 1,1-Dimethylethyl [(1R)-1-[(ethylthio)methyl]-2-(4-morpholinyl)-2-oxoethyl]carbamate

From *N*-{[(1,1-dimethylethyl)oxy]carbonyl}-*S*-ethyl-L-cysteine
RT 2.62min, MH⁺ 319

### Intermediate 12l) 1,1-Dimethylethyl [(1S)-2-(4-morpholinyl)-2-oxo-1-phenylethyl]carbamate

From (2*S*)-({[(1,1-dimethylethyl)oxy]carbonyl}amino)(phenyl)acetic acid
RT 2.73min, MH⁺ 321

### Intermediate 12m) 1,1-Dimethylethyl [(1S)-1-[(4-fluorophenyl)methyl]-2-(4-morpholinyl)-2-oxoethyl]carbamate

From *N*-{[(1,1-dimethylethyl)oxy]carbonyl}-4-fluoro-L-phenylalanine.
RT 2.85min, MH⁺ 353

### Intermediate 12n) 1,1-Dimethylethyl [(1S)-2-(4-morpholinyl)-2-oxo-1-(1,3-thiazol-4-ylmethyl)ethyl]carbamate

From *N*-{[(1,1-dimethylethyl)oxy]carbonyl}-3-(1,3-thiazol-4-yl)-L-alanine.
RT 2.28min, MH⁺ 342

### Intermediate 12p) 1,1-Dimethylethyl [(1R)-2-(4-morpholinyl)-2-oxo-1-(2-thienyl)ethyl]carbamate

From (2*R*)-({[(1,1-dimethylethyl)oxy]carbonyl}amino)(2-thienyl)acetic acid
RT 2.75min, MH⁺ 327

### Intermediate 13k) 2-(4-Morpholinyl)-2-oxo-1-(tetrahydro-2H-pyran-4-yl)ethanamine

Prepared from Intermediate 12k) according to the method for Intermediate 5f). RT 0.54min, MH⁺ 229

Also prepared in the same manner were:

### Intermediate 13j) (2R)-3-(Ethylthio)-1-(4-morpholinyl)-1-oxo-2-propanamine

From Intermediate 12j)
RT 0.94min, MH⁺ 219

### Intermediate 13I) (1S)-2-(4-Morpholinyl)-2-oxo-1-phenylethanamine

From Intermediate 12I)
RT 1.18min, MH⁺ 221

### Intermediate 13m) (2S)-3-(4-Fluorophenyl)-1-(4-morpholinyl)-1-oxo-2-propanamine

From Intermediate 12m)
RT 1.66min, MH⁺ 252

### Intermediate 13n) (2S)-1-(4-Morpholinyl)-1-oxo-3-(1,3-thiazol-4-yl)-2-propanamine

From Intermediate 12n)
RT 0.68min, MH⁺ 242

### Intermediate 13p) (1R)-2-(4-Morpholinyl)-2-oxo-1-(2-thienyl)ethanamine

From Intermediate 12p)
RT 0.90min, MH⁺ 227

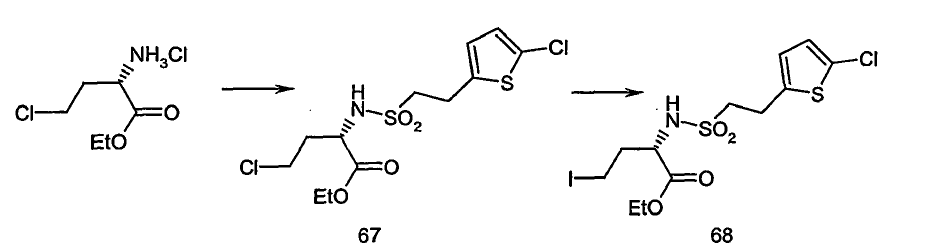

### Intermediate 67 Ethyl (2S)-4-chloro-2-({[2-(5-chloro-2-thienyl)ethyl]sulfonyl}amino)butanoate

Prepared from ethyl (2*S*)-2-amino-4-chlorobutanoate hydrochloride and Intermediate 15 according to the method for preparation of Example 15.
RT 3.45min, MH⁺ 375

### Intermediate 68 Ethyl (2S)-2-({[(E)-2-(5-chloro-2-thienyl)ethyl]sulfonyl}amino)-4-iodobutanoate

Intermediate 67 (570mg) was taken up in acetone (3ml) and sodium iodide (900mg) was added to the solution. The mixture was heated to 80°C for 6 hours, then cooled and filtered, and the filtrate evaporated under reduced pressure, to give the title compound (plus approx. 15% starting material).
RT 3.44min, MH⁺ 465

### Intermediate 9k) 1,1-Dimethylethyl {(3S)-1-[2-(4-morpholinyl)-2-oxo-1-(tetrahydro-2H-pyran-4-yl)ethyl]-2-oxo-3-pyrrolidinyl}carbamate

Prepared in a similar manner to Intermediate 9f) from Intermediate 13k).
RT 2.40min, MH⁺ 412

Also prepared in the same manner were:

### Intermediate 9l) 1,1-Dimethylethyl {(3S)-1-[(1S)-2-(4-morpholinyl)-2-oxo-1-phenylethyl]-2-oxo-3-pyrrolidinyl}carbamate

From Intermediate 13l).
RT 2.64min, MH⁺ 404

### Intermediate 9m) 1,1-Dimethylethyl {(3S)-1-[(1S)-1-[(4-fluorophenyl)methyl]-2-(4-morpholinyl)-2-oxoethyl]-2-oxo-3-pyrrolidinyl}carbamate

From Intermediate 13m).
RT 2.79min, MH⁺ 436

### Intermediate 9n) 1,1-Dimethylethyl {(3S)-1-[(1S)-2-(4-morpholinyl)-2-oxo-1-(1,3-thiazol-4-ylmethyl)ethyl]-2-oxo-3-pyrrolidinyl}carbamate

From Intermediate 13n)
RT 2.30min, MH⁺ 425

### Intermediate 9p) 1,1-Dimethylethyl {(3S)-1-[(1R)-2-(4-morpholinyl)-2-oxo-1-(2-thienyl)ethyl]-2-oxo-3-pyrrolidinyl}carbamate

From Intermediate 13p).
RT 2.60min, MH⁺ 410

### Intermediate 5k) (3S)-3-Amino-1-[2-(4-morpholinyl)-2-oxo-1-(tetrahydro-2H-pyran-4-yl)ethyl]-2-pyrrolidinone hydrochloride

Prepared according to the procedure for Intermediate 5f) from Intermediate 9k). RT 1.02min, MH⁺ 312

Prepared in a similar manner were:

### Intermediate 5l) (3S)-3-Amino-1-[(1S)-2-(4-morpholinyl)-2-oxo-1-phenylethyl]-2-Pyrrolidinone hydrochloride

From Intermediate 9l).
RT 1.83min, MH⁺ 304

### Intermediate 5m) (3S)-3-Amino-1-[(1S)-1-[(4-fluorophenyl)methyl]-2-(4-moroholinyl)-2-oxoethyl]-2-pyrrolidinone hydrochloride

From Intermediate 9m).
RT 1.97min, MH⁺ 336

### Intermediate 5n) (3S)-3-Amino-1-[(1S)-2-(4-morpholinyl)-2-oxo-1-(1,3-thiazol-4-ylmethyl)ethyl]-2-pyrrolidinone hydrochloride

From Intermediate 9n).
RT 1.50min, MH⁺ 325

### Intermediate 5p) (3S)-3-Amino-1-[(1R)-2-(4-morpholinyl)-2-oxo-1-(2-thienyl)ethyl]-2 Pyrrolidinone hydrochloride

From Intermediate 9p).
RT 1.70min, MH⁺ 310

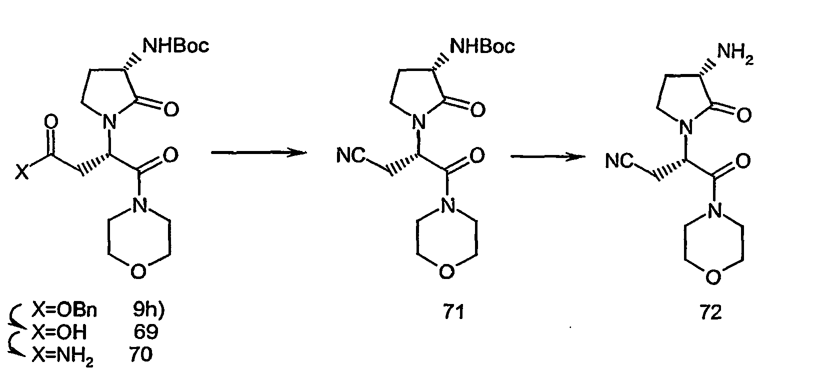

### Intermediate 69 (3S)-3-[(3S)-3-({[(1,1-Dimethylethyl)oxy]carbonyl}amino)-2-oxo-1-pyrrolidinyl]-4-(4-morpholinyl)-4-oxobutanoic acid

Intermediate 9h) (5.0g) was taken up in acetic acid (75ml), and was hydrogenated at atmospheric pressure and temperature over 10% Pd/C (500mg, 50%wt H₂0) for 2 hours. The mixture was filtered through Celite, which was washed with acetic acid. The filtrate was evaporated under reduced pressure, then co-evaporated three times with toluene (50ml) to give the title compound.
RT 2.18min, MH⁺ 386

### Intermediate 70 1,1-Dimethylethyl {(3S)-1-[(1S)-3-amino-1-(4-morpholinylcarbonyl)-3-oxopropyl]-2-oxo-3-pyrrolidinyl}carbamate

Intermediate 69 (0.5g), TBTU (0.5g) and DIPEA (0.44ml) were stirred together in dry DMF (8ml)at room temperature. After 5 minutes, 2M methanolic NH₃ (1ml) was added, and the mixture was stirred for a further 2 hours. 4g of MP-carbonate scavenger resin (Argonaut) was added sequentially, and the mixture was stirred for a further 2 hours. The mixture was then filtered, and solvent evaporated under reduced pressure. Purification via SPE chromatography (DCM:MeOH 20:1 to 10:1) gave the title compound.
RT 2.06min, MH⁺ 385

### Intermediate 71 1,1-Dimethylethyl {(3S)-1-[(1S)-1-(cyanomethyl)-2-(4-morpholinyl)-2-oxoethyl]-2-oxo-3-pyrrolidinyl}carbamate

Intermediate 70 (350mg) was stirred in dry THF (3.5ml) at 0°C under inert atmosphere. Triethylamine (0.38ml) was added, followed by dropwise addition of trifluoroacetic anhydride (0.19ml). After stirring for 5 minutes, the mixture was allowed to warm to room temperature, and was stirred for a further hour. A further portion of triethylamine (0.127 ml) and trifluoroacetic anhydride (0.064ml) were added, and stirring continued for 1 hour. The mixture was then partitioned between ethyl acetate and sat. aq. NaHCO₃, and the organic portion dried (Na₂SO₄), then solvent evaporated under reduced pressure. Purification via SPE chromatography gave the title compound.
RT 2.28min, MH⁺ 367

### Intermediate 72 (3S)-3-[(3S)-3-Amino-2-oxo-1-pyrrolidinyl]-4-(4-morpholinyl)-4-oxobutanenitrile hydrochloride

Prepared according to the procedure for Intermediate 5e) from Intermediate 71. This gave the title compound as a 7:3 mixture with the corresponding primary amide. RT 0.38min, MH⁺ 267 (plus MH⁺ 285 co-eluting, representing the amide side-product).

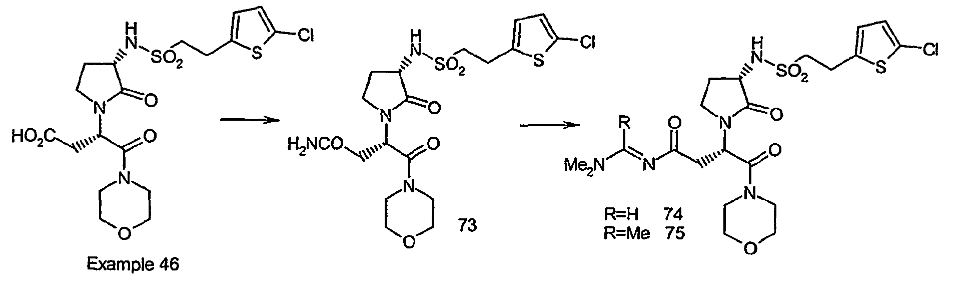

### Intermediate 73 (3S)-3-[(3S)-3-({[2-(5-Chloro-2-thienyl)ethyl]sulfonyl}amino)-2-oxo-1-Pyrrolidinyl]-4-(4-morpholinyl)-4-oxobutanamide

Example 46 (850mg) and TBTU (1.105g) were stirred in dry DMF (25ml) at room temperature. DIPEA (0.6ml) was added, followed by 2M ammonia in methanol and the mixture was stirred for 4 hours. After quenching with sat. aq.NH4Cl solution (25ml), the mixture was partitioned between ethyl acetate (50ml) and water (25ml). The aqueous phase was further extracted with 3x ethyl acetate, and the combined organic phases washed sequentially with 2N Na₂CO₃ solution, 1N HCl solution and water. Evaporation of the organic solvent under vacuum gave the crude product which was purified via SPE chromatography (DCM:MeOH 50:1 to 10:1) to give the title compound.
RT 2.51min, MH⁺ 493

### Intermediate 74 (3S)-3-[(3S)-3-({[2-(5-Chloro-2-thienyl)ethyl]sulfonyl}amino)-2-oxo-1-pyrrolidinyl]-N-[(1E)-(dimethylamino)methylidene]-4-(4-morpholinyl)-4-oxobutanamide

Intermediate 73 (100mg) was stirred with dimethylformamide dimethyl acetal (2ml) and heated to 60°C. DMF (0.1ml) was added to aid dissolution. After 3 hours, solvent was evaporated under vacuum to give the title compound.
RT 2.37min, MH⁺ 548

In a similar manner was prepared:

### Intermediate 75 (3S)-3-[(3S)-3-({[2-(5-Chloro-2-thienyl)ethyl]sulfonyl}amino)-2-oxo-1-pyrrolidinyl]-N-[(1Z)-1-(dimethylamino)ethylidene]-4-(4-morpholinyl)-4-oxobutanamide

From Intermediate 73 and dimethylacetamide dimethyl acetal. Isolated as a mixture with (3*S*)-3-{(3*S*)-3-[{[2-(5-chloro-2-thienyl)ethyl]sulfonyl}(methyl)amino]-2-oxo-1-pyrrolidinyl}-*N*-[(1*E*)-(dimethylamino)methylidene]-4-(4-morpholinyl)-4-oxobutanamide
RT 2.33min, MH⁺ 562 (+RT 2.46min, MH⁺ 576)

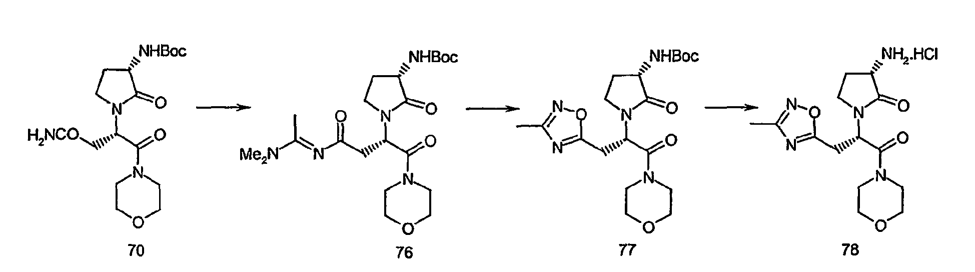

### Intermediate 76 1,1-Dimethylethyl {(3S)-1-[(1S)-3-{[(1E)-1-(dimethylamino)ethylidene]amino}-1-(4-morpholinylcarbonyl)-3-oxopropyl]-2-oxo-3-pyrrolidinyl}carbamate

Intermediate 70 (500mg) was taken up in 0.3ml of DMF, and N,N-dimethylacetamide dimethylacetal (3ml) was added. The reaction was stirred at room temperature for 3 hours, then heated to 40 °C for 90 minutes. Evaporation of solvent under reduced pressure gave the title compound.
RT 1.99min, MH⁺ 454

### Intermediate 77 1,1-Dimethylethyl {(3S)-1-[(1S)-1-[(3-methyl-1,2,4-oxadiazol-5-yl)methyl]-2-(4-morpholinyl)-2-oxoethyl]-2-oxo-3-pyrrolidinyl}carbamate

Prepared according to the procedure for Example 79 from Intermediate 76 and hydroxylamine hydrochloride. Purification via SPE chromatography (DCM:MeOH 15:1) gave the title compound.
RT 2.36min, MH⁺ 424

### Intermediate 78 (3S)-3-Amino-1-[(1S)-1-[(3-methyl-1,2,4-oxadiazol-5-yl)methyl]-2-(4-morpholinyl)-2-oxoethyl]-2-pyrrolidinone hydrochloride

Prepared according to the procedure for 5e) from Intermediate 77. RT 1.10min, MH⁺ 324

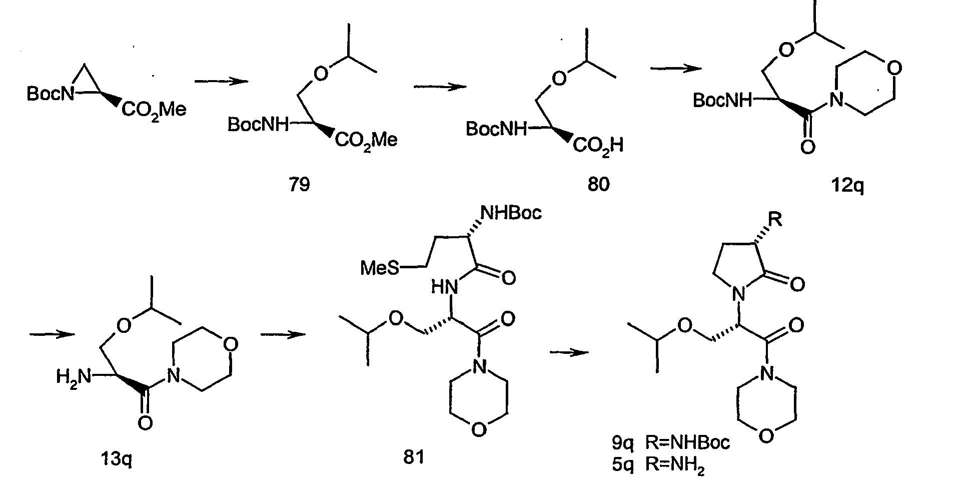

### Intermediate 79 Methyl N-{[(1,1-dimethylethyl)oxy]carbonyl}-O-(1-methylethyl)-L-serinate

1-(1,1-Dimethylethyl) 2-methyl (2*S*)-1,2-aziridinedicarboxylate (2.0g) was taken up in a mixture of chloroform (30ml) and isopropanol (35ml). BF₃ etherate (6 drops) was added and the resulting solution was stirred at room temperature for 20 hours. Solvent was evaporated under vacuum and the crude product purified via SPE chromatography (cyclohexane: ethyl acetate 6:1) to give the title compound.
¹H NMR (CDCl₃) δ 1.12 (m, 6H), 1.45 (s, 9H), 3.55 (m, 1H), 3.63 (dd, 1H), 3.76 (s, 3H), 3.83 (dd, 1 H), 4.4 (m, 1 H), 5.36 (broad d, 1 H)

### Intermediate 80 N-{[(1,1-Dimethylethyl)oxy]carbonyl}-O-(1-methylethyl)-L-serine

Intermediate 79 (1.5g) was taken up in THF (15ml) and treated with water (7.5ml) and 2N NaOH (3.45ml). The resulting mixture was stirred for 2 hours, then acidified to pH 1-2 with 2N HCl. The mixture was extracted with ethyl acetate (2x 25ml), the organics were combined and washed with brine (10ml). Solvent was evaporated under vacuum to give the title compound.
RT 2.62min, MH⁺ 248

### Intermediate 12q 1,1-Dimethylethyl [(1S)-1-{[(1-methylethyl)oxy]methyl}-2-(4-morpholinyl)-2-oxoethyl]carbamate

Prepared in a similar fashion to Intermediate 9e) from Intermediate 80 and morpholine.
¹H NMR (CDCl₃) δ 1.12 (t, 6H), 1.43 (s, 9H), 3.45 (t, 1 H), 3.50-3.75 (m, 9H), 3.80 (br. d, 1 H), 4.75 (m, 1H)

### Intermediate 13q) (2S)-3-[(1-Methylethyl)oxy]-1-(4-morpholinyl)-1-oxo-2-propanamine

Prepared in a similar fashion to Intermediate 5e) from Intermediate 12q). RT 1.02min, MH⁺ 217

### Intermediate 81 1,1-Dimethylethyl [(1S)-1-({[(1S)-1-{[(1-methylethyl)oxy]methyl}-2-(4-morpholinyl)-2-oxoethyl]amino}carbonyl)-3-(methylthio)propyl]carbamate

Prepared in a similar fashion to Intermediate 6 from Intermediate 13q) and Boc-L-methionine.
RT 2.70min, MH⁺ 448

### Intermediate 9q) 1,1-Dimethylethyl {(3S)-1-[(1S)-1-{[(1-methylethyl)oxy]methyl}-2-(4-morpholinyl)-2-oxoethyl]-2-oxo-3-pyrrolidinyl}carbamate

Prepared in a similar fashion to Intermediate 7 from Intermediate 81. RT 2.46min, MH⁺ 400

### Intermediate 5q) (3S)-3-Amino-1-[(1S)-1-{[(1-methylethyl)oxy]methyl}-2-(4-morpholinyl)-2-oxoethyl]-2-pyrrolidinone

Prepared in a similar fashion to Intermediate 5e), from Intermediate 9q). Purification via SCX column (eluting with methanolic ammonia) provided the title compound as the free base.
RT 1.63min, MH⁺ 300

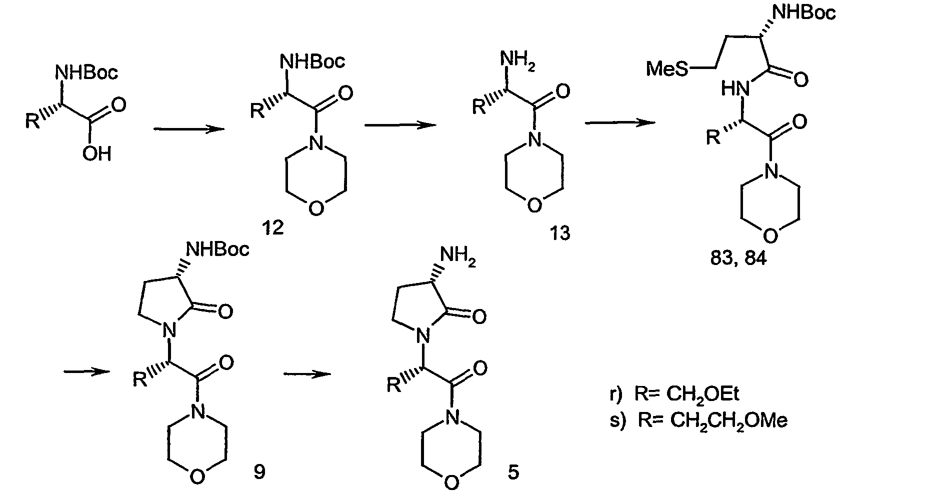

### Intermediate 12r) 1,1-Dimethylethyl [(1S)-1-[(ethyloxy)methyl]-2-(4-morpholinyl)-2-oxoethyl]carbamate

*N*-{[(1,1-Dimethylethyl)oxy]carbonyl}-*O*-ethylserine (3.89g) in dry DCM (100ml) was cooled to 4 °C under an inert atmosphere. Morpholine (2.61ml) and DIPEA (5.21ml) were added, and the mixture was stirred for 5 minutes. TBTU (9.64g) was then added in portions over 10 minutes. After stirring for a further 15 minutes, the mixture was then allowed to warm to room temperature and stirred for a further 12 hours. The reaction was extracted with sat aq. NaHCO₃ (75ml) and the aqueous layer washed with DCM (50ml). The organics were combined, dried (Na₂SO₄) and solvent evaporated under reduced pressure. Purification via SPE chromatography (ethyl acetate) gave the title compound.
RT 2.36min, MH⁺ 303

### Intermediate 12s) 1,1-Dimethylethyl [(1S)-3-(methyloxy)-1-(4-morpholinylcarbonyl)propyl]carbamate

Prepared according to the procedure for Intermediate 12r) from *N*-{[(1,1-dimethylethyl)oxy]carbonyl}-O-methylhomoserine
RT 2.26min, MH⁺ 203

### Intermediate 13r) [(1S)-1-[(Ethyloxy)methyl]-2-(4-morpholinyl)-2-oxoethyl]amine

Prepared from Intermediate 12r) according to the procedure for intermediate 5f). RT 0.38min, MH⁺ 203

### Intermediate 13s) (2S)-4-(Methyloxy)-1-(4-moroholinyl)-1-oxo-2-butanamine

Prepared from Intermediate 12s) according to the procedure for Intermediate 5f), using ethyl acetate as solvent. RT 0.42min, MH⁺ 203

### Intermediate 83 1,1-Dimethylethyl [(1S)-1-({[(1S)-1-[(ethyloxy)methyl]-2-(4-morpholinyl)-2-oxoethyl]amino}carbonyl)-3-(methylthio)propyl]carbamate

Prepared in a similar fashion to Intermediate 6 from Intermediate 13r) and Boc-L-methionine.
RT 2.56min, MH⁺ 434

### Intermediate 84 1,1-Dimethylethyl [(1S)-1-({[(1S)-3-(methyloxy)-1-(4-morpholinylcarbonyl)propyl]amino}carbonyl)-3-(methylthio)propyl]carbamate

Prepared in a similar fashion to intermediate 6 from Intermediate 13s) and Boc-L-methionine.
RT 2.46min, MH⁺ 434

### Intermediate 9r) 1,1-Dimethylethyl {(3S)-1-[(1S)-1-[(ethyloxy)methyl]-2-(4-morpholinyl)-2-oxoethyl]-2-oxo-3-pyrrolidinyl}carbamate

Prepared in a similar fashion to Intermediate 7 from Intermediate 83. Purification via SPE chromatography (ethyl acetate) gave the title compound as a single isomer. RT 2.35min, MH⁺ 386

### Intermediate 9s) 1,1-Dimethylethyl {(3S)-1-[(1S)-3-(methyloxy)-1-(4-morpholinylcarbonyl)propyl]-2-oxo-3-pyrrolidinyl}carbamate

Prepared in a similar fashion to Intermediate 7 from Intermediate 84. Purification via SPE chromatography (ethyl acetate to ethyl acetate:MeOH 5:1) gave the title compound as a single isomer.
RT 2.26min, MH⁺ 386

### Intermediate 5r) (3S)-3-Amino-1-[(1S)-1-[(ethyloxy)methyl]-2-(4-morpholinyl)-2-oxoethyl]-2-pyrrolidinone hydrochloride

Prepared in a similar fashion to Intermediate 5e), from Intermediate 9r). RT 1.26min, MH⁺ 286

### Intermediate 5s) (3S)-3-Amino-1-[(1S)-3-(methyloxy)-1-(4-morpholinylcarbonyl)propyl]-2-pyrrolidinone

Prepared in a similar fashion to Intermediate 5f), from Intermediate 9s). RT 0.98min, MH⁺ 286

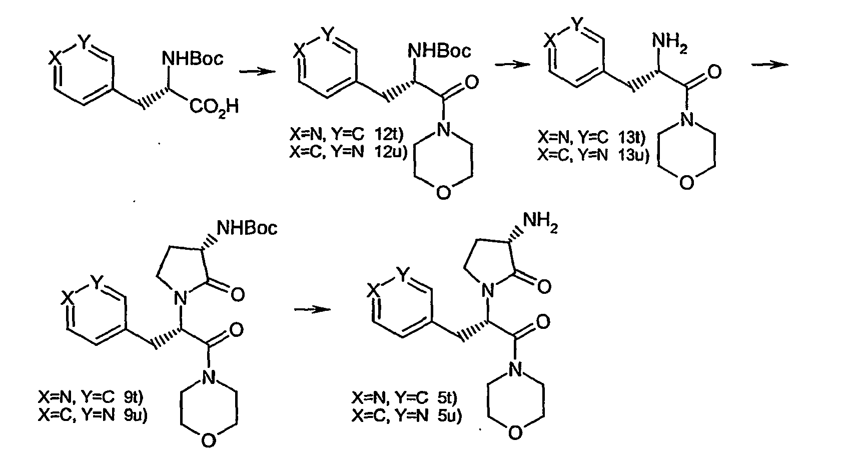

### Intermediate 12t) 1,1-Dimethylethyl [(1S)-2-(4-morpholinyl)-2-oxo-1-(4-pyridinylmethyl)ethyl]carbamate

To a suspension of Boc-3-(4-pyridyl)-L-alanine (0.5g) in DCM (20ml) at 0°C was added morpholine (0.245g) and DIPEA (0.49ml). TBTU (0.9g) was then added portionwise over 10 minutes. The mixture was stirred for 30 minutes at 0°C then was allowed to warm to room temperature and was stirred overnight. The crude product was then purified via SPE chromatography (ethyl acetate:MeOH 15:1). Following solvent removal under vacuum, the residue was taken up in ethyl acetate (50ml) and washed sequentially with sat. aq. NaHCO₃, water and brine. Evaporation of solvent under vacuum gave the title compound.
RT 1.82min, MH⁺ 336

In a similar manner was prepared:

### Intermediate 12u) 1,1-Dimethylethyl [(1S)-2-(4-morpholinyl)-2-oxo-1-(3-pyridinylmethyl)ethyl]carbamate

From Boc-3-(3-pyridyl)-L-alanine and morpholine.
RT 1.95min, MH⁺ 336

### Intermediate 13t) [(1S)-2-(4-Morpholinyl)-2-oxo-1-(4-pyridinylmethyl)ethyl]amine

Intermediate 12t) (0.54g) in ethyl acetate (10 ml) was stirred at room temperature and treated with 4M HCl in dioxan (2.4ml). A further 10ml dioxan and 15ml methanol was added, and the mixture was stirred overnight. Solvent was then evaporated under vacuum. The residue was taken up in 20ml methanol and purified via SCX column (eluting with methanol, then 2M ammonia in methanol). Evaporation of solvent under vacuum gave the title compound.
RT 0.42min, MH⁺ 236

In a similar manner was prepared:

### Intermediate 13u) [(1S)-2-(4-Morpholinyl)-2-oxo-1-(3-pyridinylmethyl)ethyl]amine

From Intermediate 12u)
RT 1.95min, MH⁺ 336

### Intermediate 9t) 1,1-Dimethylethyl {(3S)-1-[(1S)-2-(4-morpholinyl)-2-oxo-1-(4-Pyridinylmethyl)ethyl]-2-oxo-3-pyrrolidinyl}carbamate

Intermediate 13t) (84mg) in DCM (2ml) was treated with powdered 3A molecular sieves (200mg) and a solution of 2-tert-butoxycarbonylamino-4-oxo-butyric acid benzyl ester (100mg) in DCM (3ml). After stirring at room temperature for 40 minutes, acetic acid (0.12ml) was added, followed by sodium triacetoxyborohydride (90mg). After stirring for 4 days, the mixture was purified via SPE chromatography (ethyl acetate:methanol 5:1) to give the title compound.
RT 1.92min, MH⁺ 419

In a similar manner was prepared:

### Intermediate 9u) 1,1-Dimethylethyl {(3S)-1-[(1S)-2-(4-morpholinyl)-2-oxo-1-(3-pyridinylmethyl)ethyl]-2-oxo-3-pyrrolidinyl}carbamate

From Intermediate 13u) and 2-tert-butoxycarbonylamino-4-oxo-butyric acid benzyl ester.
RT 1.98min, MH⁺ 419

### Intermediate 5t) (3S)-3-Amino-1-[(1S)-2-(4-morpholinyl)-2-oxo-1-(4-Pyridinylmethyl)ethyl]-2-pyrrolidinone dihydrochloride

To a solution of Intermediate 9t) (100mg) in DCM (2ml) was added 4M HCl in dioxan (0.36ml). The mixture was stirred overnight at room temperature. Solvent was then evaporated under vacuum to give the title compound.
RT 0.42min, MH⁺ 319

In a similar manner was prepared:

### Intermediate 5u) (3S)-3-Amino-1-[(1S)-2-(4-morpholinyl)-2-oxo-1-(3-pyridinylmethyl)ethyl]-2-pyrrolidinone dihydrochloride

From Intermediate 9u)
RT 0.38min, MH⁺ 319

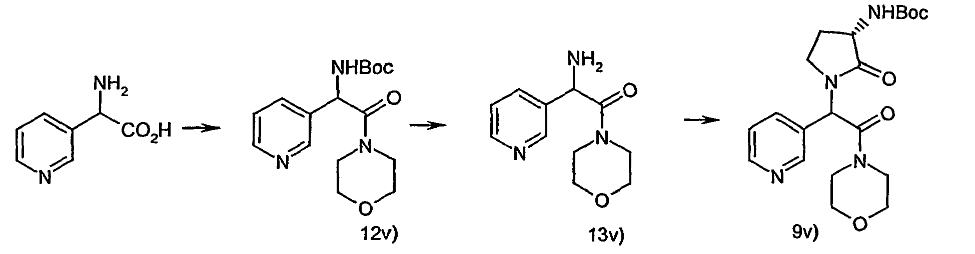

### Intermediate 12v) 1,1-Dimethylethyl [2-(4-morpholinyl)-2-oxo-1-(3-pyridinyl)ethyl]carbamate

3-Pyridylglycine (0.59g) was taken up in dry DCM (10ml) at room temperature, and treated with triethylamine (0.65ml) and a solution of di-t-butyl dicarbonate (1.01g) in dry DCM (10ml). The mixture was then stirred for 18 hours at room temperature. A further portion of triethylamine (0.65ml) was added followed by dry DMF (20ml). After 2.5hours, morpholine (1.0ml) was added and the reaction mixture was cooled to 0 °C. TBTU (2.5g) was added in portions over 5 minutes and the mixture was stirred for 30 minutes, then allowed to warm to room temperature and stirred for 18 hours. The mixture was then evaporated to dryness under reduced pressure, and the residue partitioned between ethyl acetate and sat aq. NaHCO₃. The organic layer was dried (Na₂SO₄), filtered and solvent evaporated under reduced pressure. Purification via SPE chromatography (ethyl acetate) gave the title compound.
RT 2.09min, MH⁺ 322

### Intermediate 13v) 2-(4-Morpholinyl)-2-oxo-1-(3-pyridinyl)ethanamine

Prepared according to the procedure for 5e) from Intermediate 12v).
RT 0.38min, MH⁺ 222

### Intermediate 9v) 1,1-Dimethylethyl {(3S)-1-[2-(4-morpholinyl)-2-oxo-1-(3-pyridinyl)ethyl]-2-oxo-3-pyrrolidinyl}carbamate

Prepared according to the procedure for Intermediate 9t) from Intermediate 13v) and 2-tert-butoxycarbonylamino-4-oxo-butyric acid benzyl ester.
RT 2.14min, MH⁺ 405

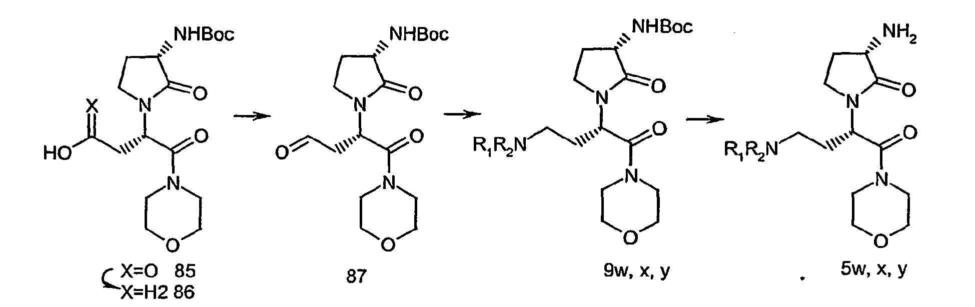

### Intermediate 85 (3S)-3-[(3S)-3-({[(1,1-Dimethylethyl)oxy]carbonyl}amino)-2-oxo-1-pyrrolidinyl]-4-(4-morpholinyl)-4-oxobutanoic acid

Intermediate 9h) (5.0g) was taken up in glacial acetic acid (75ml) and hydrogenated at room temperature and atmospheric pressure over 10% Palladium/Carbon (500mg of 50% w/w H₂O) for 2 hours. The catalyst was removed by filtration through celite, and solvent evaporated under vacuum. The residue was taken up in toluene (50ml) and evaporated under vacuum to give the title compound.
RT 2.18min, MH⁺ 386

### Intermediate 86 1,1-Dimethylethyl {(3S)-1-[(1S)-3-hydroxy-1-(4-morpholinylcarbonyl)propyl]-2-oxo-3-pyrrolidinyl}carbamate

Intermediate 85 (193mg) was stirred at -15 °C in dry THF (0.5ml). N-methylmorpholine (55ul) was added, followed by dropwise addition of isobutyl chloroformate (66ul). Stirring was continued at -15°C for 10 minutes. Dry THF (3ml) was added, followed by a solution of sodium borohydride (48mg) in water (0.5ml). After stirring for a further 20 minutes, the mixture was allowed to warm to room temperature, and was stirred for a further 45 minutes. The mixture was then partitioned between ethyl acetate and water. The aqueous layer was saturated with sodium chloride and further extracted with ethyl acetate. The combined organic portions were washed with 10% aqueous citric acid solution, followed by sat. aq. NaHCO₃ solution. Evaporation of the organic portion under vacuum gave the crude product. Purification via SPE chromatography (DCM:MeOH 20:1) gave the title compound.
RT 2.11 min, MH⁺ 372

### Intermediate 87 1,1-Dimethylethyl {(3S)-1-[(1S)-1-(4-morpholinylcarbonyl)-3-oxopropyl]-2-oxo-3-pyrrolidinyl}carbamate

Intermediate 86 (928mg) was dissolved in a mixture of dry DCM (20ml) and dry Acetonitrile (30ml). 4-Methylmorpholine-oxide (439mg) was added, followed by dried powdered molecular sieves (3A, 1.4g) and TPAP (44mg). The mixture was stirred at room temperature for 4 hours, then solvent evaporated under vacuum. The residue was purified via SPE chromatography (DCM:MeOH 20:1-10:1) to give the title compound. This was used without further characterisation to prepare intermediates 9w), 9x), and 9y).

### Intermediate 9w) 1,1-Dimethylethyl {(3S)-1-[(1S)-3-(dimethylamino)-1-(4-morpholinylcarbonyl)propyl]-2-oxo-3-pyrrolidinyl}carbamate

Intermediate 87 (265mg) was dissolved in dry DCM (8ml). Dimethylamine (38mg, 0.47ml of a 2M THF solution) was added followed by dried molecular sieves (3A, 0.4g). After stirring for 1 hour, glacial acetic acid (0.2ml) and sodium triacetoxyborohydride (200mg) were added. After stirring for a further 3 hours, the mixture was filtered and solvent reduced under vacuum. The residue was taken up in methanol and purified via SCX ion exchange cartridge eluting with 2M ammonia in methanol to give the title compound.
RT 1.88min, MH⁺ 399

In a similar fashion were prepared:

### Intermediate 9x) 1,1-Dimethylethyl {(3S)-1-[(1S)-1-(4-morpholinylcarbonyl)-3-(1-piperidinyl)propyl]-2-oxo-3-pyrrolidinyl}carbamate

From intermediate 87 and piperidine.
RT 1.94min, MH⁺ 439

### Intermediate 9y) 1,1-Dimethylethyl {(3S)-1-[(1S)-3-(4-morpholinyl)-1-(4-morpholinylcarbonyl)propyl]-2-oxo-3-pyrrolidinyl}carbamate

From Intermediate 87 and morpholine.
RT 1.88min, MH⁺ 441

Intermediate 5w) (3*S*)-3-Amino-1-[(1*S*)-3-(dimethylamino)-1-(4-morpholinvlcarbonyl)propyl]-2-pyrrolidinone dihydrochloride

Intermediate 9w) (176mg) was dissolved in dry dioxan (2ml). 4M HCl in dioxan (4ml) was added and the mixture was stirred for 4 hours at room temperature. Solvent was removed under vacuum, the residue was re-dissolved in fresh dioxan (4ml) and solvent re-evaporated to give the title compound.
RT 0.38min, MH⁺ 341

In a similar fashion were prepared:

### Intermediate 5x) (3S)-3-Amino-1-[(1S)-1-(4-morpholinylcarbonyl)-3-(1-piperidinyl)propyl]-2-pyrrolidinone dihydrochloride

RT 0.38min, MH⁺ 339

### Intermediate 5y) (3S)-3-Amino-1l(1S)-3-(4-morpholinyl)-1-(4-morpholinylcarbonyl)propyl]-2-pyrrolidinone dihydrochloride

RT 0.38min, MH⁺ 299

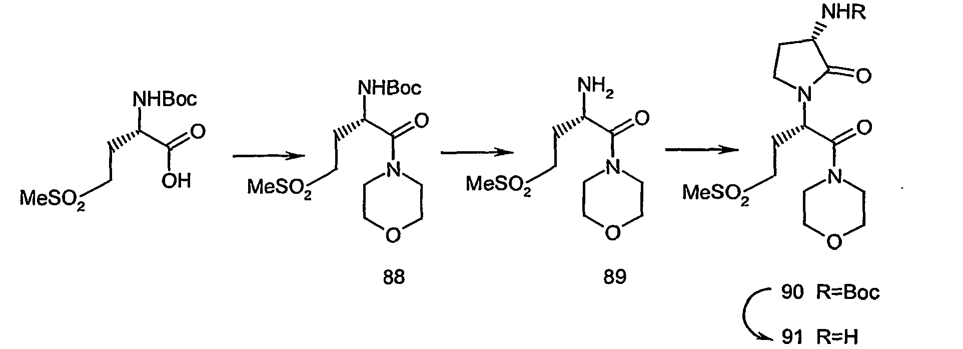

### Intermediate 88 1,1-Dimethylethyl [(1S)-3-(methylsulfonyl)-1-(4-morpholinylcarbonyl)propyl]carbamate

Prepared from (2*S*)-2-({[(1,1-dimethylethyl)oxy]carbonyl}amino)-4-(methylsulfonyl)butanoic acid according to the procedure for Intermediate 11
RT 2.14min, MH⁺ 351

### Intermediate 89 (2S)-4-(Methylsulfonyl)-1-(4-morpholinyl)-1-oxo-2-butanamine

Prepared from Intermediate 88 according to the procedure for Intermediate 5f).
RT 0.39min, MH⁺ 251

### Intermediate 90 1,1-Dimethylethyl {(3S)-1-[(1S)-3-(methylsulfonyl)-1-(4-morpholinylcarbonyl)propyl]-2-oxo-3-pyrrolidinyl}carbamate

Prepared from Intermediate 89 according to the procedure for Intermediate 9f).
RT 2.24min, MH⁺ 433

### Intermediate 91 (3S)-3-Amino-1-[(1S)-3-(methylsulfonyl)-1-(4-morpholinylcarbonyl)propyl]-2-pyrrolidinone hydrochloride

Prepared from Intermediate 90 according to the procedure for Intermediate 5e)
RT 0.48min, MH⁺ 333

| Example | | Name of compound |
|---|---|---|
| 1 | 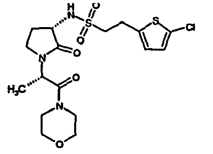 | 2-(5-Chlorothien-2-yl)-N-{(3*S*)-1-[(1 S)-1-methyl-2-morpholin-4-yl-2-oxoethyl]-2-oxopyrrolidin-3-yl}ethanesulfonamide |
| 2 | 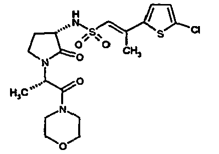 | (1 E)-2-(5-chlorothien-2-yl)-N-{(3S)-1-[(1S)-1-methyl-2-morpholin-4-yl-2-oxoethyl]-2-oxopyrrolidin-3-yl}prop-1-ene-1-sulfonamide |
| 3 | 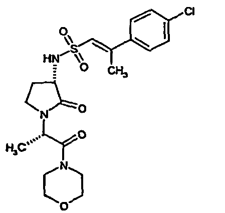 | (1 E)-2-(4-chlorophenyl)-N-{(3S)-1-[(1 S)-1-methyl-2-morpholin-4-yl-2-oxoethyl]-2-oxopyrrolidin-3-yl}prop-1-ene-1-sulfonamide |
| 4 | 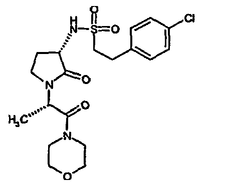 | 2-(4-chlorophenyl)-N-((3S)-1-[(1 S)-1-methyl-2-morpholin-4-yl-2-oxoethyl]-2-oxopyrrolidin-3-yl}ethanesulfonamide |
| 5 | 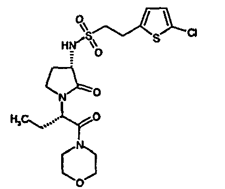 | 2-(5-chlorothien-2-yl)-N-{(3S)-1-[(1S)-1-(morpholin-4-ylcarbonyl)propyl]-2-oxopyrrolidin-3-yl}ethanesulfonamide |
| 6 | 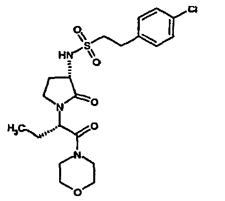 | 2-(4-chlorophenyl)-N-{(3S)-1-[(1S)-1-(morpholin-4-ylcarbonyl)propyl]-2-oxopyrrolidin-3-yl}ethanesulfonamide |
| 7 | 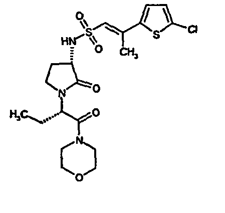 | (1E)-2-(5-chlorothien-2-yl)-N-{(3S)-1-[(1 S)-1-(morpholin-4-ylcarbonyl)propyl]-2-oxopyrrolidin-3-yl}prop-1-ene-1-sulfonamide |
| 8 | 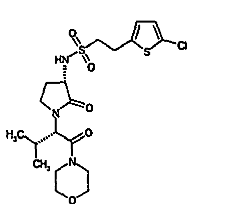 | 2-(5-chlorothien-2-yl)-N-{(3S)-1-[(1 S)-2-methyl-1-(morpholin-4-ylcarbonyl)propyl]-2-oxopyrrolidin-3-yl}ethanesulfonamide |
| 9 | 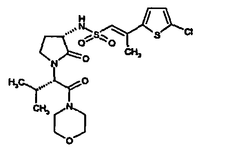 | (1 E)-2-(5-chlorothien-2-yl)-N-{(3S)-1-[(1S)-2-methyl-1-(morpholin-4-ylcarbonyl)propyl]-2-oxopyrrolidin-3-yl}prop-1-ene-1-sulfonamide |
| 10 | 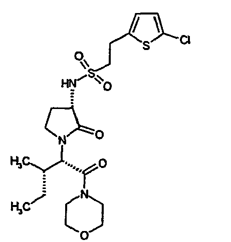 | 2-(5-chlorothien-2-yl)-N-{(3S)-1-[(1 S,2S)-2-methyl-1-(morpholin-4-ylcarbonyl)butyl]-2-oxopyrrolidin-3-yl}ethanesulfonamide |
| 11 | 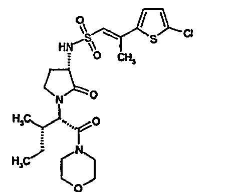 | (1 E)-2-(5-chlorothien-2-yl)-N-{(3S)-1-[(1S,2S)-2-methyl-1-(morpholin-4-ylcarbonyl)butyl]-2-oxopyrrolidin-3-yl}prop-1-ene-1-sulfonamide |
| 12 | 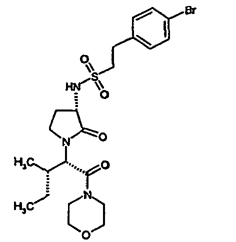 | 2-(4-bromophenyl)-N-{(3S)-1-[(1 S,2S)-2-methyl-1-(morpholin-4-ylcarbonyl)butyl]-2-oxopyrrolidin-3-yl}ethanesulfonamide |
| 13 | 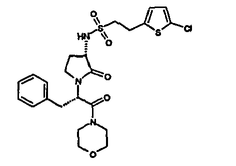 | N-{(3S)-1-[(1S)-1-benzyl-2-morpholin-4-yl-2-oxoethyl]-2-oxopyrrolidin-3-yl}-2-(5-chlorothien-2-yl)ethanesulfonamide |
| 14 | 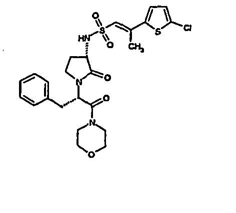 | (1 E)-N-{(3S)-1-[(1 S)-1-benzyl-2-morpholin-4-yl-2-oxoethyl]-2-oxopyrrolidin-3-yl}-2-(5-chlorothien-2-yl)prop-1-ene-1-sulfonamide |
| 15 | 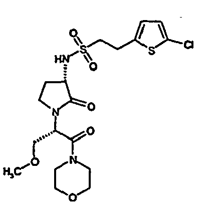 | 2-(5-chlorothien-2-yl)-N-{(3S)-1-[(1S)-1-(methoxymethyl)-2-morpholin-4-yl-2-oxoethyl]-2-oxopyrrolidin-3-yl}ethanesulfonamide |
| 16 | 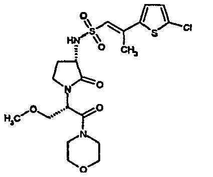 | (1E)-2-(5-chlorothien-2-yl)-N-{(3S)-1-(methoxymethyl)-2-morpholin-4-yl-2-oxoethyl]-2-oxopyrrolidin-3-yl}prop-1-ene-1-sulfonamide |
| 17 | 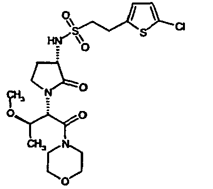 | 2-(5-chlorothien-2-yl)-N-{(3S)-1-[(1S,2R)-2-methoxy-1-(morpholin-4-ylcarbonyl)propyl]-2-oxopyrrolidin-3-yl}ethanesulfonamide |
| 18 | 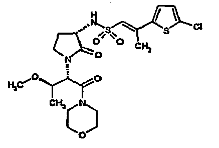 | (1E)-2-(5-chlorothien-2-yl)-N-{(3S)-1-[(1S,2R)-2-methoxy-1-(morpholin-4-ylcarbonyl)propyl]-2-oxopyrrolidin-3-yl}prop-1-ene-1-sulfonamide |
| 19 | 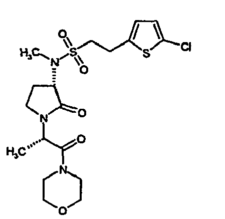 | 2-(5-Chlorothien-2-yl)-*N*-methyl-*N*-[{(3*S*)-1-[(1*S*)-1-methyl-2-morpholin-4-yl-2-oxoethyl]-2-oxopyrrolidin-3-yl}ethanesulfonamide |
| 20 | 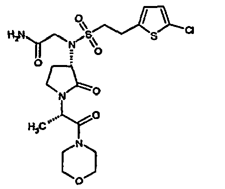 | *N*²-{[2-(5-Chlorothien-2-yl)ethyl]sulfonyl}-*N*-²-{(3*S*)-1-[(1*S*)-1-methyl-2-morpholin-4-yl-2-oxoethyl]-2-oxopyrrolidin-3-yl}glycinamide |
| 21 | 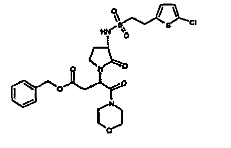 | Benzyl (3*S*)-3-[(3*S*)-3-({[2-(5-chlorothien-2-yl)ethyl]sulfonyl}amino)-2-oxopyrrolidin-1-yl]-4-morpholin-4-yl-4-oxobutanoate |
| 22 | 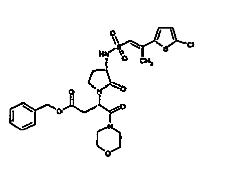 | Benzyl (3*S*)-3-[(3*S*)-3-({[(1*E*)-2-(5-Chlorothien-2-yl)prop-1-enyl]sulfonyl}amino)-2-oxopyrrolidin-1-yl]-4-morpholin-4-yl-4-oxobutanoate |
| 23 | 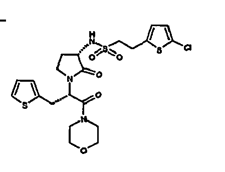 | 2-(5-chlorothien-2-yl)-*N*-{(3*S*)-1-[(1*S*)-2-morpholin-4-yl-2-oxo-1-(thien-2-ylmethyl)ethyl]-2-oxopyrrolidin-3-yl}ethanesulfonamide |
| 24 | 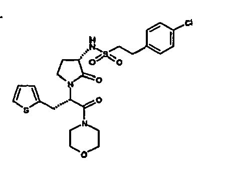 | 2-(4-chlorophenyl)-*N*-{(3*S*)-1-[(1*S*)-2-morpholin-4-yl-2-oxo-1-(thien-2-ylmethyl)ethyl]-2-oxopyrrolidin-3-yl}ethanesulfonamide |
| 25 | 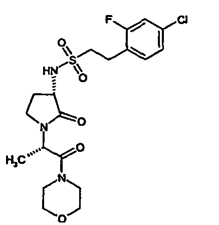 | 2-(4-chloro-2-fluorophenyl)-N-{(3S)-1-[(1S)-1-methyl-2-morpholin-4-yl-2-oxoethyl]-2-oxopyrrolidin-3-yl}ethanesulfonamide |
| 26 | 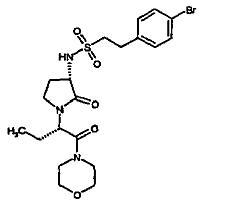 | 2-(4-bromophenyl)-N-{(3S)-1-[(1S)-1-(morpholin-4-ylcarbonyl)propyl]-2-oxopyrrolidin-3-yl}ethanesulfonamide |
| 27 | 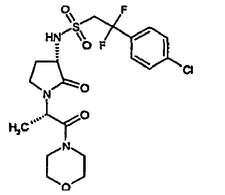 | 2-(4-chlorophenyl)-2,2-difluoro-*N*-{(3*S*)-1-[(1*S*)-1-methyl-2-morpholin-4-yl-2-oxoethyl]-2-oxopyrrolidin-3-yl}ethanesulfonamide |
| 28 | 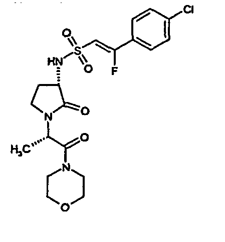 | (Z)-2-(4-chlorophenyl)-2-fluoro-*N*-{(3*S*)-1-[(1*S*)-1-methyl-2-morpholin-4-yl-2-oxoethyl]-2-oxopyrrolidin-3-yl}ethenesulfonamide |
| 29 | 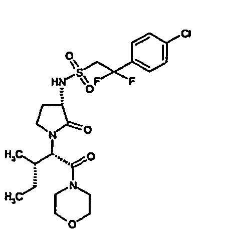 | 2-(4-chlorophenyl)-2,2-difluoro-*N*-{(3*S*)-1-[(1*S*,2*S*)-2-methyl-1-(morpholin-4-ylcarbonyl)butyl]-2-oxopyrrolidin-3-yl}ethanesulfonamide |
| 30 | 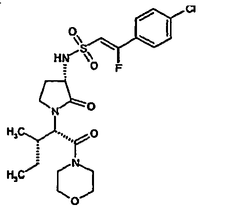 | (*Z*)-2-(4-chlorophenyl)-2-fluoro-*N*-{(3*S*)-1-[(1*S*,2*S*)-2-methyl-1-(morpholin-4-ylcarbonyl)butyl]-2-oxopyrrolidin-3-yl}ethenesulfonamide |
| 31 | 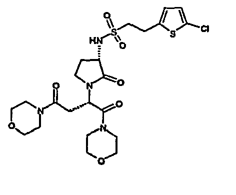 | (1*E*)-2-(5-Chlorothien-2-yl)-*N*-{(3*S*)-1-[(1*S*)-3-morpholin-4-yl-1-(morpholin-4-ylcarbonyl)-3-oxopropyl]-2-oxopyrrolidin-3-yl}prop-1-ene-1-sulfonamide |
| 32 | 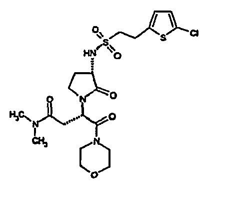 | (3*S*)-3-[(3*S*)-3-({[(1*E*)-2-(5-Chlorothien-2-yl)prop-1-enyl]sulfonyl}amino)-2-oxopyrrolidin-1-yl]-*N*,*N*-dimethyl-4-morpholin-4-yl-4-oxobutanamide |
| 33 | 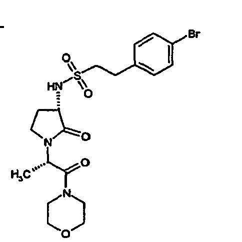 | 2-(4-bromophenyl)-N-{(3S)-1-[(1S)-1-methyl-2-morpholin-4-yl-2-oxoethyl]-2-oxopyrrolidin-3-yl}ethanesulfonamide |
| 34 | 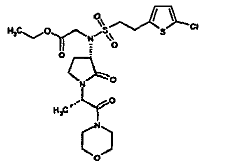 | Ethyl *N*-{[2-(5-chlorothien-2-yl)ethyl]sulfonyl}-*N*-{(3*S*)-1-[(1*S*)-1-methyl-2-morpholin-4-yl-2-oxoethyl]-2-oxopyrrolidin-3-yl}glycinate |
| 35 | 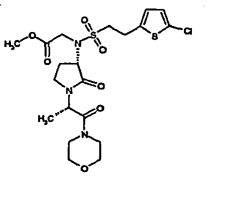 | Methyl *N*-{[2-(5-chlorothien-2-yl)ethyl]sulfonyl}-*N*-{(3*S*)-1-[(1*S*)-1-methyl-2-morpholin-4-yl-2-oxoethyl]-2-oxopyrrolidin-3-yl}glycinate |
| 36 | 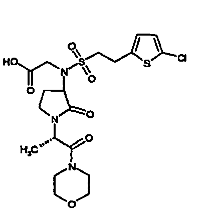 | N-{[2-(5-chlorothien-2-yl)ethyl]sulfonyl}-*N*-{(3*S*)-1-[(1*S*)-1-methyl-2-morpholin-4-yl-2-oxoethyl]-2-oxopyrrolidin-3-yl}glycine |
| 37 | 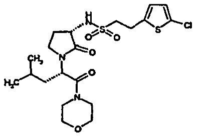 | 2-(5-chlorothien-2-yl)-*N*-{(3*S*)-1-[(1*S*)-3-methyl-1-(morpholin-4-ylcarbonyl)butyl]-2-oxopyrrolidin-3-yl}ethanesulfonamide |
| 38 | 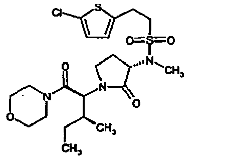 | 2-(5-chlorothien-2-yl)-N-methyl-*N* -{(3S)-1-[(1S,2S)-2-methyl-1-(morpholin-4-ylcarbonyl)butyl]-2-oxopyrrolidin-3-yl}ethanesulfonamide |
| 39 | 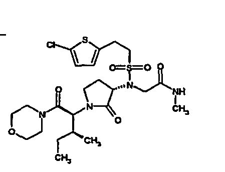 | *N*²-{[2-(5-chlorothien-2-yl)ethyl]sulfonyl}-*N*¹-methyl-*N*²-{(3*S*)-1-[(1*S*,2*S*)-2-methyl-1-(morpholin-4-ylcarbonyl)butyl]-2-oxopyrrolidin-3-yl}glycinamide |
| 40 | 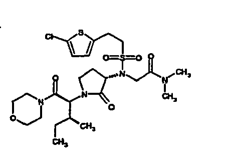 | *N*²-{[2-(5-chlorothien-2-yl)ethyl]sulfonyl}-*N*¹,*N*¹-dimethyl-*N*²-{(3*S*)-1-[(1*S*,2*S*)-2-methyl-1-(morpholin-4-ylcarbonyl)butyl]-2-oxopyrrolidin-3-yl}glycinamide |
| 41 | 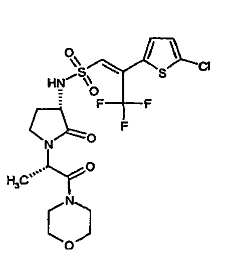 | (1*E*)-2-(5-chlorothien-2-yl)-3,3,3-trifluoro-*N*-{(3*S*)-1[(1*S*)-1-methyl-2-morpholin-4-yl-2-oxoethyl]-2-oxopyrrolidin-3-yl}prop-1-ene-1-sulfonamide |
| 42 | 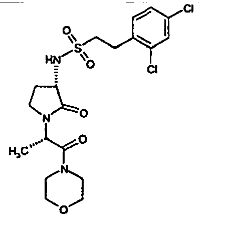 | 2-(2,4-dichlorophenyl)-N-{(3S)-1-[(1S)-1-methyl-2-morpholin-4-yl-2-oxoethyl]-2-oxopyrrolidin-3-yl}ethanesulfonamide |
| 43 | 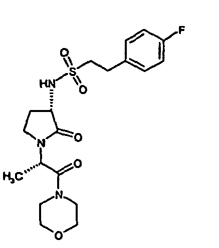 | 2-(4-fluorophenyl)-N-{(3S)-1-[(1S)-1-methyl-2-morpholin-4-yl-2-oxoethyl]-2-oxopyrrolidin-3-yl}ethanesulfonamide |
| 44 | 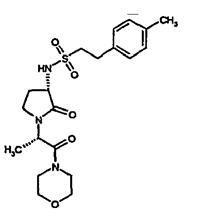 | 2-(4-methylphenyl)-N-{(3S)-1-[(1S)-1-methyl-2-morpholin-4-yl-2-oxoethyl]-2-oxopyrrolidin-3-yl}ethanesulfonamide |
| 45 | 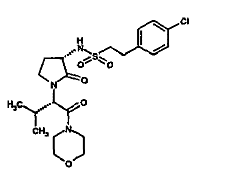 | 2-(4-chlorophenyl)-N-{(3S)-1-[(1S)-2-methyl-1-(morpholin-4-ylcarbonyl)propyl]-2-oxopyrrolidin-3-yl}ethanesulfonamide |
| 46 | 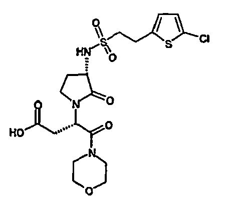 | (3*S*)-3-[(3*S*)-3-({[2-(5-Chlorothien-2-ethane]sulfonyl}amino)-2-oxopyrrolidin-1-yl]-4-morpholin-4-yl-4-oxobutanoic acid |
| 47 | 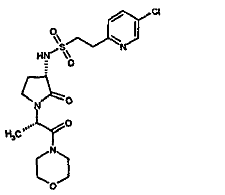 | 2-(5-chloro-2-pyridinyl)-*N*-{(3*S*)-1-[(1*S*)-1-methyl-2-(4-morpholinyl)-2-oxoethyl]-2-oxo-3-pyrrolidinyl}ethanesulfonamide |
| 48 | 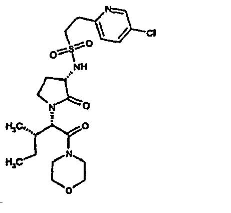 | 2-(5-chloro-2-pyridinyl)-*N*-{(3*S*)-1-[(1*S*,2*S*)-2-methyl-1-(4-morpholinylcarbonyl)butyl]-2-oxo-3-pyrrolidinyl}ethanesulfonamide |
| 49 | 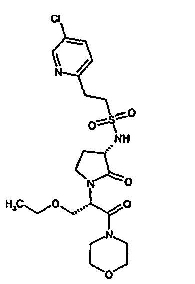 | 2-(5-chloro-2-pyridinyl)-*N*-{(3*S*)-1-[(1*S*)-1-[(ethyloxy)methyl]-2-(4-morpholinyl)-2-oxoethyl]-2-oxo-3-pyrrolidinyl}ethanesulfonamide |
| 50 | 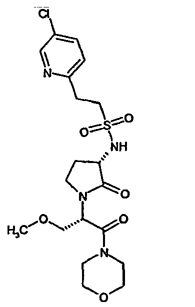 | 2-(5-chloro-2-pyridinyl)-*N*-{(3*S*)-1-[(1*S*)-1-[(methyloxy)methyl]-2-(4-morpholinyl)-2-oxoethyl]-2-oxo-3-pyrrolidinyl}ethanesulfonamide |
| 51 | 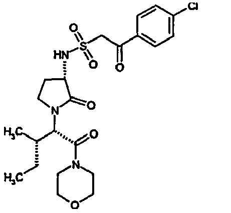 | 2-(4-chlorophenyl)-*N*-{(3*S*)-1-[(1*S*,2*S*)-2-methyl-1-(4-morpholinylcarbonyl)butyl]-2-oxo-3-pyrrolidinyl}-2-oxoethanesulfonamide |
| 52 | 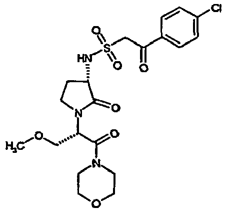 | 2-(4-chlorophenyl)-*N*-{(3*S*)-1-[(1*S*)-1-(methyloxy)-2-(4-morpholinyl)-2-oxoethyl]-2-oxo-3-pyrrolidinyl}-2-oxoethanesulfonamide |
| 53 | 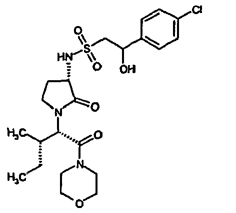 | 2-(4-chlorophenyl)-2-hydroxy-*N*-{(3*S*)-1-[(1*S*,2*S*)-2-methyl-1-(4-morpholinylcarbonyl)butyl]-2-oxo-3-pyrrolidinyl}ethanesulfonamide |
| 54 | 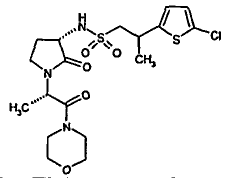 | 2-(5-chloro-2-thienyl)-*N*-{(3*S*)-1-[(1*S*)-1-methyl-2-(4-morpholinyl)-2-oxoethyl]-2-oxo-3-pyrrolidinyl}-1-propanesulfonamide |
| 55 | 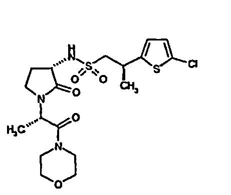 | (2*R*)-2-(5-chloro-2-thienyl)-*N*-{(3*S*)-1-[(1*S*)-1-methyl-2-(4-morpholinyl)-2-oxoethyl]-2-oxo-3-pyrrolidinyl}-1-propanesulfonamide. |
| 56 | 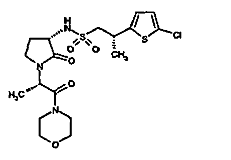 | (2*S*)-2-(5-chloro-2-thienyl)-*N*-{(3*S*)-1-[(1*S*)-1-methyl-2-(4-morpholinyl)-2-oxoethyl]-2-oxo-3-pyrrolidinyl}-1-propanesulfonamide |
| 57 | 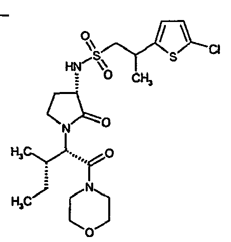 | 2-(5-chloro-2-thienyl)-*N*-{(3*S*)-1-[(1*S*,2*S*)-2-methyl-1-(4-morpholinylcarbonyl)butyl]-2-oxo-3-pyrrolidinyl}-1-propanesulfonamide |
| 58 | 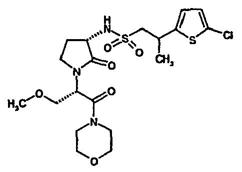 | 2-(5-chloro-2-thienyl)-*N*-{(3*S*)-1-[(1*S*)-1-[(methyloxy)methyl]-2-(4-morpholinyl)-2-oxoethyl]-2-oxo-3-pyrrolidinyl}-1-propanesulfonamide |
| 59 | 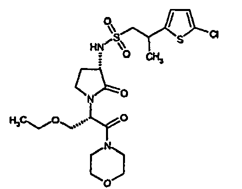 | 2-(5-chloro-2-thienyl)-*N*-{(3*S*)-1-[(1*S*)-1-[(ethyloxy)methyl]-2-(4-morpholinyl)-2-oxoethyl]-2-oxo-3-pyrrolidinyl}-1-propanesulfonamide |
| 60 | 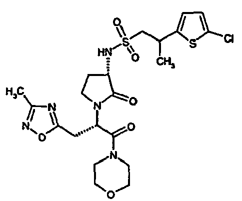 | 2-(5-chloro-2-thienyl)-*N*-{(3*S*)-1-[(1*S*)-1-[(3-methyl-1,2,4-oxadiazol-5-yl)methyl]-2-(4-morpholinyl)-2-oxoethyl]-2-oxo-3-pyrrolidinyl}-1-propanesulfonamide |
| 61 | 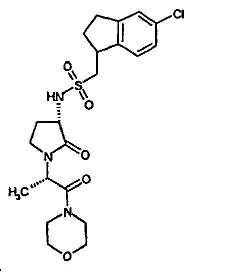 | 1-(5-chloro-2,3-dihydro-1*H*-inden-1-yl)-*N*-{(3*S*)-1-[(1*S*)-1-methyl-2-(4-morpholinyl)-2-oxoethyl]-2-oxo-3-pyrrolidinyl}methanesulfonamide |
| 62 | 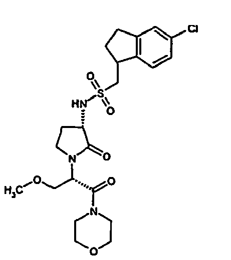 | 1-(5-chloro-2,3-dihydro-1*H*-inden-1-yl)-*N*-{(3*S*)-1-[(1*S*)-1-[(methyloxy)methyl]-2-(4-morpholinyl)-2-oxoethyl]-2-oxo-3-pyrrolidinyl}methanesulfonamide |
| 63 | 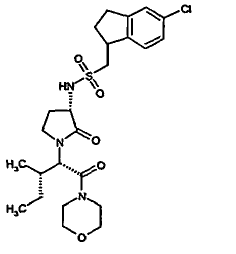 | 1-(5-chloro-2,3-dihydro-1*H*-inden-1-yl)-*N*-{(3*S*)-1-[(1*S*,2*S*)-2-methyl-1-(4-morpholinylcarbonyl)butyl]-2-oxo-3-pyrrolidinyl}methanesulfonamide |
| 64 | 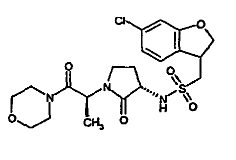 | 1-(6-chloro-2,3-dihydro-1-benzofuran-3-yl)-*N*-{(3*S*)-1-[(1*S*)-1-methyl-2-(4-morpholinyl)-2-oxoethyl]-2-oxo-3-pyrrolidinyl}methanesulfonamide |
| 65 | 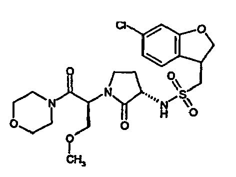 | 1-(6-chloro-2,3-dihydro-1-benzofuran-3-yl)-*N*-{(3S)-1-[(1*S*)-1-[(methyloxy)methyl]-2-(4-morpholinyl)-2-oxoethyl]-2-oxo-3-pyrrolidinyl}methanesulfonamide |
| 66 | 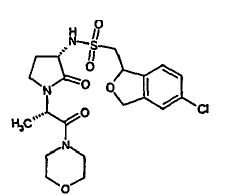 | 1-(5-chloro-1,3-dihydro-2-benzofuran-1-yl)-*N*-{(3*S*)-1-[(1*S*)-1-methyl-2-(4-morpholinyl)-2-oxoethyl]-2-oxo-3-pyrrolidinyl}methanesulfonamide |
| 67 | 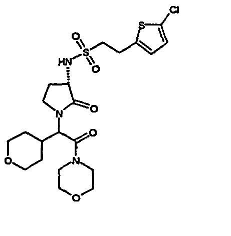 | 2-(5-chloro-2-thienyl)-*N*-{(3*S*)-1-[2-(4-morpholinyl)-2-oxo-1-(tetrahydro-2*H*-pyran-4-yl)ethyl]-2-oxo-3-pyrrolidinyl}ethanesulfonamide |
| 68 | 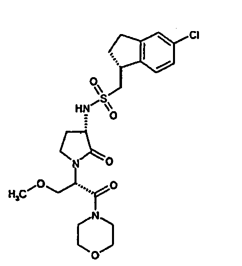 | 1-[(1*R*)-5-chloro-2,3-dihydro-1*H*-inden-1-yl]-*N*-{(3*S*)-1-[(1*S*)-1-[(methyloxy)methyl]-2-(4-morpholinyl)-2-oxoethyl]-2-oxo-3-pyrrolidinyl}methanesulfonamide |
| 69 | 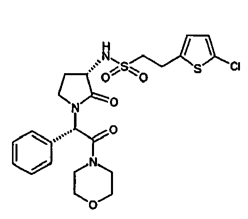 | 2-(5-chloro-2-thienyl)-*N*-{(3*S*)-1-[(1*S*)-2-(4-morpholinyl)-2-oxo-1-phenylethyl]-2-oxo-3-pyrrolidinyl}ethanesulfonamide |
| 70 | 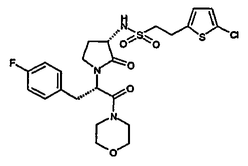 | 2-(5-chloro-2-thienyl)-*N*-{(3*S*)-1-[(1*S*)-1-[(4-fluorophenyl)methyl]-2-(4-morpholinyl)-2-oxoethyl]-2-oxo-3-pyrrolidinyl}ethanesulfonamide |
| 71 | 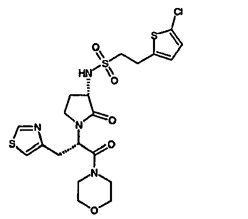 | 2-(5-chloro-2-thienyl)-*N*-{(3*S*)-1-[(1*S*)-2-(4-morpholinyl)-2-oxo-1-(1,3-thiazol-4-ylmethyl)ethyl]-2-oxo-3-pyrrolidinyl}ethanesulfonamide |
| 72 | 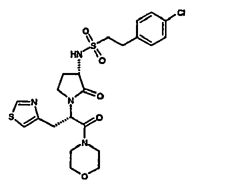 | 2-(4-chlorophenyl)-*N*-{(3*S*)-1-[(1*S*)-2-(4-morpholinyl)-2-oxo-1-(1,3-thiazol-4-ylmethyl)ethyl]-2-oxo-3-pyrrolidinyl}ethanesulfonamide |
| 73 | 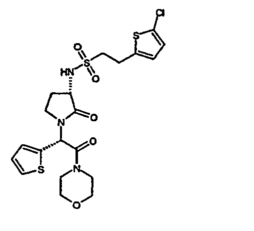 | 2-(5-chloro-2-thienyl)-*N*-{(3*S*)-1-[(1*R*)-2-(4-morpholinyl)-2-oxo-1-(2-thienyl)ethyl]-2-oxo-3-pyrrolidinyl}ethanesulfonamide |
| 74 | 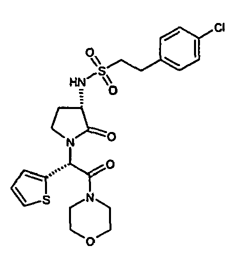 | 2-(4-chlorophenyl)-*N*-{(3*S*)-1-[(1*R*)-2-(4-morpholinyl)-2-oxo-1-(2-thienyl)ethyl]-2-oxo-3-pyrrolidinyl}ethanesulfonamide |
| 75 | 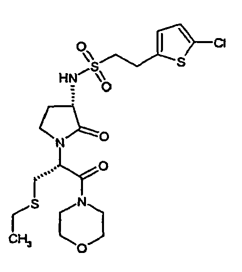 | 2-(5-chloro-2-thienyl)-*N*-{(3*S*)-1-[(1*R*)-1-[(ethylthio)methyl]-2-(4-morpholinyl)-2-oxoethyl]-2-oxo-3-pyrrolidinyl}ethanesulfonamide |
| 76 | 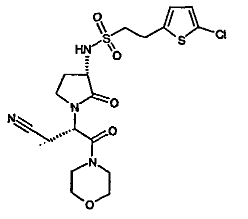 | 2-(5-chloro-2-thienyl)-*N*-{(3*S*)-1-[(1*S*)-1-(cyanomethyl)-2-(4-morpholinyl)-2-oxoethyl]-2-oxo-3-pyrrolidinyl}ethanesulfonamide |
| 77 | 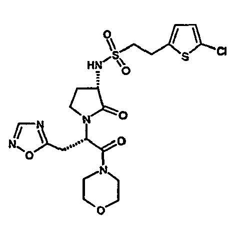 | 2-(5-chloro-2-thienyl)-*N*-{(3*S*)-1-[(1*S*)-2-(4-morpholinyl)-1-(1,2,4-oxadiazol-5-ylmethyl)-2-oxoethyl]-2-oxo-3-pyrrolidinyl}ethanesulfonamide |
| 78 | 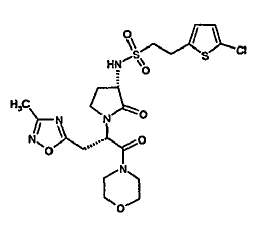 | 2-(5-chloro-2-thienyl)-*N*-{(3*S*)-1-[(1*S*)-1-[(3-methyl-1,2,4-oxadiazol-5-yl)methyl]-2-(4-morpholinyl)-2-oxoethyl]-2-oxo-3-pyrrolidinyl}ethanesulfonamide |
| 79 | 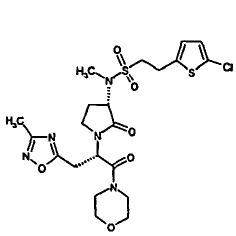 | 2-(5-chloro-2-thienyl)-*N*-methyl-*N*-{(3*S*)-1-[(1*S*)-1-[(3-methyl-1,2,4-oxadiazol-5-yl)methyl]-2-(4-morpholinyl)-2-oxoethyl]-2-oxo-3-pyrrolidinyl}ethanesulfonamide |
| 80 | 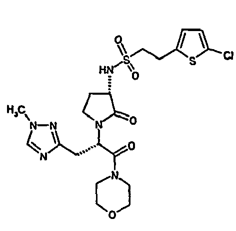 | 2-(5-chloro-2-thienyl)-*N*-{(3*S*)-1-[(1*S*)-1-[(1-methyl-1*H*-1,2,4-triazol-3-yl)methyl]-2-(4-morpholinyl)-2-oxoethyl]-2-oxo-3-pyrrolidinyl}ethanesulfonamide |
| 81 | 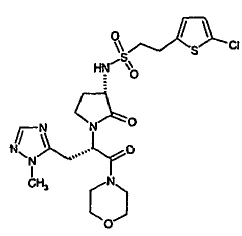 | 2-(5-chloro-2-thienyl)-*N*-{(3*S*)-1-[(1*S*)-1-[(1-methyl-1*H*-1,2,4-triazol-5-yl)methyl]-2-(4-morpholinyl)-2-oxoethyl]-2-oxo-3-pyrrolidinyl}ethanesulfonamide |
| 82 | 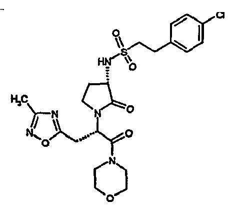 | 2-(4-chlorophenyl)-*N*-{(3*S*)-1-[(1*S*)-1-[(3-methyl-1,2,4-oxadiazol-5-yl)methyl]-2-(4-morpholinyl)-2-oxoethyl]-2-oxo-3-pyrrolidinyl}ethanesulfonamide |
| 83 | 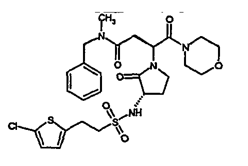 | (3*S*)-3-[(3*S*)-3-({[2-(5-chloro-2-thienyl)ethyl]sulfonyl}amino)-2-oxo-1-pyrrolidinyl]-*N*-methyl-4-(4-morpholinyl)-4-oxo-*N*-(phenylmethyl)butanamide |
| 84 | 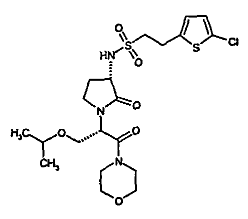 | 2-(5-chloro-2-thienyl)-*N*-{(3*S*)-1-[(1*S*)-1-{[(1-methylethyl)oxy]methyl}-2-(4-morpholinyl)-2-oxoethyl]-2-oxo-3-pyrrolidinyl}ethanesulfonamide |
| 85 | 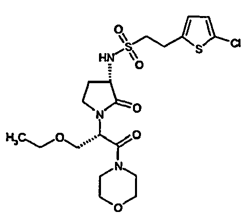 | 2-(5-chloro-2-thienyl)-*N*-{(3*S*)-1-[(1*S*)-1-[(ethyloxy)methyl]-2-(4-morpholinyl)-2-oxoethyl]-2-oxo-3-pyrrolidinyl}ethanesulfonamide |
| 86 | 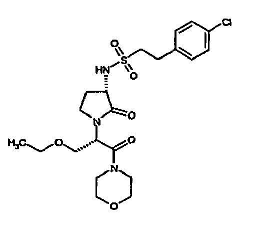 | 2-(4-chlorophenyl)-*N*-{(3*S*)-1-[(1*S*)-1-[(ethyloxy)methyl]-2-(4-morpholinyl)-2-oxoethyl]-2-oxo-3-pyrrolidinyl}ethanesulfonamide |
| 87 | 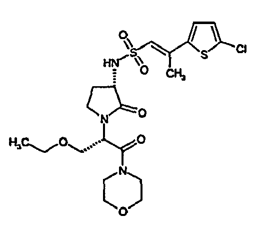 | (1*E*)-2-(5-chloro-2-thienyl)-*N*-{(3*S*)-1-[(1*S*)-1-[(ethyloxy)methyl]-2-(4-morpholinyl)-2-oxoethyl]-2-oxo-3-pyrrolidinyl}-1-propene-1-sulfonamide |
| 88 | 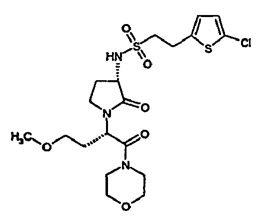 | 2-(5-chloro-2-thienyl)-*N*-{(3*S*)-1-[(1*S*)-3-(methyloxy)-1-(4-morpholinylcarbonyl)propyl]-2-oxo-3-pyrrolidinyl}ethanesulfonamide |
| 89 | 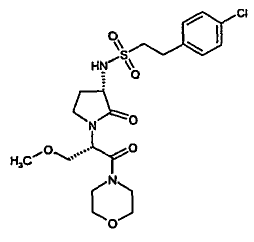 | 2-(4-chlorophenyl)-*N*-{(3*S*)-1-[(1*S*)-1-[(methyloxy)methyl]-2-(4-morpholinyl)-2-oxoethyl]-2-oxo-3-pyrrolidinyl}ethanesulfonamide |
| 90 | 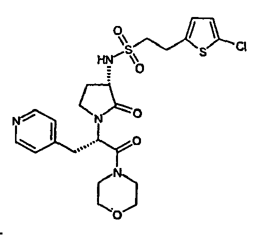 | 2-(5-chloro-2-thienyl)-*N*-{(3*S*)-1-[(1*S*)-2-(4-morpholinyl)-2-oxo-1-(4-pyridinylmethyl)ethyl]-2-oxo-3-pyrrolidinyl}ethanesulfonamide |
| 91 | 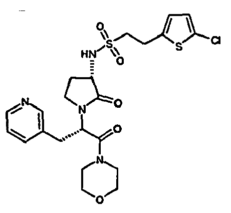 | 2-(5-chloro-2-thienyl)-*N*-{(3*S*)-1-[(1*S*)-2-(4-morpholinyl)-2-oxo-1-(3-pyridinylmethyl)ethyl]-2-oxo-3-pyrrolidinyl}ethanesulfonamide |
| 92 | 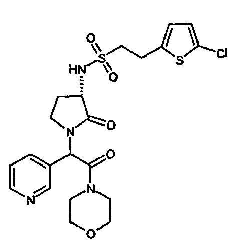 | 2-(5-chloro-2-thienyl)-*N*-{(3*S*)-1-[2-(4-morpholinyl)-2-oxo-1-(3-pyridinyl)ethyl]-2-oxo-3-pyrrolidinyl}ethanesulfonamide |
| 93 | 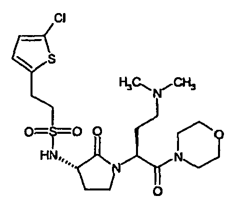 | 2-(5-chloro-2-thienyl)-*N*-{(3*S*)-1-[(1*S*)-3-(dimethylamino)-1-(4-morpholinylcarbonyl)propyl]-2-oxo-3-pyrrolidinyl}ethanesulfonamide |
| 94 | 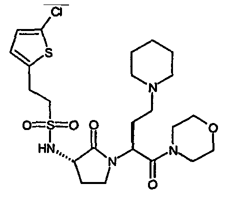 | 2-(5-chloro-2-thienyl)-*N*-{(3*S*)-1-[(1*S*)-1-(4-morpholinylcarbonyl)-3-(1-piperidinyl)propyl]-2-oxo-3-pyrrolidinyl}ethanesulfonamide |
| 95 | 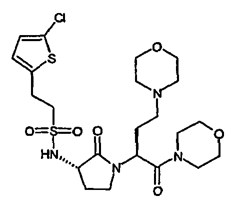 | 2-(5-chloro-2-thienyl)-*N*-{(3*S*)-1-[(1*S*)-3-(4-morpholinyl)-1-(4-morpholinylcarbonyl)propyl]-2-oxo-3-pyrrolidinyl}ethanesulfonamide |
| 96 | 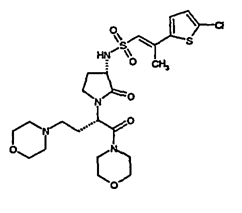 | (1*E*)-2-(5-chloro-2-thienyl)-*N*-{(3*S*)-1-[(1*S*)-3-(4-morpholinyl)-1-(4-morpholinylcarbonyl)propyl]-2-oxo-3-pyrrolidinyl}-1-propene-1-sulfonamide |
| 97 | 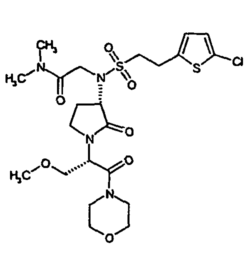 | *N*²-{[2-(5-chloro-2-thienyl)ethyl]sulfonyl}-*N*¹,*N*¹-dimethyl-*N*²-{(3*S*)-1-[(1*S*)-1-[(methyloxy)methyl]-2-(4-morpholinyl)-2-oxoethyl]-2-oxo-3-pyrrolidinyl}glycinamide |
| 98 | 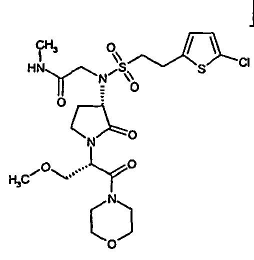 | *N*²-{[2-(5-chloro-2-thienyl)ethyl]sulfonyl}-*N*¹-methyl-*N*²-{(3*S*)-1-[(1*S*)-1-[(methyloxy)methyl]-2-(4-morpholinyl)-2-oxoethyl]-2-oxo-3-pyrrolidinyl}glycinamide |
| 99 | 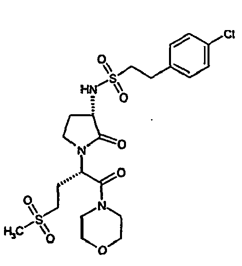 | 2-(4-chlorophenyl)-*N*-{(3*S*)-1-[(1*S*)-3-(methylsulfonyl)-1-(4-morpholinylcarbonyl)propyl]-2-oxo-3-pyrrolidinyl}ethanesulfonamide |

### Examples

### Example 1 2-(5-Chlorothien-2-yl)-N-{(3S)-1-[(1S)-1-methyl-2-momholin-4-yl-2-oxoethyl]-2-oxopyrrolidin-3-yl}ethanesulfonamide

A solution of Intermediate 35 (0.1 g) and chlorotris(triphenylphosphine)rhodium (1) (0.015g) in acetic acid (2ml) was stirred under a hydrogen atmosphere (60psi) at 60-70°C for 65h. The cooled reaction mixture was filtered through Celite and concentrated under reduced pressure to give a brown oil which was partially purified by silica gel chromatography (eluting with DCM, diethyl ether, ethyl acetate) to give an impure sample of the desired product. Further purification using mass directed preparative HPLC provided the title compound as a white solid.
RT 2.70min MH⁺ 450

Prepared in a similar manner was :

### Example 37 2-(5-Chlorothien-2-yl)-N-{(3S)-1-[(1S)-3-methyl-1-(morpholin-4-ylcarbonyl)butyl]-2-oxopyrrolidin-3-yl}ethanesulfonamide

From intermediate 39.
RT 3.16min, MH⁺ 492

### Example 2 (1E)-2-(5-Chlorothien-2-yl)-N-{(3S)-1-[(1S)-1-methyl-2-morpholin-4-yl-2-oxoethyl]-2-oxopyrrolidin-3-yl}prop-1-ene-1-sulfonamide

Intermediate 23 (190mg) was stirred in acetonitrile (15ml) at 0°C. Intermediate 5a) (120mg) and pyridine (166mg) were then added dropwise as a 5ml acetonitrile solution and the mixture was allowed to warm to room temperature., with stirrng continuing overnight. Solvent was then evaporated in vacuo and the residue partitioned between chloroform and 2N HCl/brine.. The organic layer was dried over magnesium sulphate and solvent evaporated in vacuo. Purification via silica gel chromatography (ethyl acetate), followed by further purification via HPLC gave the title compound.
RT 2.80min, MH⁺ 462

### Example 9 (1E)-2-(5-Chlorothien-2-yl)-N-{(3S)-1-[(1S)-2-methyl-1-(morpholin-4-ylcarbonyl)propyl]-2-oxopyrrolidin-3-yl}prop-1-ene-1-sulfonamide

Intermediate 5f) (60mg) was dissolved in acetonitrile (1ml) at 0°C. Pyridine (44ul) and intermediate 23 (56mg) were added. The reaction was stirred for 10 minutes then at room temperature for 2 hours. Solvent was evaporated in vacuo and purified via silica gel chromatography (ethyl acetate:cyclohexane 3:1) to give the title compound
RT 3.05 MH⁺ 491

### Example 7 (1E)-2-(5-Chlorothien-2-yl)-N-{(3S)-1-[(1S)-1-(morpholin-4-ylcarbonyl)propyl]-2-oxopyrrolidin-3-yl}prop-1-ene-1-sulfonamide

Intermediate 5b) (33mg) was dissolved in acetonitrile (0.5ml) at 0°C. Pyridine (28ul) and intermediate 23 (30mg) were added . After stirring for 15 minutes, the reaction was stirred for 1 hour. The mixture was partitioned between water (5ml) and ethyl acetate (10ml). After washing with 1N HCl and brine (5ml portions) the organics were dried (magnesium sulphate) and solvent evaporated in vacuo. Purification via silica gel chromatography (ethyl acetate:cyclohexane 3:2) gave the title compound.
RT 3.02min MH⁺ 476

### Example 10 2-(5-Chlorothien-2-yl)-N-{(3S)-1-[(1S,2S)-2-methyl-1-(morpholin-4-ylcarbonyl)butyl]-2-oxopyrrolidin-3-yl}ethanesulfonamide

Intermediate 5c) (100mg) was stirred in acetonitrile (2ml) at 0°C. Pyridine (86ul) and intermediate 15 (96mg) were added in acetonitrile (1ml). The mixture was stirred for 30 minutes at 0°C then at room temperature for 3 hours. Solvent was then evaporated in vacuo. After washing with 1N HCl and brine (5ml portions) the organics were dried (magnesium sulphate) and solvent evaporated in vacuo. Purification via silica gel chromatography (ethyl acetate:cyclohexane 1:1) gave the title compound.
RT 3.25min, MH⁺ 492

### Example 18 (1E)-2-(5-Chlorothien-2-yl)-N-{(3S)-1-[(1S,2R)-2-methoxy-1-(morpholin-4-ylcarbonyl)propyl]-2-oxopyrrolidin-3-yl}prop-1-ene-1-sulfonamide

Intermediate 5g) (37mg) was stirred in acetonitrile (1ml) at 0°C. Pyridine (28ul) and intermediate 23 (32mg) were added and the mixture stirred for 1 hour at 0°C then at room temperature for 3 hours. Evaporation of solvent in vacuo followed by purification via silica gel chromatography (ethyl acetate:cyclohexane 1:1) gave the title compound.
RT 2.91 min, MH⁺ 506

### Example 11 (1E)-2-(5-Chlorothien-2-yl)-N-{(3S)-1-[(1S,2S)-2-methyl-1-(morpholin-4-ylcarbonyl)butyl]-2-oxopyrrolidin-3-yl}prop-1-ene-1-sulfonamide

Intermediate 5c) (65mg) was stirred in acetonitrile (1.5ml) at 0°C. Pyridine (56ul) and intermediate 23 (60mg) were added in acetonitrile (1.5ml). The mixture was stirred for 30 minutes at 0°C then at room temperature for 3 hours. Solvent was then evaporated in vacuo. After washing with 1N HCl and brine (5ml portions) the organics were dried (magnesium sulphate) and solvent evaporated in vacuo.

Purification via silica gel chromatography (ethyl acetate:cyclohexane 1:1) gave the title compound.
RT 3.17min, MH⁺ 504

In a similar fashion were prepared the following:

### Example 5 2-(5-Chlorothien-2-yl)-N-{(3S)-1-[(1S)-1-(morpholin-4-ylcarbonyl)propyl]-2-oxopyrrolidin-3-yl}ethanesulfonamide

From intermediate 5b) and intermediate 15.
RT 3.17min, MH⁺ 465

Example 6 2-(4-Chlorophenyl)-N-{(3S)-1-[(1S)-1-(morpholin-4-ylcarbonyl)propyl]-2-oxopyrrolidin-3-yl}ethanesulfonamide

From intermediate 5b) and intermediate 18.
RT 3.01 min, MH⁺ 459

### Example 4 2-(4-Chlorophenyl)-N-{(3S)-1-[(1S)-1-methyl-2-morpholin-4-yl-2-oxoethyl]-2-oxopyrrolidin-3-yl}ethanesulfonamide

From intermediate 5a) and intermediate 18.
RT

Example 13 N-{(3S)-1-[(1S)-1-Benzyl-2-morpholin-4-yl-2-oxoethyl]-2-oxopyrrolidin-3-yl}-2-(5-chlorothien-2-yl)ethanesulfonamide

From intermediate 5d) and intermediate 15.
RT 3.28min, MH⁺ 527

### Example 14 (1E)-N-{(3S)-1-[(1S)-1-Benzyl-2-morpholin-4-yl-2-oxoethyl]-2-oxopyrrolidin-3-yl}-2-(5-chlorothien-2-yl)prop-1-ene-1-sulfonamide

From intermediate 5d) and intermediate 23.
RT 3.20min, MH⁺ 539

### Example 25 2-(4-Chloro-2-fluorophenyl)-N-{(3S)-1-[(1S)-1-methyl-2-morpholin-4-yl-2-oxoethyl]-2-oxopyrrolidin-3-yl}ethanesulfonamide

From Intermediate 5a) and intermediate 34.
RT 2.78min, MH⁺ 462

### Example 42 2-(2,4-Dichlorophenyl)-N-{(3S)-1-[(1S)-1-methyl-2-morpholin-4-yl-2-oxoethyl]-2-oxopyrrolidin-3-yl}ethanesulfonamide

From intermediate 5a) and intermediate 29.
RT 2.91 min, MH⁺ 478

### Example 43 2-(4-Fluorophenyl)-N-{(3S)-1-[(1S)-1-methyl-2-momholin-4-yl-2-oxoethyl]-2-oxopyrrolidin-3-yl}ethanesulfonamide

From intermediate 5a) and 2-(4-fluorophenyl)ethanesulphonyl chloride.
RT 2.59min, MH⁺ 428

### Example 44 2-(4-Methylphenyl)-N-{(3S)-1-[(1S)-1-methyl-2-morpholin-4-yl-2-oxoethyl]-2-oxopyrrolidin-3-yl}ethanesulfonamide

From intermediate 5a) and 2-(4-methylphenyl)ethanesulphonyl chloride.
RT

### Example 3 (1E)-2-(4-Chlorophenyl)-N-{(3S)-1-[(1S)-1-methyl-2-morpholin-4-yl-2-oxoethyl]-2-oxopyrrolidin-3-yl}prop-1-ene-1-sulfonamide

From intermediate 5a) and intermediate 24.
RT 2.84min, MH⁺ 455

### Example 12 2-(4-Bromophenyl)-N-{(3S)-1-[(1S,2S)-2-methyl-1-(morpholin-4-ylcarbonyl)butyl]-2-oxopyrrolidin-3-yl}ethanesulfonamide

From intermediate 5c) and 2-(4-bromophenyl)ethanesulphonyl chloride.
RT 3.19min, MH⁺ 530/532

### Example 26 2-(4-Bromophenyl)-N-{(3S)-1-[(1S)-1-(morpholin-4-ylcarbonyl)propyl]-2-oxopyrrolidin-3-yl}ethanesulfonamide

From intermediate 5b) and 2-(4-bromophenyl)ethanesulphonyl chloride.
RT 2.92, MH⁺ 502/504

### Example 33 2-(4-Bromophenyl)-N-{(3S)-1-[(1S)-1-methyl-2-morpholin-4-yl-2-oxoethyl]-2-oxopyrrolidin-3-yl}ethanesulfonamide

From intermediate 5a) and 2-(4-bromophenyl)ethanesulphonyl chloride
RT 2.80, MH⁺ 488/490

### Example 41 (1E)-2-(5-Chlorothien-2-yl)-3,3,3-trifluoro-N-{(3S)-1-[(1S)-1-methyl-2-morpholin-4-yl-2-oxoethyl]-2-oxopyrrolidin-3-yl}prop-1-ene-1-sulfonamide

From intermediate 5a) and intermediate 42.
RT 3.09min, MH⁺ 516

### Example 16 (1E)-2-(5-Chlorothien-2-yl)-N-{(3S)-1-[(1S)-1-(methoxymethyl)-2-morpholin-4-yl-2-oxoethyl]-2-oxopyrrolidin-3-yl}prop-1-ene-1-sulfonamide

Intermediate 5e) (50mg as the hydrochloride salt) was stirred in acetonitrile (1.5ml) at 0°C. N,N-diiisopropylethylamine (28ul) was added and stirring continued for 10 minutes. Pyridine (40ul) was then added followed by intermediate 23 (50mg) and stirring continued for 15 minutes at 0°C then overnight at room temperature. The mixture was partitioned between 1N HCl (10ml) and ethyl acetate (25ml). The organic layer was then washed with brine (10ml) and dried (magnesium sulphate). Solvent was evaporated in vacuo and purification via silica gel chromatography (ethyl acetate:cyclohexane 1:2) gave the title compound.
RT 2.80min, MH⁺ 492

### Example 15 2-(5-Chlorothien-2-yl)-N-{(3S)-1-[(1S)-1-(methoxymethyl)-2-morpholin-4-yl-2-oxoethyl]-2-oxopyrrolidin-3-yl}ethanesulfonamide

A solution of intermediate 5e) (100mg) was stirred in acetonitrile (4ml) at 0°C.
DIPEA (0.14ml) was added followed by DMAP (7.9mg) and intermediate 15 (100mg). The mixture was stirred for 30 minutes, then allowed to warm to room temperature and stirred for a further 90 minutes. The mixture was then partitioned between ethyl acetate (20ml) and 1 N HCl (20ml). The organic layer was washed with saturated sodium bicarbonate solution, then dried over sodium sulphate and solvent evaporated in vacuo. Purification via silica gel chromatography (ethyl acetate) gave the title compound.
RT 2.76min, MH⁺ 480

### Examples 27 2-(4-Chlorophenyl)-2,2-difluoro-N-{(3S)-1-[(1S)-1-methyl-2-morpholin-4-yl-2-oxoethyl]-2-oxopyrrolidin-3-yl}ethanesulfonamide and

### Example 28 (Z)-2-(4-Chlorophenyl)-2-fluoro-N-{(3S)-1-[(1S)-1-methyl-2-morpholin-4-yl-2-oxoethyl]-2-oxopyrrolidin-3-yl}ethenesulfonamide

Intermediate 5a) (79mg) was stirred in acetonitrile (1ml). DMAP (7mg) and N,N-diisopropylethylamine (0.1ml) were added, then the mixture was cooled to 0°C and intermediate 27 (77mg) was added as a 1ml acetonitrile solution. After 10 minutes the mixture was allowed to warm to room temperature and stirred overnight. The residue was partitioned between chloroform and 2N HCl, and the organic layer was passed through an ion exchange column then purified via silica gel chromatography.
Further HPLC purification gave Example 27 and Example 28.
Example 27 RT 2.83min, MH⁺ 480
Example 28 RT 2.75min, MH⁺ 460

The following compounds were similarly prepared.

### Example 29 and 2-(4-Chlorophenyl)-2,2-difluoro-N-{(3S)-1-[(1S,2S)-2-methyl-1-(moroholin-4-ylcarbonyl)butyl]-2-oxopyrrolidin-3-yl}ethanesulfonamide and

### Example 30 (Z)-2-(4-Chlorophenyl)-2-fluoro-N-{(3S)-1-[(1S,2S)-2-methyl-1-(morpholin-4-ylcarbonyl)butyl]-2-oxopyrrolidin-3-yl}ethenesulfonamide

From intermediate 5c) and intermediate 27. following HPLC purification.
Ex 29 RT 3.19min, MH⁺ 522
Ex 30 RT 3.13min, MH⁺ 502

### Example 21 Benzyl (3S)-3-[(3S)-3-({[2-(5-chlorothien-2-yl)ethane]sulfonyl}amino)-2-oxopyrrolidin-1-yl]-4-morpholin-4-yl-4-oxobutanoate

Intermediate 5h) (3.11g) was stirred in acetonitrile (40ml) and cooled to 0°C. DIPEA (4.6ml) was added followed by DMAP (300mg), and then a solution of intermediate 15 (2.03g) dropwise in acetonitrile. After stirring for 45 minutes at 0°C and 1 hour at room temperature, solvent was evaporated and the residue partitioned between ethyl acetate (100ml) and water (80ml). The organic layer was washed with 1 N HCl, then saturated sodium bicarbonate solution, then dried over sodium sulphate and solvent evaporated in vacuo. Purification via silica gel chromatography (ethyl acetate:cyclohexane 2:1) gave the title compound.
RT 3.24min, MH⁺ 584

### Example 17 2-(5-Chlorothien-2-yl)-N-{(3S)-1-[(1S,2R)-2-methoxy-1-(morpholin-4-ylcarbonyl)propyl]-2-oxopyrrolidin-3-yl}ethanesulfonamide

Intermediate 5g) (54mg) was stirred in acetonitrile (40ml) and cooled to 0°C. DIPEA (70ul) was added followed by DMAP (5mg), and then a solution of intermediate 15 (45mg) dropwise in acetonitrile. The mixture was stirred for 1 hour at 0°C and 1 hour at room temperature, then solvent was evaporated in vacuo and the residue partitioned chloroform (10ml) and water (10ml). The organic layer was washed with 1 N HCl, then sat. aqueous sodium bicarbonate, then dried over sodium sulphate and solvent evaporated in vacuo. Purification via silica gel chromatography (ethyl acetate:cyclohexane 2:1) gave the title compound.
RT 2.88min, MH⁺ 494

The following were prepared in a similar fashion:

### Example 23 2-(5-Chlorothien-2-yl)-N-{(3S)-1-[(1S)-2-moroholin-4-yl-2-oxo-1-(thien-2-ylmethyl)ethyl]-2-oxopyrrolidin-3-yl}ethanesulfonamide

From intermediate 5j) and intermediate 15.
RT 3.11 min, MH⁺ 532

### Example 24 2-(4-Chlorophenyl)-N-{(3S)-1-[(1S)-2-morpholin-4-yl-2-oxo-1-(thien-2-ylmethyl)ethyl]-2-oxopyrrolidin-3-yl}ethanesulfonamide

From intermediate 5j) and intermediate 18.
RT 3.13min, MH⁺ 526

### Example 8 2-(5-Chlorothien-2-yl)-N-{(3S)-1-[(1S)-2-methyl-1-(morpholin-4-ylcarbonyl)propyl]-2-oxopyrrolidin-3-yl}ethanesulfonamide

From intermediate 5f) and intermediate 15.
RT 3.06min, MH⁺ 478

### Example 45 2-(4-Chlorophenyl)-N-{(3S)-1-[(1S)-2-methyl-1-(morpholin-4-ylcarbonyl)propyl]-2-oxopyrrolidin-3-yl]ethanesulfonamide

From intermediate 5f) and intermediate 18.
RT 3.01 min, MH⁺ 471

### Example 22 Benzyl (3S)-3-[(3S)-3-({[(1E)-2-(5-chlorothien-2-yl)prop-1-enyl]sulfonyl}amino)-2-oxopyrrolidin-1-yl]-4-morpholin-4-yl-4-oxobutanoate

Intermediate 5h) (100mg) was stirred in dry acetonitrile (1.5ml) at 0°C. Pyridine (76mg) was added followed by a solution of intermediate 23 (69mg) dropwise in acetonitrile. The mixture was stirred for 20 minutes at 0°C then at room temperature for 3 hours. Solvent was evaporated and the residue partitioned between ethyl acetate (5ml) and water (5ml). The organic layer was washed with 1N HCI, then saturated sodium bicarbonate, then dried over sodium sulphate and solvent evaporated in vacuo. Purification via silica gel chromatography (ethyl acetate:cyclohexane 1:1 to 2:1) gave the title compound.
RT 3.28min MH⁺ 596

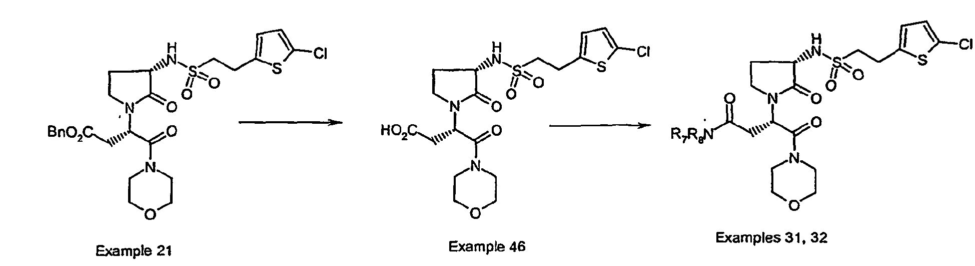

### Example 46 (3S)-3-[(3S)-3-({[2-(5-Chlorothien-2-ethane]sulfonyl}amino)-2-oxopyrrolidin-1-yl]-4-morpholin-4-yl-4-oxobutanoic acid

To example 21 (100mg) at 0°C was added 45%w/v HBr in acetic acid (2ml). The mixture was stirred for 15 minutes then allowed to warm to room temperature. After 1 hour (all starting material having dissolved) solvent was evaporated and the residue partitioned between ethyl acetate and saturated sodium bicarbonate solution. The aqueous phase was then acidified with 5N HCl and the resulting mixture extracted with ethyl acetate. Solvent was removed in vacuo to give the title compound.
RT 2.66min MH⁺ 494

### Example 31 2-(5-Chlorothien-2-yl)-N-{(3S)-1-[(1S)-3-morpholin-4-yl-1-(morpholin-4-ylcarbonyl)-3-oxopropyl]-2-oxopyrrolidin-3-yl}ethanesulfonamide

To example 46 (100mg) in DMF (2ml) was added TBTU (114mg), and the mixture was stirred at room temperature. Diisopropylethylamine (63ul) was added followed by morpholine (31ul) in DMF (1ml). After stirring for 2 hours the mixture was quenched with saturated ammonium chloride and then partitioned between ethyl acetate and water. The organic phase was washed with 2N sodium carbonate solution and dried over sodium sulphate. Solvent was evaporated to give the title compound.
RT 2.64min, MH+ 563

The following example was prepared in a similar manner:

### Example 32 (3S)-3-[(3S)-3-({[2-(5-Chlorothien-2-yl)ethane]sulfonyl}amino)-2-oxopyrrolidin-1-yl]-N,N-dimethyl-4-morpholin-4-yl-4-oxobutanamide

From example 46 + dimethylamine.
RT 2.61 min, MH⁺ 520

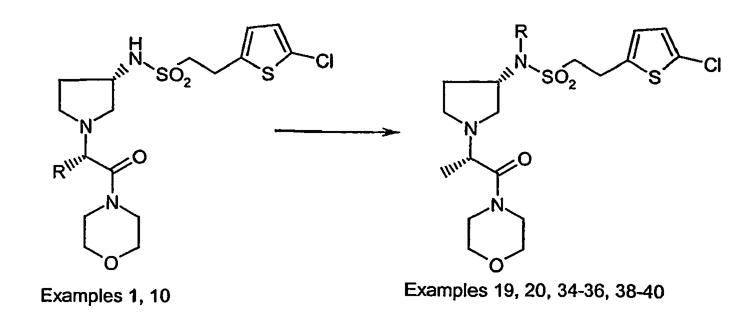

### Example 19 2-(5-Chlorothien-2-yl)-N-methyl-N-{(3S)-1-[(1S)-1-methyl-2-morpholin-4-yl-2-oxoethyl]-2-oxopyrrolidin-3-yl}ethanesulfonamide

To a solution of Example 1 (100mg) in DMF (5ml) at room temperature was added potassium carbonate (64mg) followed by methyl iodide (97mg). The mixture was stirred for 18 hours. The mixture was quenched by the addition of 2M methanolic NaOH (5ml) and DCM (5ml). The mixture was collected through a hydrophobic frit, and concentrated in vacuo to yield the title compound.
RT 3.02min, MH⁺ 464

Prepared in a similar manner were

### Example 20 N²-{[2-(5-Chlorothien-2-yl)ethyl]sulfonyl}-N²-{(3S)-1-[(1S)-1-methyl-2-moroholin-4-yl-2-oxoethyl]-2-oxopyrrolidin-3-yl}glycinamide

Example 1+ 2-bromoacetamide.
RT 2.62min, MH⁺ 507

### Example 34 Ethyl N-{[2-(5-chlorothien-2-yl)ethyl]sulfonyl}-N-{(3S)-1-[(1S)-1-methyl-2-morpholin-4-yl-2-oxoethyl]-2-oxopyrrolidin-3-yl}glycinate

Example 1 + ethyl 2-bromoacetate.
RT 3.19min, MH⁺ 536

### Example 35 Methyl N-{[2-(5-chlorothien-2-yl)ethyl]sulfonyl}-N-{(3S)-1-[(1S)-1-methyl-2-morpholin-4-yl-2-oxoethyl]-2-oxopyrrolidin-3-yl}glycinate

From Example 1 + methyl bromoacetate.
RT 3.08min, MH⁺ 522

### Example 38 2-(5-Chlorothien-2-yl)-N-methyl-N -{(3S)-1-[(1S,2S)-2-methyl-1-(momholin-4-ylcarbonyl)butyl]-2-oxopyrrolidin-3-yl}ethanesulfonamide

From Example 10 + methyl iodide.
RT 3.27min, MH⁺ 506

### Example 39 N²-{[2-(5-Chlorothien-2-yl)ethyl]sulfonyl}-N¹-methyl-N²-{(3S)-1-[(1S,2S)-2-methyl-1-(morpholin-4-ylcarbonyl)butyl]-2-oxopyrrolidin-3-yl}glycinamide

From Example 10 plus N-methyl 2-bromoacetamide.
RT 3.07min, MH⁺ 563

### Example 40 N²-{[2-(5-Chlorothien-2-yl)ethyl]sulfonyl}-N¹,N¹-dimethyl-N²-{(3S)-1-[(1S,2S)-2-methyl-1-(morpholin-4-ylcarbonyl)butyl]-2-oxopyrrolidin-3-yl}qlycinamide

From Example 10 + N,N-dimethyl 2-chloroacetamide.
RT 3.07min M⁺ 577

### Example 36 N-{[2-(5-Chlorothien-2-yl)ethyl]sulfonyl}-N-{(3S)-1-[(1S)-1-methyl-2-morpholin-4-yl-2-oxoethyl]-2-oxopyrrolidin-3-yl}glycine

Example 35 (60mg) was stirred in 1:1 THF:methanol (2ml). 2N NaOH (0.5ml) was added and the reaction stirred for 1 hour. The reaction was then acidified to pH 3 wth 2N HCl, and extracted with DCM. Concentration of the organic layer gave the title compound as a mixture of isomers.
RT 2.88 and 2.94min, both MH⁺ 508.

### Example 47 2-(5-Chloro-2-pyridinyl)-N-{(3S)-1-[(1S)-1-methyl-2-(4-morpholinyl)-2-oxoethyl]-2-oxo-3-pyrrolidinyl}ethanesulfonamide

Intermediate 5a) (100mg) was stirred in Acetonitrile (10ml) with pyridine (0.5ml) at 0°C. A solution of Intermediate 48 in Acetonitrile (5ml) was added, and the mixture was stirred, warming to room temperature, overnight. The mixture was then concentrated under reduced pressure, and the residue partitioned between water and DCM. The organic phase washed with water, dried over magnesium sulphate and solvent evaporated under reduced pressure. Purification via silica gel chromatography (ethyl acetate:MeOH 97.5:2.5-90:10) gave the title compound. RT 2.35min, MH⁺ 445

The following were prepared in a similar manner

### Example 48 2-(5-Chloro-2-pyridinyl)-N-{(3S)-1-[(1S,2S)-2-methyl-1-(4-morpholinylcarbonyl)butyl]-2-oxo-3-pyrrolidinyl}ethanesulfonamide

From Intermediate 5c) and Intermediate 48.
RT 2.84min, MH⁺ 487

### Example 49 2-(5-Chloro-2-pyridinyl)-N-{(3S)-1-[(1S)-1-[(ethyloxy)methyl]-2-(4-morpholinyl)-2-oxoethyl]-2-oxo-3-pyrrolidinyl}ethanesulfonamide

From Intermediate 5r) and Intermediate 48
RT 2.65min, MH⁺ 489

### Example 50 2-(5-chloro-2-pyridinyl)-N-{(3S)-1-[(1S)-1-[(methyloxy)methyl]-2-(4-morpholinyl)-2-oxoethyl]-2-oxo-3-pyrrolidinyl}ethanesulfonamide

From Intermediate 5e) and Intermediate 48
RT 2.35min, MH⁺ 475

### Example 51 2-(4-Chlorophenyl)-N-{(3S)-1-[(1S,2S)-2-methyl-1-(4-moroholinylcarbonyl)butyl]-2-oxo-3-pyrrolidinyl}-2-oxoethanesulfonamide

To a solution of Intermediate 51 (395mg) in THF (4ml) was added 2N HCl (3.6ml). The reaction was stirred for 10 hours at 40°C, then for 72 hours at room temperature. The reaction mixture was then partitioned between DCM and water, and the organic layer was concentrated under vacuum to give the title compound. RT 2.99min, MH⁺ 500

Prepared in a similar manner was:

### Example 52 2-(4-Chlorophenyl)-N-{(3S)-1-[(1S)-1-(methyloxy)-2-(4-morpholinyl)-2-oxoethyl]-2-oxo-3-pyrrolidinyl}-2-oxoethanesulfonamide

From Intermediate 52
RT 2.61 min, MH⁺ 488

### Example 53 2-(4-Chlorophenyl)-2-hydroxy-N-{(3S)-1-[(1S,2S)-2-methyl-1-(4-morpholinylcarbonyl)butyl]-2-oxo-3-pyrrolidinyl}ethanesulfonamide

To a solution of Example 51 (150mg) in dry ethanol (0.2ml) at 0°C was added sodium borohydride (18mg), keeping the reaction temperature below 10°C. The reaction was then stirred for 2 hours at 0°C. The reaction was quenched via addition of water, then partitioned with chloroform. After evaporation of the organic layer under vacuum, the residue was purified via silica chromatography (50:50 cyclohexane: ethyl acetate) to give the title compound as a mixture of isomers.
RT 2.92, 2.97 MH⁺ 502

### Example 54 2-(5-Chloro-2-thienyl)-N-{(3S)-1-[(1S)-1-methyl-2-(4-morpholinyl)-2-oxoethyl]-2-oxo-3-pyrrolidinyl}-1-propanesulfonamide

Prepared according to the procedure for Example 15 from Intermediate 5a) and Intermediate 54.
RT 2.81 MH⁺ 464
Chiral HPLC separation (Chiralpak AD, 9:1 EtOH:Cycloheptane, 1ml/min, @215nm) of Example 54 yielded the two isomers.

### Example 55 (2R)-2-(5-Chloro-2-thienyl)-N-{(3S)-1-[(1S)-1-methyl-2-(4-morpholinyl)-2-oxoethyl]-2-oxo-3-pyrrolidinyl}-1-propanesulfonamide and Example 56 (2S)-2-(5-Chloro-2-thienyl)-N-{(3S)-1-[(1S)-1-methyl-2-(4-morpholinyl)-2-oxoethyl]-2-oxo-3-pyrrolidinyl}-1-propanesulfonamide.

Retention times Example 55: 20.2 minutes, Example 56: 9.4 minutes

### Example 57 2-(5-Chloro-2-thienyl)-N-{(3S)-1-((1S,2S)-2-methyl-1-(4-morpholinylcarbonyl)butyl]-2-oxo-3-pyrrolidinyl}-1-propanesulfonamide

Prepared according to the procedure for Example 15 from Intermediate 5c) and Intermediate 54.
RT 3.19 MH⁺ 506

### Example 58 2-(5-Chloro-2-thienyl)-N-{(3S)-1-[(1S)-1-[(methyloxy)methyl]-2-(4-morpholinyl)-2-oxoethyl]-2-oxo-3-pyrrolidinyl}-1-propanesulfonamide

Prepared according to the procedure for Example 15 from Intermediate 5e) and Intermediate 54.
RT 2.83 MH⁺ 494

### Example 59 2-(5-Chloro-2-thienyl)-N-{(3S)-1-[(1S)-1-[(ethyloxy)methyl]-2-(4-morpholinyl)-2-oxoethyl]-2-oxo-3-pyrrolidinyl}-1-propanesulfonamide

Prepared according to the procedure for Example 15 from Intermediate 5r) and Intermediate 54.
RT 2.95 MH⁺ 508

### Example 60 2-(5-Chloro-2-thienyl)-N-{(3S)-1-[(1S)-1-[(3-methyl-1,2,4-oxadiazol-5-yl)methyl]-2-(4-morpholinyl)-2-oxoethyl]-2-oxo-3-pyrrolidinyl}-1-propanesulfonamide Prepared according to the procedure for Example 15 from Intermediate 78 and Intermediate 54.

RT 2.92 MH⁺ 546

### Example 61 1-(5-Chloro-2,3-dihydro-1H-inden-1-yl)-N-{(3S)-1-[(1S)-1-methyl-2-(4-morpholinyl)-2-oxoethyl]-2-oxo-3-pyrrolidinyl}methanesulfonamide

Intermediate 5a) (66mg) DIPEA (70ul) and DMAP (5mg) were stirred in Acetonitrile at 0°C. Intermediate 58 (86mg) was added, and the mixture was allowed to warm to room temperature and stirred for a further 3 hours. Solvent was then removed under vacuum, and the crude product purified via silica chromatography.
RT 2.90 MH⁺ 470

Prepared in a similar manner was:

### Example 62 1-(5-Chloro-2,3-dihydro-1H-inden-1-yl)-N-{(3S)-1-[(1S)-1-[(methyloxy)methyl]-2-(4-morpholinyl)-2-oxoethyl]-2-oxo-3-pyrrolidinyl}methanesulfonamide

From Intermediate 58 and intermediate 5e).
RT 2.90 MH⁺ 500

### Example 63 1-(5-Chloro-2,3-dihydro-1H-inden-1-yl)-N-{(3S)-1-[(1S,2S)-2-methyl-1-(4-morpholinylcarbonyl)butyl]-2-oxo-3-pyrrolidinyl}methanesulfonamide

From Intermediate 58 and intermediate 5c).
RT 3.35 MH⁺ 512

### Example 64 1-(6-Chloro-2,3-dihydro-1-benzofuran-3-yl)-N-{(3S)-1-[(1S)-1-methyl-2-(4-morpholinyl)-2-oxoethyl]-2-oxo-3-pyrrolidinyl}methanesulfonamide

Prepared according to the procedure for Example 15, from intermediate 5a) and Intermediate 61.
RT 2.80, 2.84 (diastereomers) MH⁺ 472

### Example 65 1-(6-Chloro-2,3-dihydro-1-benzofuran-3-yl)-N-{(3S)-1-[(1S)-1-[(methyloxy)methyl]-2-(4-morpholinyl)-2-oxoethyl]-2-oxo-3-pyrrolidinyl}methanesulfonamide

Prepared according to the procedure for Example 15, from intermediate 5e) and Intermediate 61.
RT 2.82, 2.86 (diastereomers) MH⁺ 502

### Example 66 1-(5-Chloro-1,3-dihydro-2-benzofuran-1-yl)-N-{(3S)-1-[(1S)-1-methyl-2-(4-morpholinyl)-2-oxoethyl]-2-oxo-3-pyrrolidinyl}methanesulfonamide

Prepared according to the procedure for Example 15 from Intermediate 5a) and intermediate 66.
RT 2.62min, MH⁺ 472

### Example 67 2-(5-Chloro-2-thienyl)-N-{(3S)-1-[2-(4-morpholinyl)-2-oxo-1-(tetrahydro-2H-pyran-4-yl)ethyl]-2-oxo-3-pyrrolidinyl}ethanesulfonamide

Prepared according to the procedure for Example 17 from Intermediate 5k) and intermediate 15.
RT 2.84min, MH⁺ 520

### Example 68 1-[(1R)-5-Chloro-2,3-dihydro-1H-inden-1-yl]-N-{(3S)-1-[(1S)-1-[(methyloxy)methyl]-2-(4-morpholinyl)-2-oxoethyl]-2-oxo-3-pyrrolidinyl}methanesulfonamide

Isolated via Chiral HPLC resolution of Example 62 (Chiralpak AD 25cm column, eluting with heptane:isopropanol 7:3, flow-rate 1ml/min).
RT 16.6min

Prepared in a similar manner to Example 67 were:

### Example 69 2-(5-Chloro-2-thienyl)-N-{(3S)-1-[(1S)-2-(4-morpholinyl)-2-oxo-1-phenylethyl]-2-oxo-3-pyrrolidinyl}ethanesulfonamide

From Intermediate 5l) and intermediate 15.
RT 3.07min, MH⁺ 512

### Example 70 2-(5-Chloro-2-thienyl)-N-{(3S)-1-[(1S)-1-[(4-fluorophenyl)methyl]-2-(4-morpholinyl)-2-oxoethyl]-2-oxo-3-ayrrolidinyl}ethanesulfonamide

From Intermediate 5m) and intermediate 15.
RT 3.17min, MH⁺ 544

### Example 71 2-(5-Chloro-2-thieyl)-N-{(3S)-1-[(1S)-2-(4-morpholinyl)-2-oxo-1-(1,3-thiazol-4-ylmethyl)ethyl]-2-oxo-3-pyrrolidinyl}ethanesulfonamide

From Intermediate 5n) and intermediate 15, followed by further purification on an SCX column (methanolic ammonia as eluant)
RT 2.88min, MH⁺ 533

### Example 72 2-(4-Chlorophenyl)-N-{(3S)-1-[(1S)-2-(4-morpholinyl)-2-oxo-1-(1,3-thiazol-4-ylmethyl)ethyl]-2-oxo-3-pyrrolidinyl}ethanesulfonamide

From Intermediate 5n) and intermediate 18 followed by further purification on an SCX column (methanolic ammonia as eluant)
RT 2.90min, MH⁺ 527

### Example 73 2-(5-Chloro-2-thienyl)-N-{(3S)-1-[(1R)-2-(4-morpholinyl)-2-oxo-1-(2-thienyl)ethyl]-2-oxo-3-pyrrolidinyl}ethanesulfonamide

From Intermediate 5p) and intermediate 15.
RT 3.03min, MH⁺ 518

### Example 74 2-(4-Chlorophenyl)-N-{(3S)-1-[(1R)-2-(4-morpholinyl)-2-oxo-1-(2-thienyl)ethyl]-2-oxo-3-pyrrolidinyl}ethanesulfonamide

From Intermediate 5p) and intermediate 18.
RT 3.06min, MH⁺ 512

### Example 75 2-(5-Chloro-2-thienyl)-N-{(3S)-1[(1R)-1-[(ethylthio)methyl]-2-(4-morpholinyl)-2-oxoethyl]-2-oxo-3-pyrrolidinyl}ethanesulfonamide

Intermediate 13j) (42mg) was taken up in Acetonitrile (1ml) and N-methyl morpholine (108mg) was added, followed by Intermediate 68. The mixture was then heated to reflux for 5 hours. DMAP (27mg) was then added and the mixture was heated to reflux for 24 hours. After cooling to room temperature, solvent was evaporated in vacuo, and the crude product purified via SPE chromatography (cyclohexane: ethyl acetate 3:2 to 1:4) to give the title compound.
RT 3.07min, MH⁺ 510

### Example 76 2-(5-Chloro-2-thienyl)-N-{(3S)-1-[(1S)-1-(cyanomethyl)-2-(4-morpholinyl)-2-oxoethyl]-2-oxo-3-pyrrolidinyl}ethanesulfonamide

Intermediate 72 (80mg) was taken up in dry acetonitrile (2ml) and DIPEA (0.173ml) was added. DMAP (12mg) was added, and the mixture was cooled to 0°C. Intermediate 15 (74mg) was added as a 1ml acetonitrile solution, and stirring was continued for 30 minutes, followed by warming to room temperature and further stirring for 2 hours. Solvent was evaporated under reduced pressure, and the residue was taken up in THF (3ml) containing DIPEA (0.173ml) and cooled to 0°C Trifluoroacetic anhydride (0.07ml) was added, and the mixture was stirred for 90 minutes, warming to room temperature. Solvent was again evaporated under reduced pressure, and the residue was partitioned between ethyl acetate and sat. aq. NaHCO₃. The organic layer was separated and dried over Na₂SO₄, and evaporated under reduced pressure. Purification via SPE chromatography (cyclohexane: ethyl acetate 1:2) gave the partially purified product which was recystallised (ethyl acetate / cyclohexane) to give the title compound. RT 2.78min, MH⁺ 475

### Example 77 2-(5-Chloro-2-thienyl)-N-{(3S)-1-[(1S)-2-(4-morpholinyl)-1-(1,2,4-oxadiazol-5-ylmethyl)-2-oxoethyl]-2-oxo-3-pyrrolidinyl}ethanesulfonamide

Intermediate 74 (110mg) was dissolved in dioxan (1ml) and the resulting solution was added to a mixture of hydroxylamine hydrochloride (17mg) and 5N NaOH (0.05ml) in 70% acetic acid (1ml). The mixture was then heated to 70°C for 3 hours, cooled to room temperature, and partitioned between DCM (15ml) and water (5ml). The organic phase was washed sequentially with sat. aq.NaHCO₃, and brine, and solvent evaporated under vacuum. Purification via Mass Directed Autoprep gave the title compound.
RT 2.89min, MH⁺ 518

Prepared in a similar manner were:

### Example 78 2-(5-Chloro-2-thienyl)-N-{(3S)-1-[(1S)-1-[(3-methyl-1,2,4-oxadiazol-5-yl)methyl]-2-(4-morpholinyl)-2-oxoethyl]-2-oxo-3-pyrrolidinyl}ethanesulfonamide and

### Example 79 2-(5-Chloro-2-thienyl)-N-methyl-N-{(3S)-1-[(1S)-1-[(3-methyl-1,2,4-oxadiazol-5-yl)methyl]-2-(4-morpholinyl)-2-oxoethyl]-2-oxo-3-Pyrrolidinyl}ethanesulfonamide

From intermediate 75, with purification via Mass Directed preparative HPLC.
Example 78
RT 2.85min, MH⁺ 532
Example 79
RT 2.99min, MH⁺ 546

### Example 80 2-(5-Chloro-2-thienyl)-N-{(3S)-1-[(1S)-1-[(1-methyl-1H-1,2,4-triazol-3-yl)methyl]-2-(4-morpholinyl)-2-oxoethyl]-2-oxo-3-pyrrolidinyl}ethanesulfonamide and

### Example 81 2-(5-Chloro-2-thienyl)-N-{(3S)-1-[(1S)-1-[(1-methyl-1H-1,2,4-triazol-5-yl)methyl]-2-(4-morpholinyl)-2-oxoethyl]-2-oxo-3-pyrrolidinyl}ethanesulfonamide

To Intermediate 74 (144mg) in glacial acetic acid (3ml) was added methyl hydrazine (16ul). The mixture was then heated to 70 °C for 2 hours, then for 3 hours at 80°C. The mixture was then cooled to room temperature and partitioned between DCM and 2N Na₂CO₃ solution. The organic layer was evaporated under vacuum to give the crude product. Purification via preparative HPLC (autoprep) gave the two title compounds.
Example 80
RT 2.56min, MH⁺ 531
Example 81
RT 2.60min, MH⁺ 531

### Example 82 2-(4-Chlorophenyl)-N-{(3S)-1-[(1S)-1-[(3-methyl-1,2,4-oxadiazol-5-yl)methyl]-2-(4-morpholinyl)-2-oxoethyl]-2-oxo-3-pyrrolidinyl}ethanesulfonamide

Prepared according to the procedure for Example 15 from Intermediate 78 and intermediate 18.
RT 2.85min, MH⁺ 526

### Example 83 (3S)-3-[(3S)-3-({[2-(5-Chloro-2-thienyl)ethyl]sulfonyl}amino)-2-oxo-1-pyrrolidinyl]-N-methyl-4-(4-morpholinyl)-4-oxo-N-(phenylmethyl)butanamide

Example 46 (R8446/143/2) (80mg) and TBTU (91mg) were stirred together in dry DMF (2ml) at room temperature. DIPEA (50ul) was added followed by N-benzylmethylamine (34mg). The mixture was stirred for 3 hours, then quenched with sat. aqueous ammonium chloride. Water and ethyl acetate were added, the mixture partitioned, and the organic phase washed sequentially with 2N Na₂CO₃ solution, 1 N HCl solution, and brine, then dried (Na₂SO₄) and solvent evaporated under vacuum. Purification via Biotage™ chromatography (DCM:methanol 50:1) gave the title compound.
RT 3.06min, MH⁺ 597

### Example 84 2-(5-Chloro-2-thienyl)-N-{(3S)-1-[(1S)-1-{[(1-methylethyl)oxy]methyl}-2-(4-morpholinyl)-2-oxoethyl]-2-oxo-3-pyrrolidinyl}ethanesulfonamide

Prepared in a similar fashion to Example 15, from Intermediate 5q) and intermediate 15.
RT 2.97min, MH⁺ 504

### Example 85 2-(5-Chloro-2-thienyl)-N-{(3S)-1-[(1S)-1-[(ethyloxy)methyl]-2-(4-morpholinyl)-2-oxoethyl]-2-oxo-3-pyrrolidinyl}ethanesulfonamide

Prepared in a similar fashion to Example 15, from Intermediate 5r) and Intermediate 15.
RT 2.85min, MH⁺ 494

### Example 86 2-(4-Chlorophenyl)-N-{(3S)-1-[(1S)-1-[(ethyloxy)methyl]-2-(4-morpholinyl)-2-oxoethyl]-2-oxo-3-pyrrolidinyl}ethanesulfonamide

Prepared in a similar fashion to Example 16, from Intermediate 5r) and intermediate 18.
RT 2.87min, MH⁺ 488

### Example 87 (1E)-2-(5-Chloro-2-thienyl)-N-{(3S)-1-[(1S)-1-[(ethyloxy)methyl]-2-(4-morpholinyl)-2-oxoethyl]-2-oxo-3-pyrrolidinyl}-1-propene-1-sulfonamide

Prepared in a similar fashion to Example 16, from Intermediate 5r) and Intermediate 23.
RT 2.91 min, MH⁺ 506

### Example 88 2-(5-Chloro-2-thienyl)-N-{(3S)-1-[(1S)-3-(methyloxy)-1-(4-morpholinylcarbonyl)propyl]-2-oxo-3-pyrrolidinyl}ethanesulfonamide

Prepared in a similar fashion to Example 15, from Intermediate 5s) and Intermediate 15.
RT 2.78min, MH⁺ 494

### Example 89 2-(4-Chlorophenyl)-N-{(3S)-1-[(1S)-1-[(methyloxy)methyl]-2-(4-morpholinyl)-2-oxoethyl]-2-oxo-3-pyrrolidinyl}ethanesulfonamide

Prepared according to the procedure for Example 15, from Intermediate 5e) and Intermediate 18.
RT 2.76min, MH⁺ 474

### Example 90 2-(5-Chloro-2-thienyl)-N-{(3S)-1-[(1S)-2-(4-morpholinyl)-2-oxo-1-(4-pyridinylmethyl)ethyl]-2-oxo-3-pyrrolidinyl}ethanesulfonamide

Prepared according to the procedure for Example 15, from Intermediate 5t) and Intermediate 15.
RT 2.34min, MH⁺ 527

### Example 91 2-(5-Chloro-2-thienyl)-N-{(3S)-1-[(1S)-2-(4-morpholinyl)-2-oxo-1-(3-pyridinylmethyl)ethyl]-2-oxo-3-pyrrolidinyl}ethanesulfonamide

Prepared according to the procedure for Example 15, from Intermediate 5u) and Intermediate 15.
RT 2.46min, MH⁺ 527

### Example 92 2-(5-Chloro-2-thienyl)-N-{(3S)-1-[2-(4-morpholinyl)-2-oxo-1-(3-pyridinyl)ethyl]-2-oxo-3-pyrrolidinyl}ethanesulfonamide

Intermediate 9v) (86mg) was taken up in dry DCM (5ml) and treated with 4M HCL in dioxan (0.35ml). The mixture was stirred for 24 hours, then evaporated under reduced pressure. The crude product was taken up in dry acetonitrile (3ml), treated with DIPEA (0.147ml) and the mixture cooled to 0 °C. DMAP (3mg) was added followed by intermediate 15 (74mg). After stirring for 30 minutes, the mixture was allowed to warm to room temperature. The reaction mixture was purified directly via SPE chromatography (40:1 ethyl acetate:MeOH) to give the title compound as an approximate 1:1 mixture of pyridylglycine isomers.
RT 2.67min, MH⁺ 513

### Example 93 2-(5-Chloro-2-thienyl)-N-{(3S)-1-[(1S)-3-(dimethylamino)-1-(4-morpholinylcarbonyl)propyl]-2-oxo-3-pyrrolidinyl}ethanesulfonamide

Intermediate 5w) (56mg) was taken up in dry acetonitrile (2ml) and DIPEA (104ul) added. 4-Dimethylaminopyridine (6mg) was then added and the mixture was cooled to 0°C. Intermediate 15 (33mg) was added in dry acetonitrile (0.5ml). Stirring was continued for 2 hours, warming to room temperature. The crude product was purified firstly via SCX column (eluting with 2M methanolic ammonia) and then via Mass directed preparative HPLC to yield the title compound.
RT 2.19min, MH⁺ 507

In a similar fashion were prepared:

### Example 94 2-(5-Chloro-2-thienyl)-N-{(3S)-1-[(1S)-1-(4-morpholinylcarbonyl)-3-(1-piperidinyl)propyl]-2-oxo-3-pyrrolidinyl}ethanesulfonamide

From Intermediate 5x) and Intermediate 15.
RT 2.28min, MH⁺ 547

### Example 95 2-(5-Chloro-2-thienyl)-N-{(3S)-1-[(1S)-3-(4-morohotinyl)-1-(4-morpholinylcarbonyl)propyl]-2-oxo-3-pyrrolidinyl}ethanesulfonamide

From Intermediate 5y) and Intermediate 15
RT 2.21 min, MH⁺ 549

### Example 96 (1E)-2-(5-Chloro-2-thienyl)-N-{(3S)-1-[(1S)-3-(4-morpholinyl)-1-(4-morpholinylcarbonyl)propyl]-2-oxo-3-pyrrolidinyl}-1-propene-1-sulfonamide

From Intermediate 5y) and Intermediate 23
RT 2.26min, MH⁺ 560

### Example 97 N²-{[2-(5-Chloro-2-thienyl)ethyl]sulfonyl}-N¹,N¹-dimethyl-N²-{(3S)-1-[(1S)-1-[(methyloxy)methyl]-2-(4-morpholinyl)-2-oxoethyl]-2-oxo-3-pyrrolidinyl}glycinamide

Prepared according to the procedure for Example 19 from Example 15 and 2-chloro-N,N-dimethylacetamide. Purification via SPE chromatography gave the title compound.
RT 2.75min, MH⁺ 565

### Example 98 N²-{[2-(5-Chloro-2-thienyl)ethyl]sulfonyl}-N¹-methyl-N²-{(3S)-1-[(1S)-1-[(methyloxy)methyl]-2-(4-morpholinyl)-2-oxoethyl]-2-oxo-3-pyrrolidinyl}glycinamide

Prepared according to the procedure for Example 19 from Example 15 and 2-chloro-N-methylacetamide. Purification via SPE chromatography gave the title compound. RT 2.68min, MH⁺ 551

### Example 99 2-(4-Chlorophenyl)-N-{(3S)-1-[(1S)-3-(methylsulfonyl)-1-(4-morpholinylcarbonyl)propyl]-2-oxo-3-pyrrolidinyl}ethanesulfonamide

Prepared from Intermediate 91 and Intermediate 18 according to the procedure for Example 15.
RT 2.68min, MH⁺ 536

### Biological assays

### I. Thrombin inhibitory activity

### In vitro assay for inhibition of Thrombin

### (A) Chromogenic assay

Example 2 was tested for Thrombin inhibitory activity as determined *in vitro* by its ability to inhibit human Thrombin in a chromogenic assay, using N-p-Tosyl-Gly-Pro-Lys-p-nitroanilide as the chromogenic substrate. The compound was diluted from a 10mM stock solution in dimethylsulfoxide at appropriate concentrations. Assay was performed at room temperature using buffer consisting of: 50mM HEPES, 150mM NaCl, 5mM CaCl₂, 0.1% PEG, pH 7.4. containing human Thrombin (final conc. of 1 nM). Compound and enzyme were preincubated for 15min prior to addition of the substrate (final conc. of 100 µM). The reaction was stopped after 30min with the addition of soybean trypsin inhibitor or H-D-PHE-PRO-ARG-Chloromethylketone. BioTek EL340 or Tecan SpectraFluor Plus plate readers were used to monitor the absorbance at 405nM. To obtain IC₅₀ values the data were analysed using ActivityBase® and XLfit®.

### (B) Fluorogenic assay

Compounds of the present invention (Examples 1, 3-46) were tested for their Thrombin inhibitory activity as determined *in vitro* by their ability to inhibit human Thrombin in a fluorogenic assay, using Rhodamine 110, bis-(CBZ-L-valyl-L-prolyl-L-arginine amide) as the fluorogenic substrate. Compounds were diluted from a 10mM stock solution in dimethylsulfoxide at appropriate concentrations. Assay was performed at room temperature using buffer consisting of: 50mM HEPES, 150mM NaCl, 5mM CaCl₂, 0.1% PEG, pH 7.4. containing human Thrombin (final conc. Of 0.2 nM). Compound and enzyme were preincubated for 15min prior to addition of the substrate (final conc. of 10 µM). The reaction was stopped after 3 hrs with the addition of H-D-PHE-PRO-ARG-Chloromethylketone. An LJL- Analyst fluorimeter was used to monitor fluorescence at 485 nM excitation/535 nM emission . To obtain IC₅₀ values the data were analysed using ActivityBase® and XLfit®.

All of the Examples 1-54, 57-59 showed thrombin inhibitory activity. Examples 1, 2, 3, 4, 5, 6, 7, 8, 9, 10, 11, 12, 13, 14, 15, 16, 17, 18, 19, 20,21, 22, 23, 24, 25, 26, 27, 28, 29, 30, 31, 32, 33, 34, 35, 36, 37, 38, 39, 40, 41, 42, 45, 46, 47, 48, 49, 50, 51, 52, 53, 54, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 all have thrombin inhibitory Ki (nM) values of less than 200. Examples 1, 2, 3, 4, 5, 6, 7, 8, 9, 10, 11, 12, 13, 14, 15, 16, 17, 18, 19, 20,21, 22, 23, 24, 26, 27, 28, 29, 32, 33, 34, 35, 36, 37, 38, 39, 40, 41, 45, 47, 48, 49, 51, 52, 53, 54, 57, 58, 59, 60, 61, 63, 64, 65, 66, 68, 69, 70, 71, 73, 74, 75, 76, 77, 78, 79, 80, 81, 82, 83, 84, 85, 86, 87, 88, 89, 90, 91, 92, 93, 94, 95, 96, 97, 98 all have thrombin inhibitory Ki (nM) values of less than 100. Examples 1, 2, 3, 4, 5, 7, 8, 9, 10, 11, 12, 13, 14, 15, 16, 17, 18, 19, 20,21, 22, 23, 24, 27, 28, 29, 32, 33, 34, 35, 38, 39, 40, 45, 48, 49, 51, 52, 57, 59, 60, 63, 66, 68, 70, 71, 73, 75, 77, 78, 79, 80, 83, 84, 85, 86, 87, 88, 89, 90, 91, 92, 96, 97, 98 all have thrombin inhibitory Ki (nM) values of less than 50. Examples 2, 3, 5, 7, 8, 9, 10, 11, 12, 13, 14, 16, 17, 18, 20,21, 22, 23, 24, 27, 28, 29, 33, 34, 35, 38, 39, 40, 48, 49, 57, 59,77, 78, 84, 85, 86, 87, 88, 91, 98 all have thrombin inhibitory Ki (nM) of less than 25. Examples 2, 7, 8, 9, 10, 11, 14, 16, 17, 18, 21, 22, 23, 38, 40, 77, 84, 85, 87, 91, 98 all have thrombin inhibitory Ki (nM) values of less than 10.

### II. Factor Xa inhibitory activity

### (A) Chromogenic assay

Example 2 was tested for its Factor Xa inhibitory activity as determined *in vitro* by its ability to inhibit human Factor Xa in a chromogenic assay, using N-α-benzyloxycarbonyl-D-Arg-Gly-Arg-p-nitroanilide as the chromogenic substrate. Compounds were diluted from a 10mM stock solution in dimethylsulfoxide at appropriate concentrations. Assay was performed at room temperature using buffer consisting of: 50mM Tris-HCI, 150mM NaCl, 5mM CaCl₂, pH 7.4. containing human Factor Xa (final conc. Of 0.0015 U.ml-1). Compound and enzyme were preincubated for 15min prior to addition of the substrate (final conc. of 200µM). The reaction was stopped after 30min with the addition of soybean trypsin inhibitor or H-D-PHE-PRO-ARG-Chloromethylketone. BioTek EL340 or Tecan SpectraFluor Plus plate readers were used to monitor the absorbance at 405nM. To obtain IC₅₀ values the data were analysed using ActivityBase® and XLfit®.

### (B) Fluorogenic assay

Compounds of the present invention (Examples 1, 3-46) were tested for their Factor Xa inhibitory activity as determined *in vitro* by their ability to inhibit human Factor Xa in a fluorogenic assay, using Rhodamine 110, bis-(CBZ-glycylglycyl-L-arginine amide as the fluorogenic substrate. Compounds were diluted from a 10mM stock solution in dimethylsulfoxide at appropriate concentrations. Assay was performed at room temperature using buffer consisting of: 50mM Tris-HCl, 150mM NaCl, 5mM CaCl₂, pH 7.4. containing human Factor Xa (final conc. of 0.0003U.ml-1). Compound and enzyme were preincubated for 15min prior to addition of the substrate (final conc. of 10 µM). The reaction was stopped after 3 hrs with the addition of H-D-PHE-PRO-ARG-Chloromethylketone. An LJL-Analyst fluorimeter was used to monitor fluorescence with 485 nM excitation/535 nM emission. To obtain IC₅₀ values the data were analysed using ActivityBase® and XLfit®.

The ratio of inhibitory activity at thrombin compared to Factor Xa can be calculated as Factor Xa Ki (nM)/Thrombin Ki (nm)). Examples 2, 20, 34, 35, 36, 41, 42, 94, 96 have a ratio of inihibitory activity 0-2. Examples 3, 19, 25, 28, 47, 54, 61 have a ratio of inhibitory activity of 2-5. Examples 7, 27, 95 have a ratio of inhibitory activity of 5-10. Examples 1, 4, 16, 33, 43, 44, 64, 66, 69, 92 have a ratio of inhibitory activity of 10-25. Examples 5, 6, 8, 9, 10, 11, 12, 13, 14, 15, 17, 18, 21, 22, 23, 24, 26, 29, 30, 31, 32, 37, 38, 39, 40, 45, 46, 48, 49, 50, 51, 52, 53, 57, 58, 59, 60, 62, 63, 65, 67, 68, 70, 71, 72, 73, 74, 75, 76, 77, 78, 79, 80, 81, 82, 83, 84, 85, 86, 87, 88, 89, 90, 91, 93, 97, 98, 99 have a ratio of inhibitory activity of greater than 25.

The ratio of inhibitory activity for prior art compounds (E)-2-(4-chlorophenyl)-N-{(3S)-1-[(1*S*)-1-methyl-2-morpholin-4-yl-2-oxoethyl]-2-oxopyrrolidin-3-yl}ethenesulfonamide and (E)-2-(5-chlorothien-2-yl)-N-{(3S)-1-[(1S)-1-methyl-2-morpholin-4-yl-2-oxoethyl]-2-oxopyrrolidin-3-yl}ethenesulfonamide is less than 0.04.

### Method for measurement of activated partial prothrombin time (aPTT)

Blood is collected into a sodium citrate solution (ratio 9:1) to give a final concentration of 0.38% citrate. Plasma is generated by centrifugation of citrated blood samples at 1200 x g for 20 min at 4°C and stored at -20°C until use. APTT analysis is conducted using plasma pooled from 4 separate donors (2 male and 2 female).

The aPTT test is performed using the BCS Coagulation Analyzer (Dade Behring). For assay, 50 ul of plasma containing test compound at concentrations ranging from 0.03 to 100 uM (made from a 100 uM stock containing 10% DMSO in plasma) is combined with 50 ul of Actin Activated Cephaloplastin Reagent (extracted from dehydrated rabbit brain; Dade Behring) and 50 ul of 0.025 M Calcium Chloride (Dade Behring). Upon addition of the reagents, absorbance at 405 nM is monitored and time to clot formation is determined (normal range for human plasma is 24-32 seconds). Results are expressed as the concentration required to extend the time to clot formation by 50%.

All Examples tested (Examples 1, 2, 3, 4, 5, 6, 7, 8, 10, 11, 12, 13, 14, 15, 16, 17, 18, 19, 20, 21, 22, 23, 24, 25, 26, 27, 28, 29, 32, 33, 37, 38, 39, 40, 41, 45, 48, 49, 50, 51, 52, 53, 54, 57, 58, 59, 60, 61, 63, 67, 69, 70, 71, 73, 74, 75, 76, 77, 78, 79, 80, 81, 82, 83, 84, 85, 86, 87, 88, 89, 90, 91, 92, 93, 94, 95, 96, 97, 98, 99) had 1.5x APTT values less than 30µM. Examples 1, 2, 3, 4, 5, 6, 7, 8, 10, 11, 12, 13, 14, 15, 16, 17, 18, 19, 20, 21, 22, 24, 25, 26, 27, 28, 29, 33, 37, 38, 39, 40, 45, 48, 49, 50, 51, 52, 53, 54, 57, 58, 59, 60, 67, 69, 70, 71, 73, 74, 75, 76, 77, 78, 79, 80, 81, 82, 83, 84, 85, 86, 87, 88, 89, 90, 91, 92, 93, 94, 95, 96, 97, 98, 99 had 1.5x APTT values less than 10µM. Examples 7, 11, 14, 15, 20, 22, 23, 28, 32, 38, 39, 40, 48, 49, 50, 51, 58, 59, 71, 77, 78, 80, 84, 85, 86, 87, 88, 89, 90, 91, 93, 94, 96, 98 had 1.5x APTT values less than 2µM.

## Claims

1. A compound of formula (I): 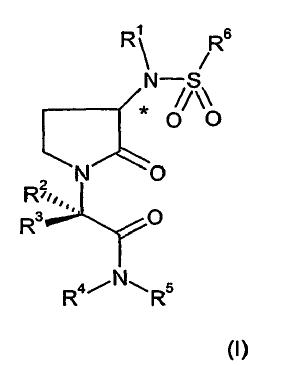 wherein:
R¹ represents hydrogen, C₁₋₄alkyl, -CH₂CO₂H, -CH₂CO₂C₁₋₂alkyl, or -CH₂CONR⁷R⁸;
R² and R³ independently represent hydrogen, -C₁₋₆alkyl, -C₁₋₃alkylCN, -C₁₋₃alkylCO₂H, -C₁₋₄alkylOC₁₋₄alkyl, -C₁₋₄alkylS(O)ₙC₁₋₄alkyl, -C₁₋₄alkylNR¹⁰R¹¹, -C₁₋₃alkylNCO₂C₁₋₄alkyl, -C₁₋₃alkylCONR⁷R⁸, -C₁₋₃alkylCO₂C₀₋₂alkylR⁹, -C₁₋₃alkylCOC₀₋₂alkylR⁹, -C₁₋₃alkylCON(R⁸)C₀₋₂alkylR⁹, -C₁₋₃alkylNCO₂C₀₋₂alkylR⁹, -C₁₋₃alkylNCOC₀₋₂alkylR⁹ or -C₀₋₂alkylR⁹, with the proviso that one of R² and R³ is hydrogen and the other is a substituent other than hydrogen;
n is an integer between 0 and 2;
R⁴ and R⁵ together with the nitrogen atom to which they are attached form a morpholino ring;
R⁶ represents a group selected from: 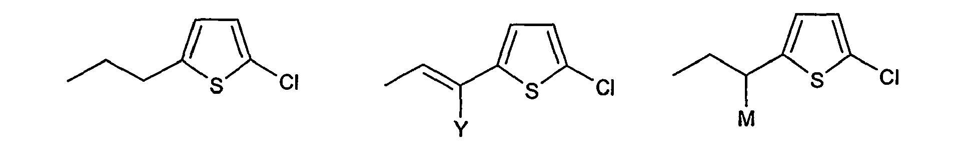 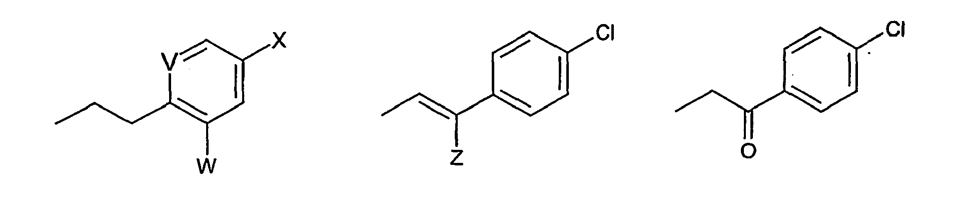 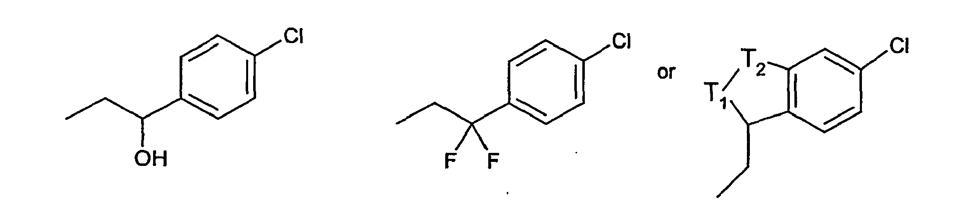
Wherein T₁ and T₂ independently represent CH₂, NH, S or O with the proviso that when one of T₁ or T₂ represents NH, S or O the other represents CH₂;
M represents CH₃, -OH or =O;
V represents CH or N;
W represents H, CH₃, Cl or F;
X represents Cl, Br, F or -CH₃;
Y represents CH₃ or CF₃;
Z represents -CH₃ or F;
R⁷ and R⁸ are independently hydrogen, C₁₋₄alkyl or together with the N atom to which they are bonded form a 5- or 6- membered non-aromatic heterocyclic ring, optionally containing an additional heteroatom selected from O, N or S;
R¹⁰ and R¹¹ independently represent C₁₋₄alkyl or together with the N atom to which they are bonded form a 5- or 6- membered non-aromatic heterocyclic ring, optionally containing an additional heteroatom selected from O, N or S;
R⁹ represents phenyl or a 5- or 6- membered aromatic or non-aromatic heterocyclic group, containing at least one heteroatom selected from O, N or S, each of which is optionally substituted by 0-2 groups selected from: C₁₋₃alkyl or halogen;
and pharmaceutically acceptable derivatives thereof.

2. A compound of formula (I) as claimed in claim 1 wherein R¹ represents hydrogen, methyl, -CH₂CO₂C₁₋₂alkyl, or -CH₂CONR⁷R⁸.

3. A compound of formula (I) as claims in claim 1 or claim 2 wherein R² and R³ independently represent -C₁₋₆alkyl, -C₁₋₃alkylCN, -C₁₋₄alkylOC₁₋₄alkyl, -C₁₋₄alkylS(O)ₙC₁₋₄alkyl, -C₁₋₄alkylNR¹⁰R¹¹, -C₁₋₃alkylCONR⁷R⁸, -C₁₋₃alkylCO₂C₀₋₂alkylR⁹, -C₁₋₃alkylCON(R⁸)C₀₋₂alkylR⁹ or -C₀₋₂alkylR⁹, with the proviso that one of R² and R³ is hydrogen and the other is a substituent other than hydrogen.

4. A compound of formula (I) as claimed in any of claims 1-3 wherein R³ represents hydrogen.

5. A compound of formula (I) as claimed in any of claims 1-4 wherein R⁶ represents a group selected from:

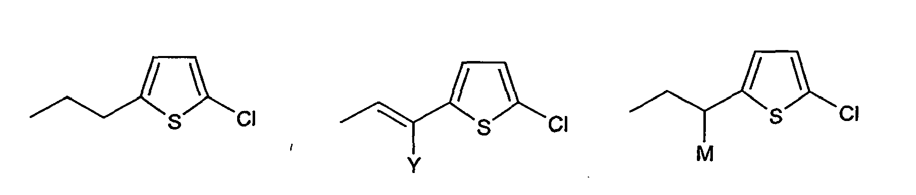

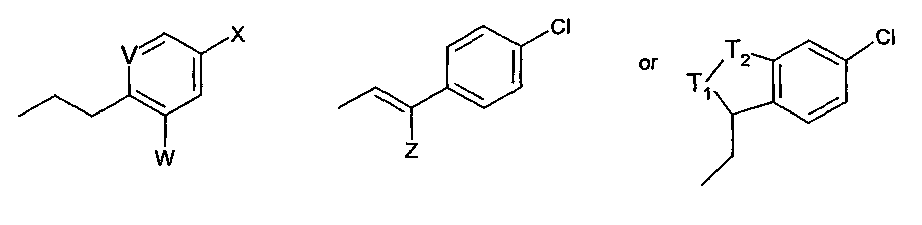

6. A compound as claimed in claim 1 wherein:
R¹ represents hydrogen, methyl, -CH₂CO₂H, -CH₂CO₂C₁₋₂alkyl, or -CH₂CONR⁷R⁸;
R² represents -C₁₋₄alkyl, -CH₂CO₂H, -CH₂OCH₃, -CH(CH₃)OCH₃, -CH₂CON(CH₃)₂, benzyl, -CH₂CO₂-benzyl, -CH₂CO-morpholine, or -CH₂-thiophene;
R³ represents hydrogen;
R⁴ and R⁵ together with the nitrogen atom to which they are attached form a morpholino ring;
R⁶ represents a group selected from: 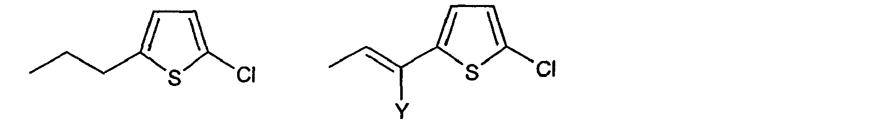 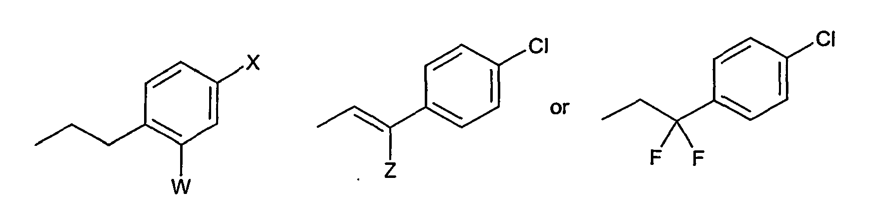
wherein W represents H, Cl or F;
X represents Cl, Br, F or -CH₃;
Y represents CH₃ or CF₃;
Z represents -CH₃ or F;
R⁷ and R⁸ are independently hydrogen or methyl.

7. A compound according to any of claims 1-6 for use in therapy.

8. A pharmaceutical composition comprising a compound according to any of claims 1-6 together with a pharmaceutical carrier and/or excipient.

9. Use of a compound according to any of claims 1-6 for the manufacture of a medicament for the treatment of a patient suffering from a condition susceptible to amelioration by a thrombin inhibitor.

10. A process for preparing a compound of formula (I) which comprises reacting a compound of formula (II) with a compound of formula (III):

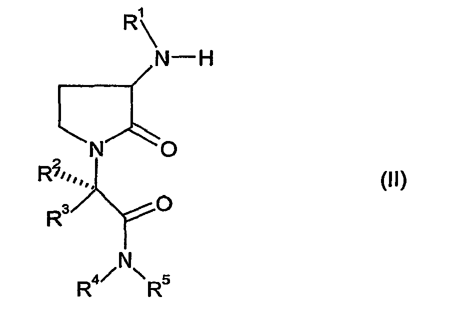

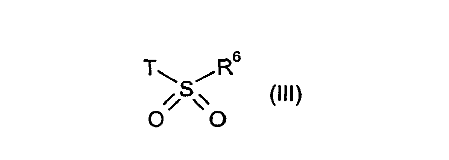

## Patentansprüche

1. Verbindung der Formel (I): 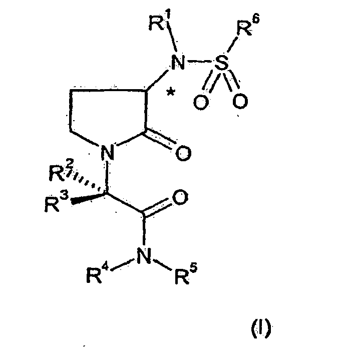 wobei:
R¹ für ein Wasserstoffatom, C₁₋₄Alkyl, -CH₂CO₂H, -CH₂CO₂C₁₋₂Alkyl oder -CH₂CONR⁷R⁸ steht;
R² und R³ unabhängig voneinander für ein Wasserstoffatom, -C₁₋₆Alkyl, -C₁₋₃AlkylCN, -C₁₋₃AlkylCO₂H, -C₁₋₄AlkylOC₁₋₄alkyl, -C₁₋₄AlkylS(O)ₙC₁₋₄alkyl, -C₁₋₄AlkylNR¹⁰R¹¹, -C₁₋₃AlkylNCO₂C₁₋₄alkyl, -C₁₋₃AlkylCONR⁷R⁸, -C₁₋₃AlkylCO₂C₀₋₂alkylR⁹, -C₁₋₃AlkylCOC₀₋₂alkylR⁹, -C₁₋₃AlkylCON(R⁸)C₀₋₂alkylR⁹, -C₁₋₃AlkylNCO₂-C₀₋₂alkylR⁹, -C₁₋₃AlkylNCOC₀₋₂alkylR⁹ oder -C₀₋₂AlkylR⁹ stehen, mit der Maßgabe, dass entweder R² oder R³ ein Wasserstoffatom ist und das andere ein von einem Wasserstoffatom verschiedener Substituent ist;
n eine ganze Zahl zwischen 0 und 2 ist;
R⁴ und R⁵ zusammen mit dem Stickstoffatom, an das sie gebunden sind, einen Morpholinring bilden;
R⁶ für eine Gruppe steht, der aus 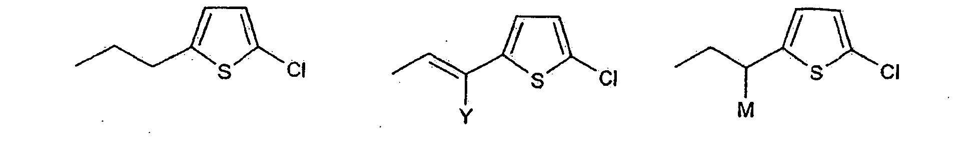 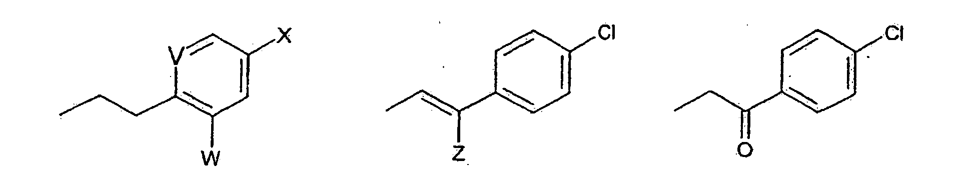 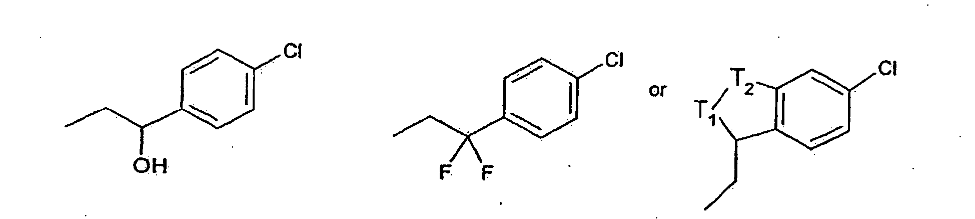
ausgewählt ist,
wobei T₁ und T₂ unabhängig voneinander für CH₂, NH, S oder O stehen, mit der Maßgabe, dass wenn entweder T₁ oder T₂ für NH, S oder O steht, der andere für CH₂ steht;
M für CH₃, -OH oder =O steht;
V für CH oder N steht;
W für H, CH₃, Cl oder F steht;
X für Cl, Br, F oder -CH₃ steht;
Y für CH₃ oder CF₃ steht;
Z für -CH₃ oder F steht;
R⁷ und R⁸ unabhängig voneinander ein Wasserstoffatom oder C₁₋₄Alkyl sind oder zusammen mit dem N-Atom, an das sie gebunden sind, einen 5- oder 6-gliedrigen,
nichtaromatischen heterocyclischen Ring bilden, der gegebenenfalls ein weiteres Heteroatom, ausgewählt aus O, N oder S, enthält;
R¹⁰ und R¹¹ unabhängig voneinander für C₁₋₄Alkyl stehen oder zusammen mit dem N-Atom, an das sie gebunden sind, einen 5- oder 6-gliedrigen, nichtaromatischen heterocyclischen Ring bilden, der gegebenenfalls ein weiteres Heteroatom, ausgewählt aus O, N oder S, enthält;
R⁹ für eine Phenylgruppe oder eine 5- oder 6-gliedrige, aromatische oder nichtaromatische heterocyclische Gruppe steht, die mindestens ein Heteroatom, ausgewählt aus O, N oder S, enthält, von denen jede gegebenenfalls durch 0 bis 2 Gruppen, ausgewählt aus C₁₋₃Alkyl oder Halogen, substituiert ist;
und pharmazeutisch verträgliche Derivate davon.

2. Verbindung der Formel (I) nach Anspruch 1, wobei R¹ für ein Wasserstoffatom, eine Methylgruppe, -CH₂CO₂C₁₋₂Alkyl oder -CH₂CONR⁷R⁸ steht.

3. Verbindung der Formel (I) nach Anspruch 1 oder Anspruch 2, wobei R² und R³ unabhängig voneinander für -C₁₋₆Alkyl, -C₁₋₃AlkylCN, -C₁₋₄AlkylOC₁₋₄alkyl, -C₁₋₄AlkylS(O)ₙC₁₋₄alkyl, -C₁₋₄AlkylNR¹⁰R¹¹, -C₁₋₃AlkylCONR⁷R⁸, -C₁₋₃AlkylCO₂-C₀₋₂alkylR⁹, -C₁₋₃AlkylCON(R⁸)C₀₋₂alkylR⁹ oder -C₀₋₂AlkylR⁹ stehen, mit der Maßgabe, dass entweder R² oder R³ ein Wasserstoffatom ist und das andere ein von einem Wasserstoffatom verschiedener Substituent ist.

4. Verbindung der Formel (I) nach einem der Ansprüche 1 bis 3, wobei R³ für ein Wasserstoffatom steht.

5. Verbindung der Formel (I) nach einem der Ansprüche 1 bis 4, wobei R⁶ für eine Gruppe steht, die aus 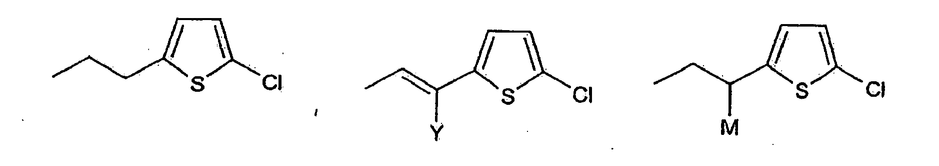 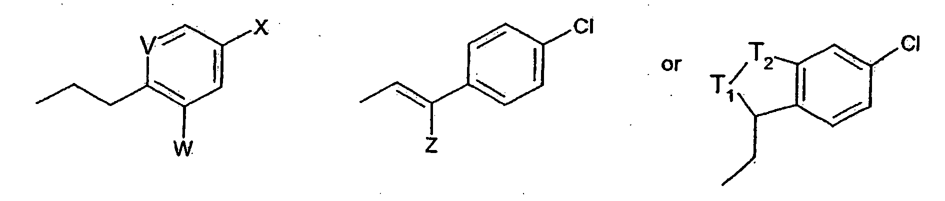 ausgewählt ist.

6. Verbindung nach Anspruch 1, wobei:
R¹ für ein Wasserstoffatom, eine Methylgruppe, -CH₂CO₂H, -CH₂CO₂C₁₋₂Alkyl oder -CH₂CONR⁷R⁸ steht;
R² für -C₁₋₄Alkyl, -CH₂CO₂H, -CH₂OCH₃, -CH(CH₃)OCH₃, -CH₂CON(CH₃)₂, eine Benzylgruppe, -CH₂CO₂-Benzyl, -CH₂CO-Morpholin oder -CH₂-Thiophen steht;
R³ für ein Wasserstoffatom steht;
R⁴ und R⁵ zusammen mit dem Stickstoffatom, an das sie gebunden sind, einen Morpholinring bilden;
R⁶ für eine Gruppe steht, die aus 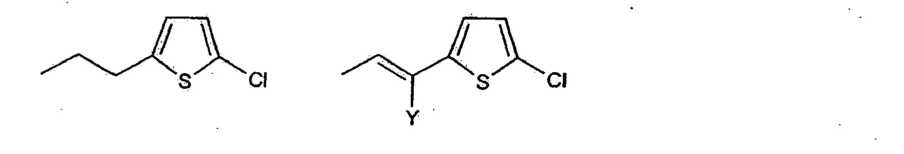 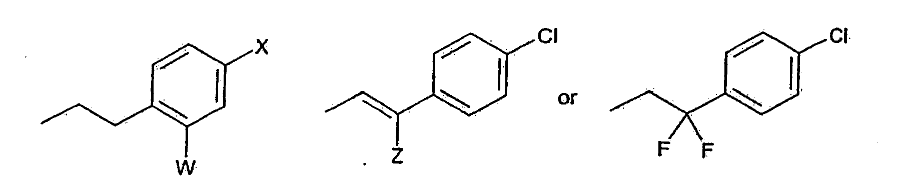
ausgewählt ist,
wobei W für H, Cl oder F steht;
X für Cl, Br, F oder -CH₃ steht;
Y für CH₃ oder CF₃ steht;
Z für -CH₃ oder F steht;
R⁷ und R⁸ unabhängig voneinander ein Wasserstoffatom oder eine Methylgruppe sind.

7. Verbindung nach einem der Ansprüche 1 bis 6 zur Verwendung in der Therapie.

8. Arzneimittel, das eine Verbindung nach einem der Ansprüche 1 bis 6, zusammen mit einem pharmazeutischen Träger und/oder Exzipienten, umfasst.

9. Verwendung einer Verbindung nach einem der Ansprüche 1 bis 6 zur Herstellung eines Medikaments zur Behandlung eines Patienten, der an einem Zustand leidet, der durch einen Thrombininhibitor gebessert werden kann.

10. Verfahren zur Herstellung einer Verbindung der Formel (I), das die Reaktion einer Verbindung der Formel (II) mit einer Verbindung der Formel (III) umfasst:

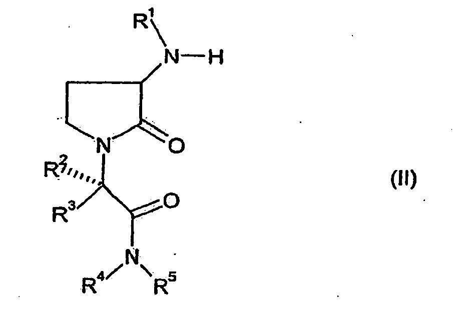

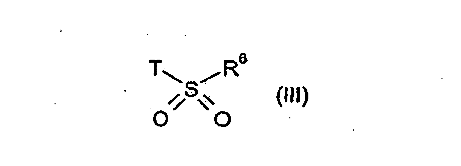

## Revendications

1. Composé de formule (I) : 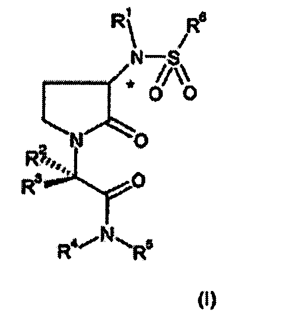 dans laquelle :
R¹ représente un atome d'hydrogène, un groupe alkyle en C₁ à C₄, -CH₂CO₂H, -CH₂CO₂ (alkyle en C₁ ou C₂) ou -CH₂CONR⁷R⁸ ;
R² et R³ représentent indépendamment un atome d'hydrogène, un groupe -alkyle en C₁ à C₆, - (alkyle en C₁ à C₃)CN, -(alkyle en C₁ à C₃)CO₂H, -(alkyle en C₁ à C₄)O(alkyle en C₁ à C₄), -(alkyle en C₁ à C₄)S(O)ₙ(alkyle en C₁ à C₄), -(alkyle en C₁ à C₄) NR¹⁰R¹¹, -(alkyle en C₁ à C₃) NCO₂ (alkyle en C₁ à C₄), - (alkyle en C₁ à C₃)CONR⁷R⁸, -(alkyle en C₁ à C₃)CO₂(alkyle en C₀ à C₂)R⁹, -(alkyle en C₁ à C₃)CO(alkyle en C₀ à C₂)R⁹, -(alkyle en C₁ à C₃) CON(R⁸) (alkyle en C₀ à C₂)R⁹, -(alkyle en C₁ à C₃)NCO₂ (alkyle en C₀ à C₂)R⁹, -(alkyle en C₁ à C₃)-NCO (alkyle en C₀ à C₂)R⁹ ou -(alkyle en C₀ à C₂)R⁹, sous réserve qu'un des groupes R² et R³ représente un atome d'hydrogène et l'autre représente un substituant autre qu'un atome d'hydrogène ;
n représente un nombre entier de 0 à 2 ;
R⁴ et R⁵, conjointement avec l'atome d'azote auquel ils sont fixés, forment un noyau morpholino ;
R⁶ représente un groupe choisi entre des groupes : 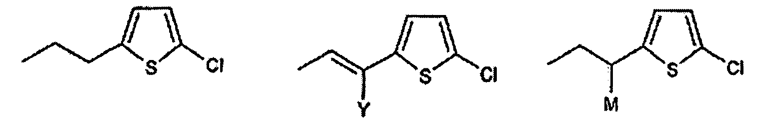 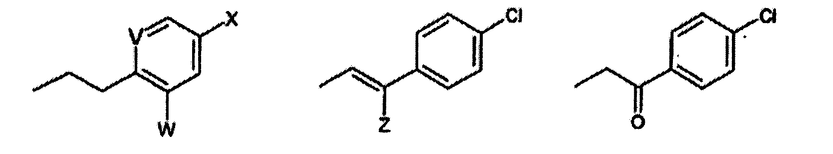 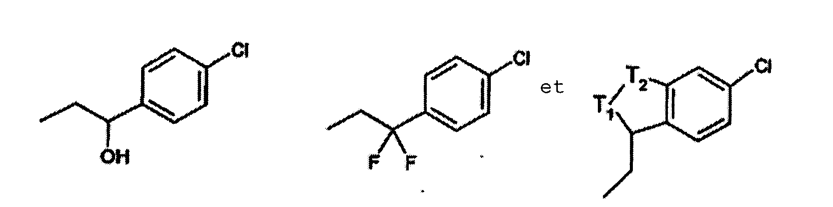
dans lesquels T₁ et T₂ représentent indépendamment un groupe CH₂, NH, S ou O, sous réserve que, lorsqu'un des groupes T₁ et T₂ représente un groupe NH, S ou O, l'autre représente un groupe CH₂ ;
M représente un groupe CH₃, -OH ou =O ;
V représente un groupe CH ou N ;
W représente H, un groupe CH₃, Cl ou F ;
X représente un groupe Cl, Br, F ou -CH₃ ;
Y représente un groupe CH₃ ou CF₃ ;
Z représente un groupe -CH₃ ou F ;
R⁷ et R⁸ représentent indépendamment un atome d'hydrogène ou un groupe alkyle en C₁ à C₄ ou bien, conjointement avec l'atome de N auquel ils sont liés, forment un noyau hétérocyclique non aromatique penta- ou hexagonal, contenant facultativement un hétéroatome supplémentaire choisi entre O, N et S ;
R¹⁰ et R¹¹ représentent indépendamment un groupe alkyle en C₁ à C₄ ou bien, conjointement avec l'atome de N auquel ils sont liés, forment un noyau hétérocyclique non aromatique penta- ou hexagonal, contenant facultativement un hétéroatome supplémentaire choisi entre O, N et S ;
R⁹ représente un groupe phényle ou un groupe hétérocyclique aromatique ou non aromatique penta- ou hexagonal, contenant au moins un hétéroatome choisi entre O, N et S, chacun de ces groupes étant facultativement substitué avec 0 à 2 groupes choisis entre des groupes : alkyle en C₁ à C₃ et halogéno ;
et ses dérivés pharmaceutiquement acceptables.

2. Composé de formule (I) suivant la revendication 1, dans lequel R¹ représente un atome d'hydrogène, un groupe méthyle, -CH₂CO₂(alkyle en C₁ ou C₂) ou -CH₂CONR⁷R⁸.

3. Composé de formule (I) suivant la revendication 1 ou la revendication 2, dans lequel R² et R³ représentent indépendamment un groupe -alkyle en C₁ à C₆, -(alkyle en C₁ à C₃)CN, -(alkyle en C₁ à C₄)O(alkyle en C₁ à C₄), -(alkyle en C₁ à C₄)S(O)ₙ(alkyle en C₁ à C₄), -(alkyle en C₁ à C₄)NR¹⁰R¹¹, -(alkyle en C₁ à C₃)CONR⁷R⁸, -(alkyle en C₁ à C₃)CO₂(alkyle en C₀ à C₂)R⁹, -(alkyle en C₁ à C₃) CON(R⁸) (alkyle en C₀ à C₂)R⁹ ou - (alkyle en C₀ à C₂)R⁹, sous réserve qu'un des groupes R² et R³ représente un atome d'hydrogène et l'autre représente un substituant autre qu'un atome d'hydrogène.

4. Composé de formule (I) suivant l'une quelconque des revendications 1 à 3, dans lequel R³ représente un atome d'hydrogène.

5. Composé de formule (I) suivant l'une quelconque des revendications 1 à 4, dans lequel R⁶ représente un groupe choisi entre des groupes :

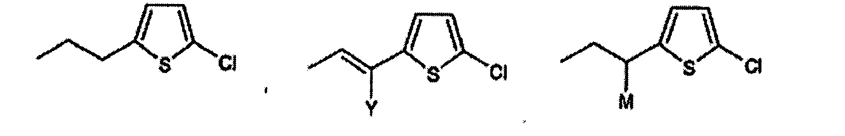

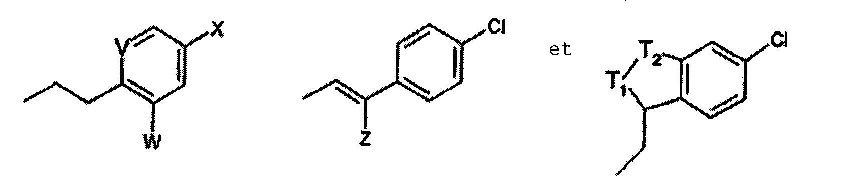

6. Composé suivant la revendication 1, dans lequel :
R¹ représente un atome d'hydrogène, un groupe méthyle, -CH₂CO₂H, -CH₂CO₂(alkyle en C₁ ou C₂) ou -CH₂CONR⁷R⁸ ;
R² représente un groupe -alkyle en C₁ à C₄, -CH₂CO₂H, -CH₂OCH₃, -CH (CH₃) OCH₃, -CH₂CON (CH₃)₂, benzyle, -CH₂CO₂-benzyle, -CH₂CO-morpholine ou -CH₂-thiophène ;
R³ représente un atome d'hydrogène ;
R⁴ et R⁵, conjointement avec l'atome d'azote auquel ils sont fixés, forment un noyau morpholino ;
R⁶ représente un groupe choisi entre des groupes : 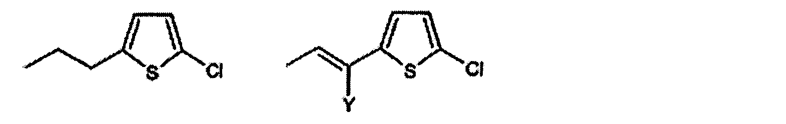 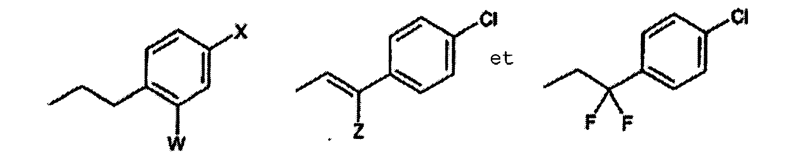
dans lesquels W représente H, un groupe Cl ou F ;
X représente un groupe Cl, Br, F ou -CH₃ ;
Y représente un groupe CH₃ ou CF₃ ;
Z représente un groupe -CH₃ ou F ;
R⁷ et R⁸ représentent indépendamment un atome d'hydrogène ou un groupe méthyle.

7. Composé suivant l'une quelconque des revendications 1 à 6, destiné à être utilisé en thérapie.

8. Composition pharmaceutique comprenant un composé suivant l'une quelconque des revendications 1 à 6, conjointement avec un support et/ou excipient pharmaceutique.

9. Utilisation d'un composé suivant l'une quelconque des revendications 1 à 6, pour la production d'un médicament destiné au traitement d'un patient souffrant d'une affection apte à l'amélioration par un inhibiteur de thrombine.

10. Procédé pour la préparation d'un composé de formule (I), qui comprend la réaction d'un composé de formule (II) avec un composé de formule (III) :
